# EUROPEAN PATENT SPECIFICATION

(11) **EP 1 229 782 B1**
(45) Date of publication and mention of the grant of the patent: **07.11.2012**
(21) Application number: 00982129.9
(22) Date of filing: 14.11.2000
(51) Int. Cl.: A01H 1/00, A01H 5/00, C12N 5/14, C12N 15/82

(54) **PATHOGEN TOLERANCE GENES**
PATHOGENTOLERANZGENE
GENES DE RESISTANCE AUX ELEMENTS PATHOGENES

(30) Priority: 17.11.1999 US 166228 P; 17.04.2000 US 197899 P; 22.08.2000 US 227439 P
(43) Date of publication of application: 14.08.2002
(73) Proprietor: Mendel Biotechnology, Inc., Hayward, CA 94545 (US)
(72) Inventor: HEARD, Jacqueline, San Mateo, CA 94402 (US); RATCLIFFE, Oliver, Oakland, CA 94606 (US); CREELMAN, Robert, Castro Valley, CA 94546 (US); JIANG, Cai-Zhong, Fremont, CA 94555 (US); PINEDA, Omaira, Vero Beach, FL 32960 (US); REUBER, Lynne T., San Mateo, CA 94402 (US); ADAM, Luc, Hayward, CA 94545 (US)
(74) Representative: Brasnett, Adrian Hugh
(86) International application number: PCT/US2000/031418
(87) International publication number: WO 2001/035726

(56) References cited:
- EP-A- 0 812 917
- EP-A- 1 033 405
- WO-A-01/25458
- WO-A-01/26459
- WO-A-01/36598
- WO-A-01/49840
- WO-A-02/08410
- WO-A-02/08411
- WO-A-02/15675
- WO-A-02/16655
- WO-A-02/22675
- WO-A-97/47183
- WO-A-99/41974
- WO-A1-97/47183
- US-A- 5 939 601
- US-A1- 2003 046 723
- "KAOS information about MXC20.3"[Online] 10 November 1999 (1999-11-10), XP002303477 Retrieved from the Internet: URL:http://web.archive.org/web/19991110092 029/http://www.kazusa.or.jp/arabi/chr5/clo ne/MXC20/MXC20.3.html> [retrieved on 2004-10-28]
- DATABASE EMBL [Online] 3 September 1999 (1999-09-03), "Arabidopsis thaliana genomicDNA, chromosome 5, P1 clone: MXC20." XP002303478 Database accession no. AB009055
- SATO ET AL: "Structural analysis of Arabidopsis thaliana chromosome 5. IV. Sequence features of the regions of 1,456,315 bp covered by nineteen physically assigned P1 and AC clones" DNA RESEARCH, UNIVERSAL ACADEMY PRESS, JP, vol. 5, 1998, pages 41-54, XP002146371 ISSN: 1340-2838
- DATABASE EMBL [Online] 24 June 1997 (1997-06-24), "Arabidopsis thaliana BAC IG002N01." XP002303479 Database accession no. AF007269
- EULGEM THOMAS ET AL: "Early nuclear events in plant defence signalling: Rapid gene activation by WRKY transcription factors" EMBO JOURNAL, OXFORD UNIVERSITY PRESS, SURREY, GB, vol. 18, no. 17, 1 September 1999 (1999-09-01), pages 4689-4699, XP002169346 ISSN: 0261-4189
- MEISSNER RUTH C ET AL: "Function search in a large transcription factor gene family in Arabidopsis: Assessing the potential of reverse genetics to identify insertional mutations in R2R3 MYB genes" PLANT CELL, AMERICAN SOCIETY OF PLANT PHYSIOLOGISTS, ROCKVILLE, MD, US, vol. 11, no. 10, October 1999 (1999-10), pages 1827-1840, XP002192999 ISSN: 1040-4651
- DATABASE EMBL [Online] 28 July 1997 (1997-07-28), "Arabidopsis thaliana AP2 domain containing protein RAP2.3 mRNA, complete cds." XP002314275 retrieved from EBI accession no. EM_PRO:AF003096 Database accession no. AF003096 -& DATABASE UniProt [Online] 1 November 1995 (1995-11-01), "AP2 domain transcription factor RAP2.3 (Related to AP2 protein 3) (Cadmium-induced protein AS30)." XP002314276 retrieved from EBI accession no. UNIPROT:P42736 Database accession no. P42736 -& OKAMURO J ET AL: "The AP2 domain of APETALA2 defines a large new family of DNA binding proteins in Arabidopsis" PROCEEDINGS OF THE NATIONAL ACADEMY OF SCIENCES OF USA, NATIONAL ACADEMY OF SCIENCE. WASHINGTON, US, vol. 94, no. 13, June 1997 (1997-06), pages 7076-7081, XP002108957 ISSN: 0027-8424
- DATABASE EMBL [Online] 21 August 1998 (1998-08-21), "Arabidopsis thaliana AtERF-1 mRNA for ethylene responsive element binding factor 1, complete cds." XP002314277 retrieved from EBI accession no. EM_PRO:AB008103 Database accession no. AB008103 -& DATABASE UniProt [Online] 30 May 2000 (2000-05-30), "Ethylene-responsive transcription factor 1A (Ethylene-responsive element binding factor 1A) (EREBP-1A) (AtERF1)." XP002314278 retrieved from EBI accession no. UNIPROT:O80337 Database accession no. O80337 -& HAO DONGYUN ET AL: "Unique mode of GCC box recognition by the DNA-binding domain of ethylene-responsive element-binding factor (ERF domain) in plant" JOURNAL OF BIOLOGICAL CHEMISTRY, vol. 273, no. 41, 9 October 1998 (1998-10-09), pages 26857-26861, XP002314272 ISSN: 0021-9258 -& FUJIMOTO SUSAN Y ET AL: "Arabidopsis ethylene-responsive element binding factors act as transcriptional activato
- DATABASE EMBL [Online] 4 July 1997 (1997-07-04), "Arabidopsis thaliana DNA chromosome 4, ESSA I FCA contig fragment No. 8" XP002314279 retrieved from EBI accession no. EM_PRO:Z97343 Database accession no. Z97343
- DATABASE EMBL [Online] 28 June 1997 (1997-06-28), "Lycopersicon esculentum DNA-binding protein Pti4 mRNA, complete cds." XP002314280 retrieved from EBI accession no. EM_PRO:U89255 Database accession no. U89255 -& ZHOU JIANMIN ET AL: "The Pto kinase conferring resistance to tomato bacterial speck disease interacts with proteins that bind a cis-element of pathogenesis-related genes" EMBO (EUROPEAN MOLECULAR BIOLOGY ORGANIZATION) JOURNAL, vol. 16, no. 11, 1997, pages 3207-3218, XP002314274 ISSN: 0261-4189
- DATABASE GENBANK [Online] US NATIONAL LIBRARY OF MEDICINE, (BETHESDA, MD, USA) 27 December 2000 SATO S. ET AL.: 'Structural analysis of arabidopsis thaliana chromosone 5. IV. Sequence features of the regions of 1,456,315 bp covered by nineteen physically assigned P1 and TAC clones', XP002937718 Retrieved from NCBI Database accession no. AB009055 & DNA RES. vol. 5, no. 1, 1998, pages 41 - 54

## Description

### FIELD OF THE INVENTION

This invention relates to the field of plant biology. More particularly, the present invention pertains to compositions and methods for phenotypically modifying a plant.

### BACKGROUND OF THE INVENTION

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

WO 97/47183 discloses three plant genes, Pti4, Pti5 and Pti6 which interact with the plant resistance gene Pto in a two-hybrid assay in yeast.

### SUMMARY OF THE INVENTION

The present invention provides a method for producing a transgenic plant having an improved tolerance or resistance to a biotropic or necrotropic fungal pathogen, the method comprising:
transforming a plant or part thereof with a nucleotide sequence which encodes a transcription factor polypeptide having at least 75% sequence identity to the polypeptide of SEQ ID NO:10, wherein said polypeptide also has a conserved domain having at least 80% sequence identity to amino acids 145-213 of SEQ ID NO:10,
wherein expression of the transcription factor polypeptide in the transgenic plant increases the transgenic plant's tolerance or resistance to a biotropic or necrotropic fungal pathogen compared to a wild-type plant, and
selecting a transgenic plant that expresses said nucleotide and provides for said improved pathogen tolerance or resistance thereby providing the transgenic plant with said improved tolerance or resistance to a biotropic or necrotropic fungal pathogen compared to a wild-type plant.

In one aspect, the polynucleotide is a polynucleotide that encodes a polypeptide having at least 90% identity to SEQ ID NO:10. In another aspect, the biotropic fungal pathogen is *Erisyphe orontii.*

In another embodiment, a constitutive, inducible, or tissue-active promoter is operably linked to said nucleotide sequence.

The plant may be selected from the group consisting of soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

The present disclosure also relates to a cloning or expression vector comprising the polynucleotide described above, or cells comprising the cloning or expression vector.

Furthermore, described herein is a method for producing a plant having improved pathogen tolerance. The method comprises altering the expression of an isolated or recombinant polynucleotide or altering the expression or activity of a polypeptide in a plant to produce a modified plant, and selecting the modified plant for modified pathogen tolerance.

Also disclosed herein is a method of linking the one or more of the polynucleotides described below, or encoded polypeptides, to a modified plant pathogen tolerance phenotype.

### BRIEF DESCRIPTION OF THE DRAWINGS

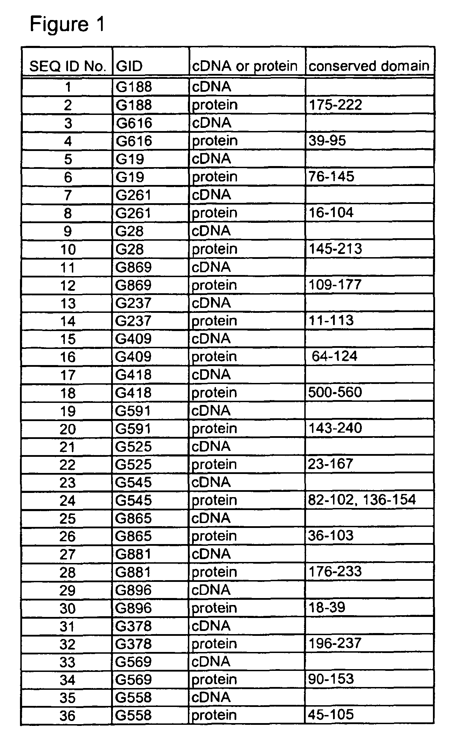

Figure 1 provides a table of exemplary polynucleotide and polypeptide sequences. The table includes from left to right for each sequence: the SEQ ID No., the internal code reference number (GID), whether the sequence is a polynucleotide or polypeptide sequence, and identification of any conserved domains for the polypeptide sequences.

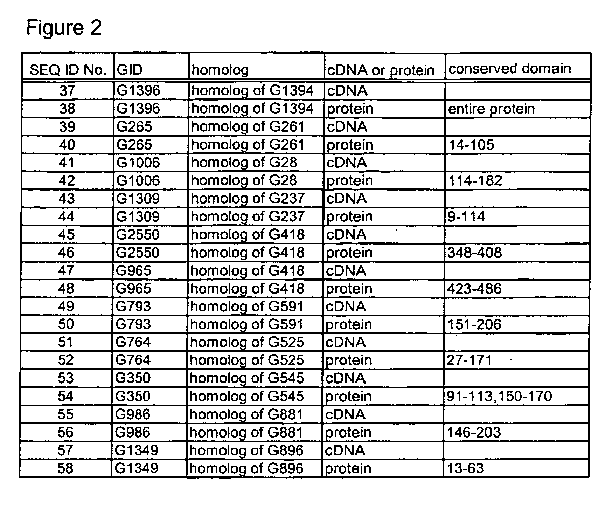

Figure 2 provides a table of exemplary sequences that are homologous to other sequences provided in the Sequence Listing and that are derived from *Arabidopsis thaliana.* The table includes from left to right: the SEQ ID No., the internal code reference number (GID), identification of the homologous sequence, whether the sequence is a **polynucleotide** or polypeptide sequence, and identification of any conserved domains for the polypeptide sequences.

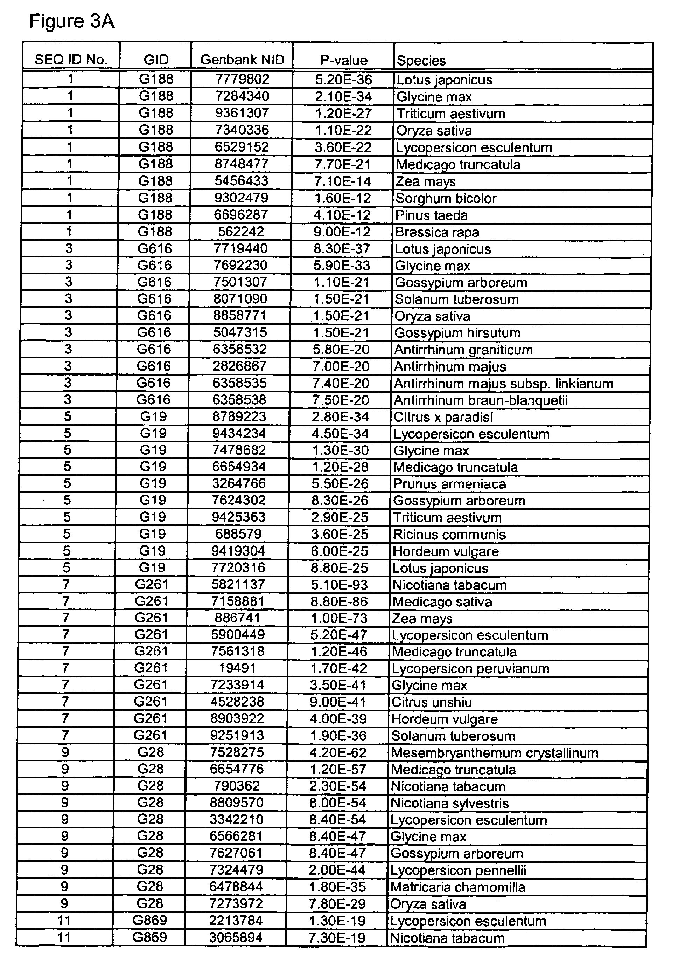

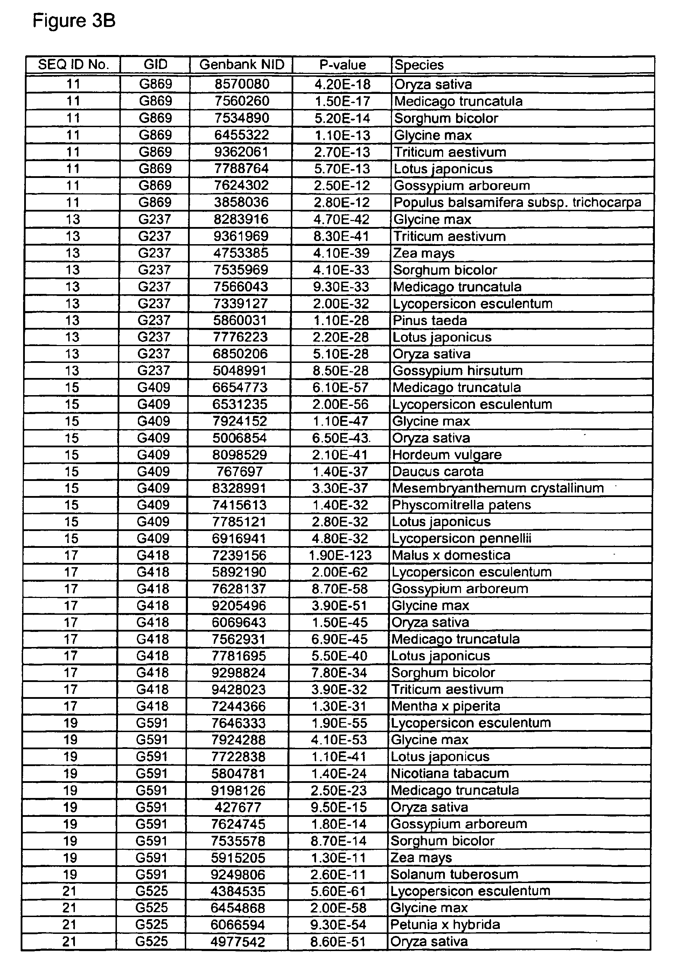

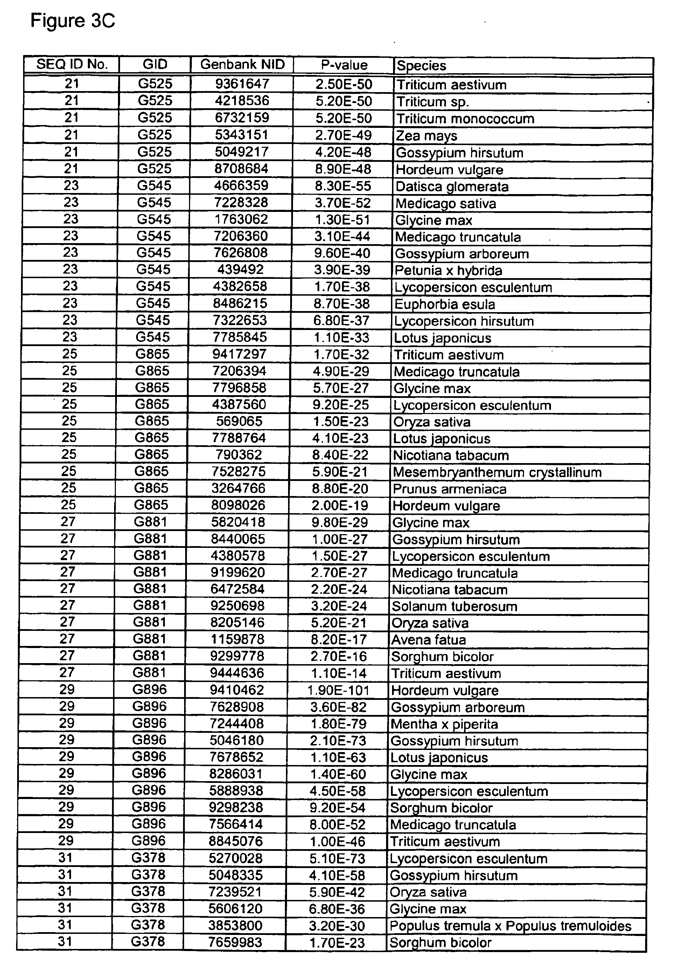

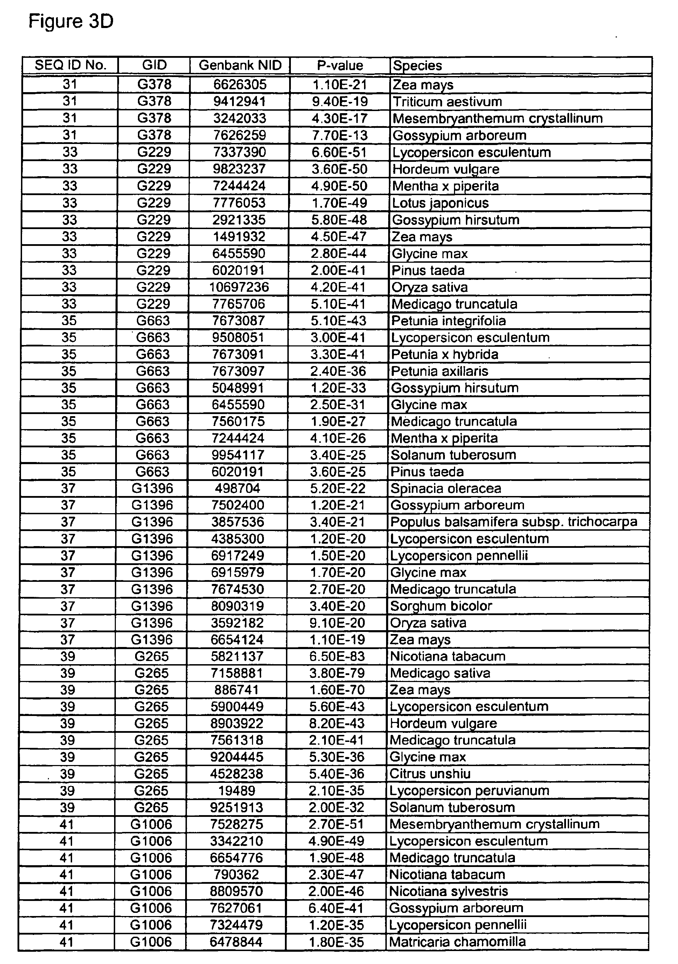

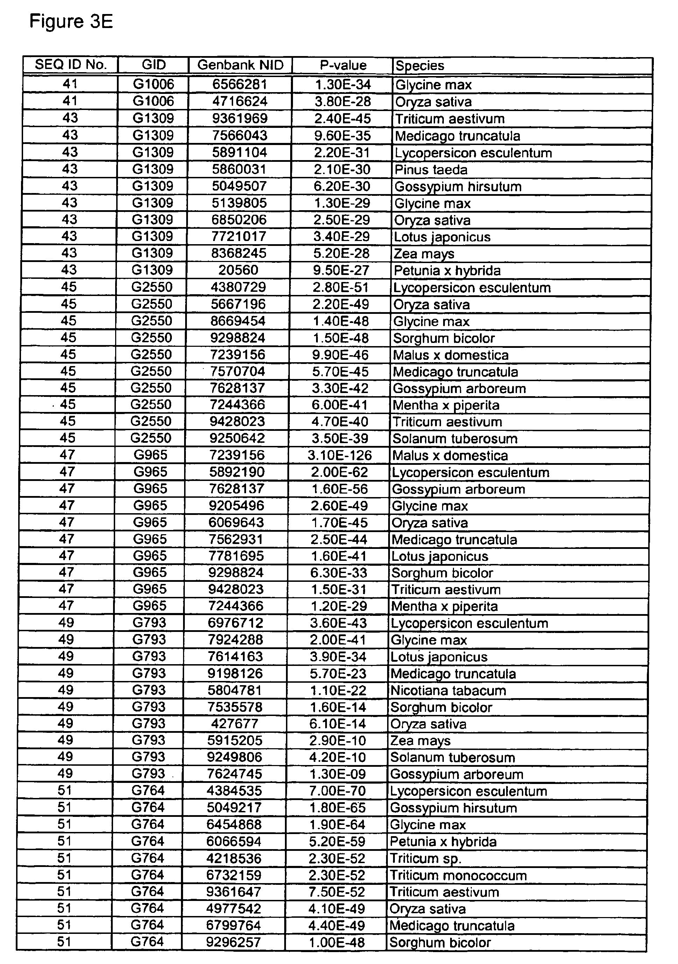

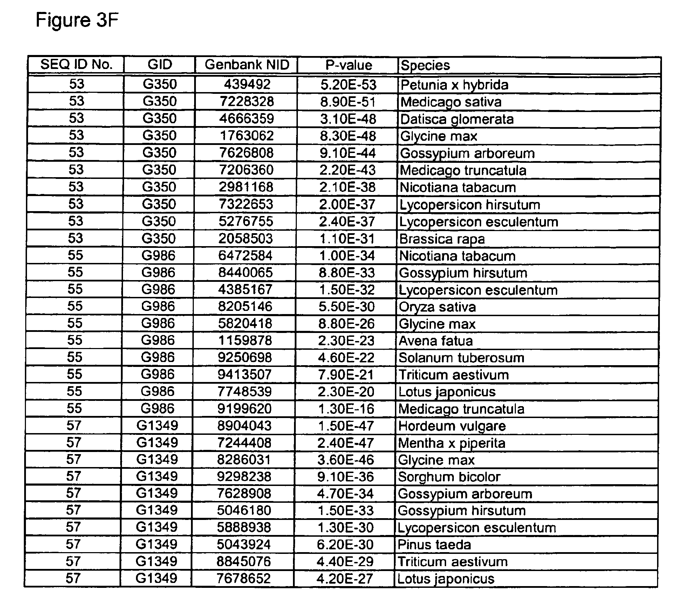

Figure 3 provides a table of exemplary sequences that are homologous to the sequences provided in Figures 1 and 2 and that are derived from plants other than *Arabidopsis thaliana.* The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

The present invention relates to modifying phenotypes of plants.

In particular, polynucleotides or polypeptides described herein are useful for modifying traits associated with a plant's pathogen tolerance when the expression levels of the polynucleotides or expression levels or activity levels of the polypeptides are altered. Specifically, the polynucleotides and polypeptides are useful for modifying traits associated with a plant's pathogen tolerance, such as alterations in cell wall composition, trichome number or structure, callose induction, phytoalexin induction, alterations in the cell death response, or the like. Transgenic plants employing the polynucleotides or polypeptides described herein are more tolerant to biotrophic or necrotrophic pathogens such as fungi, bacteria, mollicutes, viruses, nematodes, parasitic higher plants or the like,

The polynucleotides described herein encode plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) J. Biol. Chem. 379:633-646); the MYB transcription factor family (Martin and Paz-Ares (1997) Trends Genet. 13:67-73); the MADS domain transcription factor family (Riechmann and Meyerowitz (1997) J. Biol. Chem 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Duboule (1994) Guidebook to the Homeobox Genes. Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides described herein, the polynucleotides and polypeptides have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide described herein relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed ( such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES

Described herein are among other things, transcription factors (TFs), and transcription factor homologue polypeptides, and isolated or recombinant polynucleotides encoding the polypeptides. These polypeptides and polynucleotides may be employed to modify a plant's pathogen tolerance.

Exemplary polynucleotides encoding the polypeptides described herein were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides described herein were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides described herein were ectopically expressed in overexpressor or knockout plants and changes in the pathogen tolerance of the plants was observed. Therefore, the polynucleotides and polypeptides can be employed to improve the pathogen resistance of plants.

### Making polynucleotides

The polynucleotides described herein include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides described herein Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 2000) ("Ausubel").

Alternatively, polynucleotides described herein, can be produced by a variety of in vitro amplification methods by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids described herein are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* e.g., Ausubel, Sambrook and Berger, *all supra.*

Alternatively, polynucleotides and oligonucleotides described herein can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides described herein can be custom ordered from any of a number of commercial suppliers.

### HOMOLOGOUS SEQUENCES

Sequences homologous, i.e., that share significant sequence identity or similarity, to those provided in the Sequence Listing, derived from *Arabidopsis thaliana* or from other plants of choice are also available. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to SEQ ID NO: 10 will share at least 75%, e.g., about 80% or about 90% or about 95% or about 98% or more sequence identity with the SEQ ID NO: 10 Factors that are most closely related to the listed sequences share, e.g., the SEQ ID NO: 10. Factors that are most closely related to the listed sequences share, e.g., at least about 85%, about 90% or about 95% or more % sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family exhibit a higher degree of sequence homology, such as at least 80% sequence identity including conservative substitutions.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radio active label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E. coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptides described herein. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table may be employed.

In addition to silent variations, other conservative variations that alter one, or a few amino acids in the encoded polypeptide, can be made without altering the function of the polypeptide, these conservative variants may, likewise, be employed.

For example, substitutions, deletions and insertions introduced into the sequences provided in the Sequence Listing are also envisioned. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES-MUTATION/ FORCED EVOLUTION

In addition to generating silent or conservative substitutions as noted, above, methods of modifying the sequences of the Sequence Listing may be employed. In the methods, nucleic acid or protein modification methods are used to alter the given sequences to produce new sequences and/or to chemically or enzymatically modify given sequences to change the properties of the nucleic acids or proteins.

Thus given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, modification of any given nucleic acid by mutation, evolution, chemical or enzymatic modification, or other available methods, as well as for the products produced by practicing such methods, e.g., using the sequences herein as a starting substrate for the various modification approaches, may be employed

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S*. *cerevisiae* and mammals are TAA and TGA, respectively. The preferred stop codon for monocotyledonous plants is TGA, whereas insects and *E. coli* prefer to use TAA as the stop codon.

The polynucleotide sequences described herein can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides described herein can be combined with domains derived from other transcription factors or synthetic domains to modify the biological activity of a transcription factor. For instance, a DNA binding domain derived from a transcription factor described herein can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences described herein are incorporated into recombinant DNA (or RNA) molecules that direct expression of polypeptides described herein in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

Recombinant constructs comprising one or more of the nucleic acid sequences herein may be used. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence described herein has been inserted, in a forward orientation. In a preferred aspect the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see,* e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide described herein in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI,* Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present description also relates to host cells which are transduced with vectors described herein, and the production of polypeptides described herein (including fragments thereof) by recombinant techniques. Host cells are genetically engineered (i.e, nucleic acids are introduced, e.g., transduced, transformed or transfected) with the vectors described herein, which may be, for example, a cloning vector or an expression vector comprising the relevant nucleic acids herein. The vector is optionally a plasmid, a viral particle, a phage, a naked nucleic acids, *etc.* The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small bcads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably intcgratcd into the plant genome (Horsch et al. (1984) Science 233:496-498; Fraley et al. (1983) Proc. Natl. Acad. Sci. USA 80, 4803).

The cell can include a nucleic acid described herein which encodes a polypeptide, wherein the cells expresses a polypeptide. described herein. The cell can also include vector sequences, or the like. Furthermore, cells and transgenic plants which include any polypeptide or nucleic acid above or throughout this specification, e.g., produced by transduction of a vector described herein, are an additional feature.

For long-term, high-yield production of recombinant proteins, stable expression can be used. Host cells transformed with a nucleotide sequence encoding a polypeptide described herein are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins described herein can be designed with signal sequences which direct secretion of the mature polypeptides through a prokaryotic or eukaryotic cell membrane.

### Modified Amino Acids

Polypeptides described herein may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor provided by the present disclosure can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor described herein to modify a phenotype as desired. For example, the transcription factors can be employed to identify one or more downstream gene with which is subject to a regulatory effect of the transcription factor. In one approach, a transcription factor or transcription factor homologue described herein is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor described herein can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and PCT/US96/10287), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J.Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with the present disclosure e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides according to the present disclosure.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

Described herein is a method for identifying composition that modulate the activity or expression of a polynucleotide or polypeptide described herein. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide described herein.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra.*

Subsequences of the polynucleotides described herein, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues described herein including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, described herein is an isolated or recombinant polypeptide including a subsequence of at least about 15 contiguous amino acids encoded by the recombinant or isolated polynucleotides described herein. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides described herein are favorably employed to produce transgenic plants with various traits, or characteristics, that have been modified in a desirable manner, e.g., to improve the pathogen resistance of a plant. For example, alteration of expression levels or patterns (e.g., spatial or temporal expression patterns) of one or more of the transcription factors (or transcription factor homologues) described herein, as compared with the levels of the same protein found in a wild type plant, can be used to modify a plant's traits. An illustrative example of trait modification, improved pathogen tolerance, by altering expression levels of a particular transcription factor is described further in the Examples and the Sequence Listing.

A plant trait can also be modified by using the cre-lox system (for example, as described in US Patent No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides described herein can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide described herein (*See*, e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides described herein and/or expressing the polypeptides described herein can be produced by a variety of well established techniques as described above. Following construction of a vector, most typically an expression cassette, including a polynucleotide, e.g., encoding a transcription factor or transcription factor homologue, described herein standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; microinjection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modifed trait can be any of those traits described above. Additionally, to confirm that the modified trait is due to changes in expression levels or activity of the polypeptide or polynucleotide described herein can be determined by analyzing mRNA expression using Northern blots, RT-PCR or microarrays, or protein expression using immunoblots or Western blots or gel shift assays.

### INTEGRATED SYSTEMS—SEQUENCE IDENTITY

Additionally, described herein is an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved pathogen tolerance, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods of determining sequence relationships can be used, including manual alignment and computer assisted sequence alignment and analysis. This later approach is a preferred approach, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The above methods can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and the computers may be components of an intra-net or an internet.

Thus, methods are described herein for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20. and the amplified DNA fragment were digested separately with Sall and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E*. *coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ∼10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 bases to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF PATHOGEN INDUCED GENES

In some instances, expression patterns of the pathogen induced genes (such as defense genes) was monitored by microarray experiments. cDNAs were generated by PCR and resuspended at a final concentration of ∼ 100 ng/ul in 3X SSC or 150mM Na-phosphate (Eisen and Brown (1999) Meth. in Enzymol. 303:179-205). The cDNAs were spotted on microscope glass slides coated with polylysine. The prepared cDNAs were aliquoted into 384 well plates and spotted on the slides using an x-y-z gantry (OmniGrid) purchased from GeneMachines (Menlo Park, CA) outfitted with quill type pins purchased from Telechem International (Sunnyvale, CA). After spotting, the arrays were cured for a minimum of one week at room temperature, rehydrated and blocked following the protocol recommended by Eisen and Brown (1999).

Sample total RNA (10 ug) samples were labeled using fluorescent Cy3 and Cy5 dyes. Labeled samples were resuspended in 4X SSC/0.03% SDS/4 ug salmon sperm DNA/2 ug tRNA/ 50mM Na-pyrophosphate, heated for 95°C for 2.5 minutes, spun down and placed on the array. The array was then covered with a glass coverslip and placed in a sealed chamber. The chamber was then kept in a water bath at 62°C overnight. The arrays were washed as described in Eisen and Brown (1999) and scanned on a General Scanning 3000 laser scanner. The resulting files are subsequently quantified using Imagene a software purchased from BioDiscovery (Los Angeles, CA).

### EXAMPLE VIII. IDENTIFICATION OF PATHOGEN TOLERANCE PHENOTYPE IN OVEREXPRESSOR OR GENE KNOCKOUT PLANTS

Experiments were performed to identify those transformants or knockouts that exhibited an improved pathogen tolerance. For such studies, the transformants were exposed to biotropic fungal pathogens, such as *Erisyphe orontii,* and necrotropic fungal pathogens, such as *Fusarium oxysporum. Fusarium oxysporum* isolates cause vascular wilts and damping off of various annual vegetables, perennials and weeds (Mauch-Mani and Slusarenko (1994) Molecular Plant-Microbe Interactions 7: 378-383). For *Fusarium oxysporum* experiments, plants grown on petri dishes were sprayed with a fresh spore suspension of *F oxysporum.* The spore suspension was prepared as follows: A plug of fungal hyphae from a plate culture was placed on a fresh potato dextrose agar plate and allowed to spread for one week. 5 ml sterile water was then added to the plate, swirled, and pipetted into 50 ml Armstrong Fusarium medium. Spores were grown overnight in Fusarium medium and then sprayed onto plants using a Preval paint sprayer. Plant tissue was harvested and frozen in liquid nitrogen 48 hours post infection.

*Erysiphe orontii* is a causal agent of powdery mildew. For *Erysiphe orontii* experiments, plants were grown approximately 4 weeks in a greenhouse under 12 hour light (20 C, ∼30% relative humidity (rh)). Individual leaves were infected with *E. orontii* spores from infected plants using a camel's hair brush, and the plants were transferred to a Percival growth chamber (20 C, 80% rh.). Plant tissue was harvested and frozen in liquid nitrogen 7 days post infection.

Botrytis cinerea is a necrotrophic pathogen. Botrytis cinerea was grown on potato dextrose agar in the light. A spore culture was made by spreading 10 ml of sterile water on the fungus plate, swirling and transferring spores to 10 ml of sterile water. The spore inoculum (approx. 105 spores/ml) was used to spray 10 day-old seedlings grown under sterile conditions on MS (-sucrose) media. Symptoms were evaluated every day up to approximately 1 week.

Infection with bacterial pathogens Pseudomonas syringae pv maculicola strain 4326 and pv maculicola strain 4326 was performed by hand inoculation at two doses. Two inoculation doses allows the differentiation between plants with enhanced susceptibility and plants with enhanced resistance to the pathogen. Plants were grown for 3 weeks in the greenhouse, then transferred to the growth chamber for the remainder of their growth. Psm ES4326 was hand inoculated with 1 ml syringe on 3 fully-expanded leaves per plant (4 1/2 wk old), using at least 9 plants per overexpressing line at two inoculation doses, OD=0.005 and OD=0.0005. Disease scoring occured at day 3 post-inoculation with pictures of the plants and leaves taken in parallel.

Table 3 shows the phenotypes observed for particular overexpressor or knockout plants and provides the SEQ ID No., the internal reference code (GID), whether a knockout or overexpressor plant was analyzed and the observed phenotype.

**Table 3**

| SEQ ID No. | GID | Knockout (KO) or overexpressor (OE) | Phenotype |
|---|---|---|---|
| 1 | G188 | KO | Increased susceptibility to Fusarium |
| 3 | G616 | OE | Increased tolerance to Erysiphe |
| 5 | G19 | OE | Increased tolerance to Erysiphe |
| 7 | G261 | OE | Increased susceptibility to Botrytis |
| 9 | G28 | OE | Increased resistance to Erysiphe |
| 11 | G869 | OE | Increased susceptibility to Fusarium |
| 13 | G237 | OE | Increased tolerance to Erysiphe |
| 15 | G409 | OE | Increased tolerance to Erysiphe |
| 17 | G418 | OE | Increased tolerance to Pseudomonas |
| 19 | G591 | OE | Increased tolerance to Erysiphe |
| 21 | G525 | OE | Increased tolerance to Pseudomonas |
| 23 | G545 | OE | Increased susceptibility to Pseudomonas, Erysiphe and Fusarium |
| 25 | G865 | OE | Increased susceptibility to Erysiphe and Botrytis |
| 27 | G881 | OE | Increased susceptibility to Erysiphe and Botrytis |
| 29 | G896 | KO | Increased susceptibility to Fusarium |
| 31 | G378 | OE | Increased resistance to Erysiphe |
| 33 | G569 | OE | Decreased expression of defense genes |
| 35 | G558 | OE | Increased expression of defense genes |

For a particular overexpressor that shows an increased susceptibility to a pathogen, it may be more useful to select a plant with a decreased expression of the particular transcription factor. For a particular knockout that shows an increased susceptibility to a pathogen, it may be more useful to select a plant with an increased expression of the particular transcription factor.

Other than *Fusarium oxysporum, Erysyphe orontii,* the transgenic plants are more tolerant to *Sclerotinia spp.,* soil-borne oomycetes, foliar oomycetes, *Botrytis spp., Rhizoctonia spp, Verticillium dahliae*/*albo-atrum, Alternaria spp.,* rusts, *Mycosphaerella spp, Fusarium solani,* or the like. The transgenic plants are more resistant to fungal diseases such as rusts, smuts, wilts, yellows, root rot, leaf drop, ergot, leaf blight of potato, brown spot of rice, leaf blight, late blight, powdery mildew, downy mildew, and the like; viral diseases such as sugarcane mosaic, cassava mosaic, sugar beet yellows, plum pox, barley yellow dwarf, tomato yellow leaf curl, tomato spotted wilt virus, and the like; bacterial diseases such as citrus canker, bacterial leaf blight, bacterial will, soft rot of vegetables, and the like; nematode diseases such as root knot, sugar beet cyst nematode or the like.

### EXAMPLE IX. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919).

Identified *Arabidopsis* homologous sequences are provided in Figure 2 and included in the Sequence Listing. The percent sequence identity among these sequences is as low as 47% sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*). These sequences were compared to sequences representing genes of SEQ IDs Nos. 1-58 on 9/26/2000 using the Washington University TBLASTX algorithm (version 2.0a19MP). For each gene of SEQ IDs Nos. 1-58, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 3.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 1: 38%-76%; SEQ ID No. 3: 36%-72%; SEQ ID No. 5: 51%-75%; SEQ ID No. 7: 37%-76%; SEQ ID No. 9: 48%-75%; SEQ ID No. 11: 31%-68%; SEQ ID No. 13: 59%-81%; SEQ ID No. 15: 49%-81%; SEQ ID No. 17: 53%-87%; SEQ ID No. 19: 48%-84%; SEQ ID No. 21: 73%-89%; SEQ ID No. 23: 52%-64%; SEQ ID No. 25: 48%-83%; SEQ ID No. 27: 35%-92%; SEQ ID No. 29: 56%-89%; SEQ ID No. 31: 50%-90%; SEQ ID No. 33: 50%-93%; SEQ ID No. 35: 52%-81%; SEQ ID No. 37: 75%-81%; SEQ ID No. 39: 35%-72%; SEQ ID No. 41: 55%-89%; SEQ ID No. 43: 56%-77%; SEQ ID No. 45: 34%-72%; SEQ ID No. 47: 51%-86%; SEQ ID No. 49: 46%-86%; SEQ ID No. 51: 58%-80%; SEQ ID No. 53: 46%-55%; SEQ ID No. 55: 84%-89%; and SEQ ID No. 57: 43%-71%.

The polynucleotides and polypeptides in the Sequence Listing and the identified homologous sequences may be stored in a computer system and have associated or linked with the sequences a function, such as that the polynucleotides and polypeptides are useful for modifying the pathogen tolerance of a plant.

## Claims

1. A method for producing a transgenic plant having an improved tolerance or resistance to a biotropic or necrotropic fungal pathogen, the method comprising:
transforming a plant or part thereof with a nucleotide sequence which encodes a transcription factor polypeptide having at least 75% sequence identity to the polypeptide of SEQ ID NO:10, wherein said polypeptide also has a conserved domain having at least 80% sequence identity to amino acids 145-213 of SEQ ID NO:10,
wherein expression of the transcription factor polypeptide in the transgenic plant increases the transgenic plant's tolerance or resistance to a biotropic or necrotropic fungal pathogen compared to a wild-type plant, and
selecting a transgenic plant that expresses said nucleotide sequence and has said improved pathogen tolerance or resistance thereby providing the transgenic plant with said improved tolerance or resistance to a biotropic or necrotropic fungal pathogen compared to a wild-type plant.

2. The method of claim 1, wherein the polynucleotide is a polynucleotide that encodes a polypeptide having at least 90% identity to SEQ ID NO:10.

3. The method of claim 1 or 2, wherein the biotropic fungal pathogen is *Erisyphe orontii.*

4. The method of any one of claims 1 to 3 wherein a constitutive, inducible, or tissue-active promoter is operably linked to said nucleotide sequence.

5. The method of any one of claims 1 to 4 wherein the plant is selected from the group consisting of soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

## Patentansprüche

1. Verfahren zur Herstellung einer transgenen Pflanze mit verbesserter Toleranz oder Resistenz gegenüber einem biotrophen oder nekrotrophen Pilzpathogen, wobei das Verfahren Folgendes umfasst:
Transformieren einer Pflanze oder eines Teils davon mit einer Nucleotidsequenz, die für ein Transkriptionsfaktorpolypeptid mit zumindest 75 % Sequenzidentität mit dem Polypeptid der Seq.-ID Nr. 10 kodiert, wobei das Polypeptid auch eine konservierte Domäne mit zumindest 80 % Sequenzidentität mit den Aminosäuren 145-213 der Seq.-ID Nr. 10 aufweist,
wobei die Expression des Transkriptionsfaktorpolypeptids in der transgenen Pflanze die Toleranz oder Resistenz der transgenen Pflanze gegenüber einem biotrophen oder nekrotrophen Pilzpathogen im Vergleich zu einer Wildtyppflanze erhöht, und
Selektieren einer transgenen Pflanze, welche die Nucleotidsequenz exprimiert und die verbesserte Pathogentoleranz oder -resistenz aufweist, wodurch die transgene Pflanze mit der verbesserten Toleranz oder Resistenz gegenüber einem biotrophen oder nekrotrophen Pilzpathogen im Vergleich zu einer Wildtyppflanze bereitgestellt wird.

2. Verfahren nach Anspruch 1, wobei das Polynucleotid ein Polynucleotid ist, das für ein Polypeptid mit zumindest 90 % Identität mit Seq.-ID Nr. 10 kodiert.

3. Verfahren nach Anspruch 1 oder 2, wobei das biotrophe Pilzpathogen *Erisyphe orontii* ist.

4. Verfahren nach einem der Ansprüche 1 bis 3, wobei ein konstitutiver, induzierbarer oder gewebeaktiver Promotor operabel mit der Nucleotidsequenz verbunden ist.

5. Verfahren nach einem der Ansprüche 1 bis 4, wobei die Pflanze aus der aus Sojabohne, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Weintraube, Honigmelone, Kopfsalat, Mango, Melone, Zwiebel, Papaya, Erbse, Paprika, Ananas, Spinat, Kürbis, Zuckermais, Tabak, Tomate, Wassermelone, Obstrosengewächse und Gemüsekohl bestehenden Gruppe ausgewählt ist.

## Revendications

1. Procédé de production d'une plante transgénique d'une plus grande tolérance ou résistance à un pathogène fongique biotropique ou nécrotropique, le procédé comprenant:
transformer une plante ou une partie de celle-ci avec une séquence de nucléotides qui code pour un polypeptide facteur de transcription ayant au moins 75% d'identité de séquence avec le polypeptide de la SEQ ID NO: 10, où ledit polypeptide a également un domaine conservé ayant au moins 80% d'identité de séquence aux acides aminés 145-213 de la SEQ ID NO: 10,
où l'expression du polypeptide facteur de transcription dans la plante transgénique augmente la tolérance ou résistance de la plante transgénique à un pathogène fongique biotropique ou nécrotropique en comparaison à une plante de type sauvage, et
sélectionner une plante transgénique qui exprime ladite séquence de nucléotides et qui a ladite plus grande tolérance ou résistance au pathogène en réalisant ainsi la plante transgénique avec ladite plus grande tolérance ou résistance à un pathogène fongique biotropique ou nécrotropique comparée à une plante de type sauvage.

2. Procédé selon la revendication 1, où le polynucléotide est un polynucléotide qui code pour un polypeptide ayant au moins 90% d'identité avec la SEQ ID NO:10.

3. Procédé selon la revendication 1 ou 2, où le pathogène fongique biotropique est *Erisyphe orontii.*

4. Procédé selon l'une quelconque des revendications 1 à 3, où un promoteur constitutif, inductible ou actif tissulaire est fonctionnellement lié à ladite séquence de nucléotides.

5. Procédé selon l'une quelconque des revendications 1 à 4, où la plante est sélectionnée dans le groupe consistant en soja, blé, maïs, pomme de terre, coton, riz, sémences de colza, tournesol, alfalfa, canne à sucre, gazon, bananes, mûres, myrtilles, fraises, framboises, cantaloups, carottes, chou-fleur, café, concombre, aubergine, raisins, miellée, laitue, mangue, melon, oignon, papayes, petits pois, poivres, ananas, épinards, courge, maïs sucré, tabac, tomates, pastèque, fruits de rosacées et brassicacées de légumes.


### SEQUENCE LISTING

<110> Heard, Jacqueline Keddie, James Yu, Guo-Liang Ratcliffe, Oliver Creelman, Robert Jiang, Cai-zhong Pineda, Omaira Reuber, Lynne Adam, Luc
<120> Pathogen Resistance Genes
<130> MBI-0015
<150> 60/166,228
   <151> 1999-11-17
<150> Plant Trait Modification III
   <151> 2000-08-22
<150> 60/197,899
   <151> 2000-04-17
<160> 58
<170> PatentIn version 3.0
<210> 1
   <211> 1187
   <212> DNA
   <213> Arabidopsis thaliana
<220>
   <221> CDS
   <222> (50)..(1096)
   <223> G188
<400> 1
   ctctcaccaa cataatcaaa gaagctttcc tcacgaattc aagatcgcc atg tcc tcc      58
                                                         Met Ser Ser
                                                         1
   gag gat tgg gat ctc ttc gcc gtc gtc aga agc tgc agc tct tct gtt      106
   Glu Asp Trp Asp Leu Phe Ala Val Val Arg Ser Cys Ser Ser Ser Val
       5                   10                  15
   tcc acc acc aat tct tgt gct ggt cat gaa gac gac ata gga aac tgt      154
   Ser Thr Thr Asn Ser Cys Ala Gly His Glu Asp Asp Ile Gly Asn Cys
   20                  25                  30                  35
   aaa caa caa caa gat cct cct cct cct cct ctg ttt caa gct tct tct      202
   Lys Gln Gln Gln Asp Pro Pro Pro Pro Pro Leu Phe Gln Ala Ser Ser
                   40                  45                  50
   tct tgc aac gag tta caa gat tct tgc aaa cca ttt tta ccc gtt act      250
   Ser Cys Asn Glu Leu Gln Asp Ser Cys Lys Pro Phe Leu Pro Val Thr
               55                  60                  65
   act act act act act act tgg tct cct cct cct cta ctt cct cct cct      298
   Thr Thr Thr Thr Thr Thr Trp Ser Pro Pro Pro Leu Leu Pro Pro Pro
           70                  75                  80
   aaa gcc tca tca cca tct ccc aat atc tta cta aaa caa gaa caa gta      346
   Lys Ala Ser Ser Pro Ser Pro Asn Ile Leu Leu Lys Gln Glu Gln Val
       85                  90                  95
   ctt ctc gaa tca caa gat caa aaa cct cct ctt agt gtt agg gtt ttc      394
   Leu Leu Glu Ser Gln Asp Gln Lys Pro Pro Leu Ser Val Arg Val Phe
   100                 105                 110                 115
   cca cca tcc act tct tct tct gtc ttt gtt ttt aga ggt caa cgc gac      442
   Pro Pro Ser Thr Ser Ser Ser Val Phe Val Phe Arg Gly Gln Arg Asp
                   120                     125                     130
   cag ctt ctt caa caa caa tcc caa cct ccc ctt cga tct aga aaa aga      490
   Gln Leu Leu Gln Gln Gln Ser Gln Pro Pro Leu Arg Ser Arg Lys Arg
               135                 140                 145
   aag aat cag caa aaa aga acc ata tgt cat gta acg caa gag aat ctt      538
   Lys Asn Gln Gln Lys Arg Thr Ile Cys His Val Thr Gln Glu Asn Leu
           150                 155                 160
   tct tct gat ttg tgg gct tgg cgt aaa tac ggt caa aaa ccc atc aaa      586
   Ser Ser Asp Leu Trp Ala Trp Arg Lys Tyr Gly Gln Lys Pro Ile Lys
       165                 170                 175
   ggc tct cct tat cca agg aat tat tac aga tgt agt agc tca aaa gga      634
   Gly Ser Pro Tyr Pro Arg Asn Tyr Tyr Arg Cys Ser Ser Ser Lys Gly
   180                 185                 190                 195
   tgt tta gca cga aaa caa gtt gaa aga agt aat tta gat cct aat atc      682
   Cys Leu Ala Arg Lys Gln Val Glu Arg Ser Asn Leu Asp Pro Asn Ile
                   200                 205                 210
   ttc atc gtt act tac acc gga gaa cac act cat cca cgt cct act cac      730
   Phe Ile Val Thr Tyr Thr Gly Glu His Thr His Pro Arg Pro Thr His
               215                 220                 225
   cgg aac tct ctc gcc gga agt act cgt aac aaa tct cag ccc gtt aac      778
   Arg Asn Ser Leu Ala Gly Ser Thr Arg Asn Lys Ser Gln Pro Val Asn
           230                 235                 240
   ccg gtt cct aaa ccg gac aca tct cct tta tcg gat aca gta aaa gaa      826
   Pro Val Pro Lys Pro Asp Thr Ser Pro Leu Ser Asp Thr Val Lys Glu
       245                 250                 255
   gag att cat ctt tct ccg acg aca ccg ttg aaa gga aac gat gac gtt      874
   Glu Ile His Leu Ser Pro Thr Thr Pro Leu Lys Gly Asn Asp Asp Val
   260                 265                 270                 275
   caa gaa acg aat gga gat gaa gat atg gtt ggt caa gaa gtc aac atg      922
   Gln Glu Thr Asn Gly Asp Glu Asp Met Val Gly Gln Glu Val Asn Met
                   280                 285                 290
   gaa gag gaa gag gag gaa gaa gaa gtg gaa gaa gat gat gaa gaa gaa      970
   Glu Glu Glu Glu Glu Glu Glu Glu Val Glu Glu Asp Asp Glu Glu Glu
               295                 300                 305
   gaa gat gat gat gac gtg gat gat ctt ttg ata cca aat tta gcg gtg     1018
   Glu Asp Asp Asp Asp Val Asp Asp Leu Leu Ile Pro Asn Leu Ala Val
           310                 315                 320
   aga gat cga gat gat ttg ttc ttc gct gga agt ttt cca tct tgg tcc     1066
   Arg Asp Arg Asp Asp Leu Phe Phe Ala Gly Ser Phe Pro Ser Trp Ser
       325                 330                 335
   gcc gga tcc gcc ggt gac ggt ggt gga tga tgaaaacgaa taaaatctca      1116
   Ala Gly Ser Ala Gly Asp Gly Gly Gly
   340                 345
   atttacaatt tacaaaaaga aaaaagtcag tttttaatta ttatttttgt ttgttaaaac   1176
   ttgacattta t                                                        1187
<210> 2
   <211> 348
   <212> PRT
   <213> Arabidopsis thaliana
<400> 2
   Met Ser Ser Glu Asp Trp Asp Leu Phe Ala Val Val Arg Ser Cys Ser
   1               5                   10                  15
   Ser Ser Val Ser Thr Thr Asn Ser Cys Ala Gly His Glu Asp Asp Ile
   20 25 30
   Gly Asn Cys Lys Gln Gln Gln Asp Pro Pro Pro Pro Pro Leu Phe Gln
   35 40 45
   Ala Ser Ser Ser Cys Asn Glu Leu Gln Asp Ser Cys Lys Pro Phe Leu
   50 55 60
   Pro Val Thr Thr Thr Thr Thr Thr Thr Trp Ser Pro Pro Pro Leu Leu
   65 70 75 80
   Pro Pro Pro Lys Ala Ser Ser Pro Ser Pro Asn Ile Leu Leu Lys Gln
   85 90 95
   Glu Gln Val Leu Leu Glu Ser Gln Asp Gln Lys Pro Pro Leu Ser Val
   100 105 110
   Arg Val Phe Pro Pro Ser Thr Ser Ser Ser Val Phe Val Phe Arg Gly
   115 120 125
   Gln Arg Asp Gln Leu Leu Gln Gln Gln Ser Gln Pro Pro Leu Arg Ser
   130 135 140
   Arg Lys Arg Lys Asn Gln Gln Lys Arg Thr Ile Cys His Val Thr Gln
   145 150 155 160
   Glu Asn Leu Ser Ser Asp Leu Trp Ala Trp Arg Lys Tyr Gly Gln Lys
   165 170 175
   Pro Ile Lys Gly Ser Pro Tyr Pro Arg Asn Tyr Tyr Arg Cys Ser Ser
   180 185 190
   Ser Lys Gly Cys Leu Ala Arg Lys Gln Val Glu Arg Ser Asn Leu Asp
   195 200 205
   Pro Asn Ile Phe Ile Val Thr Tyr Thr Gly Glu His Thr His Pro Arg
   210 215 220
   Pro Thr His Arg Asn Ser Leu Ala Gly Ser Thr Arg Asn Lys Ser Gln
   225 230 235 240
   Pro Val Asn Pro Val Pro Lys Pro Asp Thr Ser Pro Leu Ser Asp Thr
   245 250 255
   Val Lys Glu Glu Ile His Leu Ser Pro Thr Thr Pro Leu Lys Gly Asn
   260 265 270
   Asp Asp Val Gln Glu Thr Asn Gly Asp Glu Asp Met Val Gly Gln Glu
   275 280 285
   Val Asn Met Glu Glu Glu Glu Glu Glu Glu Glu Val Glu Glu Asp Asp
   290 295 300
   Glu Glu Glu Glu Asp Asp Asp Asp Val Asp Asp Leu Leu Ile Pro Asn
   305 310 315 320
   Leu Ala Val Arg Asp Arg Asp Asp Leu Phe Phe Ala Gly Ser Phe Pro
                   325                 330                 335
   Ser Trp Ser Ala Gly Ser Ala Gly Asp Gly Gly Gly
               340                 345
<210> 3
   <211> 1431
   <212> DNA
   <213> Arabidopsis thaliana
<220>
   <221> CDS
   <222> (129)..(1211)
   <223> G616
<400> 3
   ctttaaatcc caaaccaacc ctaaagtttt gatttttaat tttgggggta accaaaaaaa      60
   aaacaaaacc ctaatttttt ttctttagtg atgagattat tggtgatgat gaaatgattg     120
   gagatcta atg aag aat aac aac aat ggc gac gtt gtg gat aac gaa gtg     170
            Met Lys Asn Asn Asn Asn Gly Asp Val Val Asp Asn Glu Val
            1               5                   10
   aac aac cgg tta agc cgg tgg cat cac aat tct tcc cgg ata att agg      218
   Asn Asn Arg Leu Ser Arg Trp His His Asn Ser Ser Arg Ile Ile Arg
   15                  20                  25                  30
   gtt tca cga gct tcc ggt ggt aaa gat cga cac agc aaa gtc ttg act      266
   Val Ser Arg Ala Ser Gly Gly Lys Asp Arg His Ser Lys Val Leu Thr
                   35                  40                  45
   tct aaa gga cca cgt gac cgt cgt gtc cgg tta tca gtc tcc acc gct      314
   Ser Lys Gly Pro Arg Asp Arg Arg Val Arg Leu Ser Val Ser Thr Ala
               50                  55                  60
   ctt caa ttc tat gat ctt caa gat cgg tta ggt tat gat caa cct agc      362
   Leu Gln Phe Tyr Asp Leu Gln Asp Arg Leu Gly Tyr Asp Gln Pro Ser
           65                  70                  75
   aaa gct gtt gaa tgg tta atc aaa gct gct gaa gat tca atc tct gag      410
   Lys Ala Val Glu Trp Leu Ile Lys Ala Ala Glu Asp Ser Ile Ser Glu
       80                  85                  90
   ctt cct tca ctc aac aac act cat ttt ccg acc gat gac gag aat cac      458
   Leu Pro Ser Leu Asn Asn Thr His Phe Pro Thr Asp Asp Glu Asn His
   95                  100                 105                 110
   cag aat cag aca tta aca aca gtt gct gct aat tcc ttg tct aaa tct      506
   Gln Asn Gln Thr Leu Thr Thr Val Ala Ala Asn Ser Leu Ser Lys Ser
                   115                 120                 125
   gct tgt agt agc aat tca gac acg agc aag aac tct tct ggt ttg tct      554
   Ala Cys Ser Ser Asn Ser Asp Thr Ser Lys Asn Ser Ser Gly Leu Ser
               130                 135                 140
   tta tca aga tcg gag ctt aga gat aaa gct aga gag agg gct aga gag      602
   Leu Ser Arg Ser Glu Leu Arg Asp Lys Ala Arg Glu Arg Ala Arg Glu
           145                 150                 155
   aga aca gct aaa gag acc aag gaa aga gat cat aac cac act tcg ttt      650
   Arg Thr Ala Lys Glu Thr Lys Glu Arg Asp His Asn His Thr Ser Phe
       160                 165                 170
   acg gat ttg tta aat tcc ggt tca gat ccg gtt aac tca aac cgg caa      698
   Thr Asp Leu Leu Asn Ser Gly Ser Asp Pro Val Asn Ser Asn Arg Gln
   175                 180                 185                 190
   tgg atg gct tca gct cct tct tca tct cca atg gag tat ttc agt tcg      746
   Trp Met Ala Ser Ala Pro Ser Ser Ser Pro Met Glu Tyr Phe Ser Ser
                   195                 200                 205
   ggt tta att ctc ggg tcg ggt caa caa acc cat ttc cct att tca aca      794
   Gly Leu Ile Leu Gly Ser Gly Gln Gln Thr His Phe Pro Ile Ser Thr
               210                 215                 220
   aat tct cat cct ttc tca tca atc tcc gat cat cat cat cat cat cct      842
   Asn Ser His Pro Phe Ser Ser Ile Ser Asp His His His His His Pro
           225                 230                 235
   cat cat cag cat caa gag ttt tca ttc gtt ccc gac cat ttg ata tca      890
   His His Gln His Gln Glu Phe Ser Phe Val Pro Asp His Leu Ile Ser
       240                 245                 250
   ccg gca gaa tcc aac ggc gga gca ttc aat ctt gat ttt aat atg tca      938
   Pro Ala Glu Ser Asn Gly Gly Ala Phe Asn Leu Asp Phe Asn Met Ser
   255                 260                 265                 270
   aca ccc tcc ggc gcc gga gct gcc gtc tcc gcc gca tca ggt ggt ggc      986
   Thr Pro Ser Gly Ala Gly Ala Ala Val Ser Ala Ala Ser Gly Gly Gly
                   275                 280                 285
   ttc agt ggt ttc aac agg ggg acc ctt cag tcc aat tca aca aat cag     1034
   Phe Ser Gly Phe Asn Arg Gly Thr Leu Gln Ser Asn Ser Thr Asn Gln
               290                 295                 300
   cat cag tca ttc ctc gct aat cta cag agg ttt cca aca tca gaa agt     1082
   His Gln Ser Phe Leu Ala Asn Leu Gln Arg Phe Pro Thr Ser Glu Ser
           305                 310                 315
   gga gga ggt cca cag ttc tta ttc ggt gca ctg cct gca gag aat cac     1130
   Gly Gly Gly Pro Gln Phe Leu Phe Gly Ala Leu Pro Ala Glu Asn His
       320                 325                 330
   cac cac aat cac cag ttt cag ctt tac tat gaa aat gga tgc aga aac     1178
   His His Asn His Gln Phe Gln Leu Tyr Tyr Glu Asn Gly Cys Arg Asn
   335                 340                 345                 350
   tca tca gaa cat aag ggt aaa ggc aag aac tga tgatattaat tattgcatct   1231
   Ser Ser Glu His Lys Gly Lys Gly Lys Asn
                   355                 360
   ttggttttgt tcaaatgctc attttgtatg tttatctttg gtttatttca aaacaaatgt    1291
   taatctcttt cgttgtctga tgtgtgttag ggttttgttt tatgtattga gggtctttgg   1351
   aaatcttttt gcattgtgct tgtaatgttg tatttgtgat aatagcattt tgtttgtgag   1411
   ttaaaaaaaa aaaaaaaaaa                                               1431
<210> 4
   <211> 360
   <212> PRT
   <213> Arabidopsis thaliana
<400> 4
   Met Lys Asn Asn Asn Asn Gly Asp Val Val Asp Asn Glu Val Asn Asn
   1               5                   10                  15
   Arg Leu Ser Arg Trp His His Asn Ser Ser Arg Ile Ile Arg Val Ser
               20                  25                  30
   Arg Ala Ser Gly Gly Lys Asp Arg His Ser Lys Val Leu Thr Ser Lys
           35                  40                  45
   Gly Pro Arg Asp Arg Arg Val Arg Leu Ser Val Ser Thr Ala Leu Gln
       50                  55                  60
   Phe Tyr Asp Leu Gln Asp Arg Leu Gly Tyr Asp Gln Pro Ser Lys Ala
   65                  70                  75                  80
   Val Glu Trp Leu Ile Lys Ala Ala Glu Asp Ser Ile Ser Glu Leu Pro
                   85                  90                  95
   Ser Leu Asn Asn Thr His Phe Pro Thr Asp Asp Glu Asn His Gln Asn
               100                 105                 110
   Gln Thr Leu Thr Thr Val Ala Ala Asn Ser Leu Ser Lys Ser Ala Cys
           115                 120                 125
   Ser Ser Asn Ser Asp Thr Ser Lys Asn Ser Ser Gly Leu Ser Leu Ser
       130                 135                 140
   Arg Ser Glu Leu Arg Asp Lys Ala Arg Glu Arg Ala Arg Glu Arg Thr
   145                 150                 155                 160
   Ala Lys Glu Thr Lys Glu Arg Asp His Asn His Thr Ser Phe Thr Asp
                   165                 170                 175
   Leu Leu Asn Ser Gly Ser Asp Pro Val Asn Ser Asn Arg Gln Trp Met
               180                 185                 190
   Ala Ser Ala Pro Ser Ser Ser Pro Met Glu Tyr Phe Ser Ser Gly Leu
           195                 200                 205
   Ile Leu Gly Ser Gly Gln Gln Thr His Phe Pro Ile Ser Thr Asn Ser
       210                 215                 220
   His Pro Phe Ser Ser Ile Ser Asp His His His His His Pro His His
   225                 230                 235                 240
   Gln His Gln Glu Phe Ser Phe Val Pro Asp His Leu Ile Ser Pro Ala
                   245                 250                 255
   Glu Ser Asn Gly Gly Ala Phe Asn Leu Asp Phe Asn Met Ser Thr Pro
               260                 265                 270
   Ser Gly Ala Gly Ala Ala Val Ser Ala Ala Ser Gly Gly Gly Phe Ser
           275                 280                 285
   Gly Phe Asn Arg Gly Thr Leu Gln Ser Asn Ser Thr Asn Gln His Gln
       290                 295                 300
   Ser Phe Leu Ala Asn Leu Gln Arg Phe Pro Thr Ser Glu Ser Gly Gly
   305                 310                 315                 320
   Gly Pro Gln Phe Leu Phe Gly Ala Leu Pro Ala Glu Asn His His His
                   325                 330                 335
   Asn His Gln Phe Gln Leu Tyr Tyr Glu Asn Gly Cys Arg Asn Ser Ser
               340                 345                 350
   Glu His Lys Gly Lys Gly Lys Asn
           355                 360
<210> 5
   <211> 1055
   <212> DNA
   <213> Arabidopsis thaliana
<220>
   <221> CDS
   <222> (70)..(816)
   <223> G19
<400> 5
   ataaaggcat ttcagctcca ccgtaggaaa ctttctcttg aaagaaaccc acagcaacaa      60
   acagagaaa atg tgt ggc ggt gct att att tcc gat tat gcc cct ctc gtc     111
             Met Cys Gly Gly Ala Ile Ile Ser Asp Tyr Ala Pro Leu Val
             1               5                   10
   acc aag gcc aag ggc cgt aaa ctc acg gct gag gaa ctc tgg tca gag      159
   Thr Lys Ala Lys Gly Arg Lys Leu Thr Ala Glu Glu Leu Trp Ser Glu
   15                  20                  25                  30
   ctc gat gct tcc gcc gcc gac gac ttc tgg ggt ttc tat tcc acc tcc      207
   Leu Asp Ala Ser Ala Ala Asp Asp Phe Trp Gly Phe Tyr Ser Thr Ser
                   35                  40                  45
   aaa ctc cat ccc acc aac caa gtt aac gtg aaa gag gag gca gtg aag      255
   Lys Leu His Pro Thr Asn Gln Val Asn Val Lys Glu Glu Ala Val Lys
               50                  55                  60
   aag gag cag gca aca gag ccg ggg aaa cgg agg aag agg aag aat gtt      303
   Lys Glu Gln Ala Thr Glu Pro Gly Lys Arg Arg Lys Arg Lys Asn Val
           65                  70                  75
   tat aga ggg ata cgt aag cgt cca tgg gga aaa tgg gcg gct gag att      351
   Tyr Arg Gly Ile Arg Lys Arg Pro Trp Gly Lys Trp Ala Ala Glu Ile
       80                  85                  90
   cga gat cca cga aaa ggt gtt aga gtt tgg ctt ggt acg ttc aac acg      399
   Arg Asp Pro Arg Lys Gly Val Arg Val Trp Leu Gly Thr Phe Asn Thr
   95                  100                 105                 110
   gcg gag gaa gct gcc atg gct tat gat gtt gcg gcc aag cag atc cgt      447
   Ala Glu Glu Ala Ala Met Ala Tyr Asp Val Ala Ala Lys Gln Ile Arg
                   115                 120                 125
   ggt gat aaa gcc aag ctc aac ttc cca gat ctg cac cat cct cct cct      495
   Gly Asp Lys Ala Lys Leu Asn Phe Pro Asp Leu His His Pro Pro Pro
               130                 135                 140
   cct aat tat act cct ccg ccg tca tcg cca cga tca acc gat cag cct      543
   Pro Asn Tyr Thr Pro Pro Pro Ser Ser Pro Arg Ser Thr Asp Gln Pro
           145                 150                 155
   ccg gcg aag aag gtc tgc gtt gtc tct cag agt gag agc gag tta agt      591
   Pro Ala Lys Lys Val Cys Val Val Ser Gln Ser Glu Ser Glu Leu Ser
       160                 165                 170
   cag ccg agt ttc ccg gtg gag tgt ata gga ttt gga aat ggg gac gag      639
   Gln Pro Ser Phe Pro Val Glu Cys Ile Gly Phe Gly Asn Gly Asp Glu
   175                 180                 185                 190
   ttt cag aac ctg agt tac gga ttt gag ccg gat tat gat ctg aaa cag      687
   Phe Gln Asn Leu Ser Tyr Gly Phe Glu Pro Asp Tyr Asp Leu Lys Gln
                   195                 200                 205
   cag ata tcg agc ttg gaa tcg ttc ctt gag ctg gac ggt aac acg gcg      735
   Gln Ile Ser Ser Leu Glu Ser Phe Leu Glu Leu Asp Gly Asn Thr Ala
               210                 215                 220
   gag caa ccg agt cag ctt gat gag tcc gtt tcc gag gtg gat atg tgg      783
   Glu Gln Pro Ser Gln Leu Asp Glu Ser Val Ser Glu Val Asp Met Trp
           225                 230                 235
   atg ctt gat gat gtc att gcg tcg tat gag taa aagaaaaaaa ataagtttaa    836
   Met Leu Asp Asp Val Ile Ala Ser Tyr Glu
   240 245
   aaaaagttaa ataaagtctg taatatatat gtaaccgccg ttacttttaa aaggttttta 896
   ccgtcgcatt ggactgctga tgatgtctgt tgtgtaatgt gtagaatgtg accaaatgga 956
   cgttatatta cggtttgtgg tattattagt ttcttagatg gaaaaactta catgtgtaaa 1016
   taagatttgt aatgtaagac gaagtactta taacttctt 1055
<210> 6
   <211> 248
   <212> PRT
   <213> Arabidopsis thaliana
<400> 6
   Met Cys Gly Gly Ala Ile Ile Ser Asp Tyr Ala Pro Leu Val Thr Lys
   1               5                   10                  15
   Ala Lys Gly Arg Lys Leu Thr Ala Glu Glu Leu Trp Ser Glu Leu Asp
               20                  25                  30
   Ala Ser Ala Ala Asp Asp Phe Trp Gly Phe Tyr Ser Thr Ser Lys Leu
           35                  40                  45
   His Pro Thr Asn Gln Val Asn Val Lys Glu Glu Ala Val Lys Lys Glu
       50                  55                  60
   Gln Ala Thr Glu Pro Gly Lys Arg Arg Lys Arg Lys Asn Val Tyr Arg
   65                  70                  75                  80
   Gly Ile Arg Lys Arg Pro Trp Gly Lys Trp Ala Ala Glu Ile Arg Asp
                   85                  90                  95
   Pro Arg Lys Gly Val Arg Val Trp Leu Gly Thr Phe Asn Thr Ala Glu
               100                 105                 110
   Glu Ala Ala Met Ala Tyr Asp Val Ala Ala Lys Gln Ile Arg Gly Asp
           115                 120                 125
   Lys Ala Lys Leu Asn Phe Pro Asp Leu His His Pro Pro Pro Pro Asn
       130                 135                 140
   Tyr Thr Pro Pro Pro Ser Ser Pro Arg Ser Thr Asp Gln Pro Pro Ala
   145                 150                 155                 160
   Lys Lys Val Cys Val Val Ser Gln Ser Glu Ser Glu Leu Ser Gln Pro
                   165                 170                 175
   Ser Phe Pro Val Glu Cys Ile Gly Phe Gly Asn Gly Asp Glu Phe Gln
               180                 185                 190
   Asn Leu Ser Tyr Gly Phe Glu Pro Asp Tyr Asp Leu Lys Gln Gln Ile
           195                 200                 205
   Ser Ser Leu Glu Ser Phe Leu Glu Leu Asp Gly Asn Thr Ala Glu Gln
       210                 215                 220
   Pro Ser Gln Leu Asp Glu Ser Val Ser Glu Val Asp Met Trp Met Leu
   225                 230                 235                 240
   Asp Asp Val Ile Ala Ser Tyr Glu
                   245
<210> 7
   <211> 1857
   <212> DNA
   <213> Arabidopsis thaliana
<220>
   <221> CDS
   <222> (458)..(1663)
   <223> G261
<400> 7
   gtttaggttc gagaagcaga gagggttcga gaagctaata agggtttctt ctttttgatt      60
   ttaatgctaa aagggttcta gattcgttga attttacaag ggttttaggg gttcttagaa     120
   gcttttgctt gattgtcttt tatttagaaa cagtggtgag tttttagtct ttcactttgt     180
   tcaagttcga agcttttttt ggagggaatt ttgggcttct gattttgatc gaaacttact     240
   gatagtaagt tctttgagtc ctccttaact gtagtttctg tgtactgaag ttattgaatt     300
   gaaagttttt atcttttttg gttattgaaa ctttcatagt ttgatcaaaa gagtctcttg     360
   ctctgttttt ggctctgttt ttgtgagtgt gattgtaagc tttgttgtga gtagattgaa     420
   tcaaggagtg tgagagttgt taaaagtgtt ttcagag atg gat gag aat aat cat      475
                                            Met Asp Glu Asn Asn His
                                            1               5
   gga gtt tca tca agc tca ctt cca cct ttc ctc acc aaa aca tat gag      523
   Gly Val Ser Ser Ser Ser Leu Pro Pro Phe Leu Thr Lys Thr Tyr Glu
               10                  15                  20
   atg gtt gat gat tct tca tcc gat tct atc gtc tct tgg agt cag agc      571
   Met Val Asp Asp Ser Ser Ser Asp Ser Ile Val Ser Trp Ser Gln Ser
           25                  30                  35
   aat aag agt ttc atc gtt tgg aat ccg ccg gag ttt tct aga gat ctt      619
   Asn Lys Ser Phe Ile Val Trp Asn Pro Pro Glu Phe Ser Arg Asp Leu
       40                  45                  50
   ctt ccg aga ttc ttc aag cac aat aac ttc tct agc ttt atc cgc cag      667
   Leu Pro Arg Phe Phe Lys His Asn Asn Phe Ser Ser Phe Ile Arg Gln
   55                  60                  65                  70
   ctt aac aca tat ggt ttt aga aaa gct gat cct gag caa tgg gaa ttt      715
   Leu Asn Thr Tyr Gly Phe Arg Lys Ala Asp Pro Glu Gln Trp Glu Phe
                   75                  80                  85
   gcg aat gat gat ttt gtg aga ggt caa cct cat ctt atg aag aac att      763
   Ala Asn Asp Asp Phe Val Arg Gly Gln Pro His Leu Met Lys Asn Ile
               90                  95                  100
   cat aga cgc aaa cca gtt cat agc cac tct tta ccg aat ctt caa gct      811
   His Arg Arg Lys Pro Val His Ser His Ser Leu Pro Asn Leu Gln Ala
           105                 110                 115
   cag tta aac ccg ttg acg gat tca gaa cga gtg aga atg aat aat cag      859
   Gln Leu Asn Pro Leu Thr Asp Ser Glu Arg Val Arg Met Asn Asn Gln
       120                 125                 130
   att gag aga ttg aca aaa gag aaa gaa gga ttg ctt gaa gag tta cat      907
   Ile Glu Arg Leu Thr Lys Glu Lys Glu Gly Leu Leu Glu Glu Leu His
   135                 140                 145                 150
   aaa caa gac gag gaa cga gaa gtg ttt gag atg caa gtg aaa gaa ctt      955
   Lys Gln Asp Glu Glu Arg Glu Val Phe Glu Met Gln Val Lys Glu Leu
                   155                     160                     165
   aaa gaa cga tta caa cac atg gag aag cgt cag aaa aca atg gtt tcg      1003
   Lys Glu Arg Leu Gln His Met Glu Lys Arg Gln Lys Thr Met Val Ser
               170                     175                     180
   ttt gtt tct caa gta ttg gaa aag cca ggg ctt gct ttg aac cta tcg      1051
   Phe Val Ser Gln Val Leu Glu Lys Pro Gly Leu Ala Leu Asn Leu Ser
           185                     190                     195
   ccg tgt gtt ccc gaa aca aac gag agg aaa aga agg ttc cct agg atc      1099
   Pro Cys Val Pro Glu Thr Asn Glu Arg Lys Arg Arg Phe Pro Arg Ile
       200                     205                     210
   gag ttc ttt ccc gat gaa ccg atg ttg gaa gag aac aaa act tgt gtt      1147
   Glu Phe Phe Pro Asp Glu Pro Met Leu Glu Glu Asn Lys Thr Cys Val
   215                     220                     225                 230
   gtt gtg aga gag gaa ggt tct aca agc cct tct tca cac aca aga gag      1195
   Val Val Arg Glu Glu Gly Ser Thr Ser Pro Ser Ser His Thr Arg Glu
                   235                     240                     245
   cat caa gtg gaa cag tta gag tca tcg ata gcg att tgg gag aat ctt      1243
   His Gln Val Glu Gln Leu Glu Ser Ser Ile Ala Ile Trp Glu Asn Leu
               250                     255                     260
   gta tcg gat tct tgt gag agt atg tta caa tca aga agt atg atg aca      1291
   Val Ser Asp Ser Cys Glu Ser Met Leu Gln Ser Arg Ser Met Met Thr
           265                     270                     275
   ctt gat gtg gat gaa tca tct act ttt cca gag agc cct cct ctt tct      1339
   Leu Asp Val Asp Glu Ser Ser Thr Phe Pro Glu Ser Pro Pro Leu Ser
       280                     285                     290
   tgc ata cag tta agt gtc gat tca cgt ctc aaa tct cct cct tct cca      1387
   Cys Ile Gln Leu Ser Val Asp Ser Arg Leu Lys Ser Pro Pro Ser Pro
   295                     300                     305                 310
   agg atc atc gat atg aac tgt gag ccc gat ggt tcg aaa gaa cag aac      1435
   Arg Ile Ile Asp Met Asn Cys Glu Pro Asp Gly Ser Lys Glu Gln Asn
                   315                     320                     325
   act gtt gct gct cct cct cct cct cca gta gca gga gcg aat gat ggc      1483
   Thr Val Ala Ala Pro Pro Pro Pro Pro Val Ala Gly Ala Asn Asp Gly
               330                     335                     340
   ttc tgg cag cag ttt ttc tca gag aat cct ggc tca acc gag caa cgg      1531
   Phe Trp Gln Gln Phe Phe Ser Glu Asn Pro Gly Ser Thr Glu Gln Arg
           345                     350                     355
   gaa gtt caa tta gag agg aaa gac gat aaa gat aaa gcc gga gta cgt      1579
   Glu Val Gln Leu Glu Arg Lys Asp Asp Lys Asp Lys Ala Gly Val Arg
       360                     365                     370
   act gag aaa tgt tgg tgg aat tcg aga aat gtt aat gca att aca gaa      1627
   Thr Glu Lys Cys Trp Trp Asn Ser Arg Asn Val Asn Ala Ile Thr Glu
   375                     380                     385                 390
   cag ctt gga cat ctg act tct tca gag aga agt tga tatgtcaaag           1673
   Gln Leu Gly His Leu Thr Ser Ser Glu Arg Ser
                   395                     400
   attaaatttc tagtctgttt tagttacttg taaaataggg tttctcagtt ttattgtttt    1733
   cgattccagt acttaggtat ggttcagctg tttatttatc acttgtatga tctttcccag    1793
   ttcattgtag cagacttcaa tggtaatgat aagctagagc ttatggatag tattcataaa    1853
   aaaa                                                                 1857
<210> 8
   <211> 401
   <212> PRT
   <213> Arabidopsis thaliana
<400> 8
   Met Asp Glu Asn Asn His Gly Val Ser Ser Ser Ser Leu Pro Pro Phe
   1               5                   10                  15
   Leu Thr Lys Thr Tyr Glu Met Val Asp Asp Ser Ser Ser Asp Ser Ile
               20                  25                  30
   Val Ser Trp Ser Gln Ser Asn Lys Ser Phe Ile Val Trp Asn Pro Pro
           35                  40                  45
   Glu Phe Ser Arg Asp Leu Leu Pro Arg Phe Phe Lys His Asn Asn Phe
       50                  55                  60
   Ser Ser Phe Ile Arg Gln Leu Asn Thr Tyr Gly Phe Arg Lys Ala Asp
   65                  70                  75                  80
   Pro Glu Gln Trp Glu Phe Ala Asn Asp Asp Phe Val Arg Gly Gln Pro
                   85                  90                  95
   His Leu Met Lys Asn Ile His Arg Arg Lys Pro Val His Ser His Ser
               100                 105                 110
   Leu Pro Asn Leu Gln Ala Gln Leu Asn Pro Leu Thr Asp Ser Glu Arg
           115                 120                 125
   Val Arg Met Asn Asn Gln Ile Glu Arg Leu Thr Lys Glu Lys Glu Gly
       130                 135                 140
   Leu Leu Glu Glu Leu His Lys Gln Asp Glu Glu Arg Glu Val Phe Glu
   145                 150                 155                 160
   Met Gln Val Lys Glu Leu Lys Glu Arg Leu Gln His Met Glu Lys Arg
                   165                 170                 175
   Gln Lys Thr Met Val Ser Phe Val Ser Gln Val Leu Glu Lys Pro Gly
               180                 185                 190
   Leu Ala Leu Asn Leu Ser Pro Cys Val Pro Glu Thr Asn Glu Arg Lys
           195                 200                 205
   Arg Arg Phe Pro Arg Ile Glu Phe Phe Pro Asp Glu Pro Met Leu Glu
       210                 215                 220
   Glu Asn Lys Thr Cys Val Val Val Arg Glu Glu Gly Ser Thr Ser Pro
   225                 230                 235                 240
   Ser Ser His Thr Arg Glu His Gln Val Glu Gln Leu Glu Ser Ser Ile
                   245                 250                 255
   Ala Ile Trp Glu Asn Leu Val Ser Asp Ser Cys Glu Ser Met Leu Gln
               260                 265                 270
   Ser Arg Ser Met Met Thr Leu Asp Val Asp Glu Ser Ser Thr Phe Pro
           275                 280                 285
   Glu Ser Pro Pro Leu Ser Cys Ile Gln Leu Ser Val Asp Ser Arg Leu
       290                 295                 300
   Lys Ser Pro Pro Ser Pro Arg Ile Ile Asp Met Asn Cys Glu Pro Asp
   305                 310                 315                 320
   Gly Ser Lys Glu Gln Asn Thr Val Ala Ala Pro Pro Pro Pro Pro Val
                   325                 330                 335
   Ala Gly Ala Asn Asp Gly Phe Trp Gln Gln Phe Phe Ser Glu Asn Pro
               340                 345                 350
   Gly Ser Thr Glu Gln Arg Glu Val Gln Leu Glu Arg Lys Asp Asp Lys
           355                 360                 365
   Asp Lys Ala Gly Val Arg Thr Glu Lys Cys Trp Trp Asn Ser Arg Asn
       370                 375                 380
   Val Asn Ala Ile Thr Glu Gln Leu Gly His Leu Thr Ser Ser Glu Arg
   385                 390                 395                 400
   Ser
<210> 9
   <211> 964
   <212> DNA
   <213> Arabidopsis thaliana
<220>
   <221> CDS
   <222> (63)..(869)
   <223> G28
<400> 9
   gaaatctcaa caagaaccaa accaaacaac aaaaaaacat tcttaataat tatctttctg      60
   tt atg tcg atg acg gcg gat tct caa tct gat tat gct ttt ctt gag        107
      Met Ser Met Thr Ala Asp Ser Gln Ser Asp Tyr Ala Phe Leu Glu
      1               5                   10                  15
   tcc ata cga cga cac tta cta gga gaa tcg gag ccg ata ctc agt gag       155
   Ser Ile Arg Arg His Leu Leu Gly Glu Ser Glu Pro Ile Leu Ser Glu
                   20                  25                  30
   tcg aca gcg agt tcg gtt act caa tct tgt gta acc ggt cag agc att       203
   Ser Thr Ala Ser Ser Val Thr Gln Ser Cys Val Thr Gly Gln Ser Ile
               35                  40                  45
   aaa ccg gtg tac gga cga aac cct agc ttt agc aaa ctg tat cct tgc       251
   Lys Pro Val Tyr Gly Arg Asn Pro Ser Phe Ser Lys Leu Tyr Pro Cys
           50                  55                  60
   ttc acc gag agc tgg gga gat ttg ccg ttg aaa gaa aac gat tct gag       299
   Phe Thr Glu Ser Trp Gly Asp Leu Pro Leu Lys Glu Asn Asp Ser Glu
       65                  70                  75
   gat atg tta gtt tac ggt atc ctc aac gac gcc ttt cac ggc ggt tgg       347
   Asp Met Leu Val Tyr Gly Ile Leu Asn Asp Ala Phe His Gly Gly Trp
   80                  85                  90                  95
   gag ccg tct tct tcg tct tcc gac gaa gat cgt agc tct ttc ccg agt       395
   Glu Pro Ser Ser Ser Ser Ser Asp Glu Asp Arg Ser Ser Phe Pro Ser
                   100                 105                 110
   gtt aag atc gag act ccg gag agt ttc gcg gcg gtg gat tct gtt ccg       443
   Val Lys Ile Glu Thr Pro Glu Ser Phe Ala Ala Val Asp Ser Val Pro
               115                 120                 125
   gtc aag aag gag aag acg agt cct gtt tcg gcg gcg gtg acg gcg gcg       491
   Val Lys Lys Glu Lys Thr Ser Pro Val Ser Ala Ala Val Thr Ala Ala
           130                 135                 140
   aag gga aag cat tat aga gga gtg aga caa agg ccg tgg ggg aaa ttt       539
   Lys Gly Lys His Tyr Arg Gly Val Arg Gln Arg Pro Trp Gly Lys Phe
       145                 150                 155
   gcg gcg gag att aga gat ccg gcg aag aac gga gct agg gtt tgg tta       587
   Ala Ala Glu Ile Arg Asp Pro Ala Lys Asn Gly Ala Arg Val Trp Leu
   160                 165                 170                 175
   gga acg ttt gag acg gcg gag gac gcg gcg ttg gct tac gac aga gct       635
   Gly Thr Phe Glu Thr Ala Glu Asp Ala Ala Leu Ala Tyr Asp Arg Ala
                   180                 185                 190
   gct ttc agg atg cgt ggt tcc cgc gct ttg ttg aat ttt ccg ttg aga       683
   Ala Phe Arg Met Arg Gly Ser Arg Ala Leu Leu Asn Phe Pro Leu Arg
               195                 200                 205
   gtt aat tca gga gaa ccc gac ccg gtt cga atc aag tcc aag aga tct       731
   Val Asn Ser Gly Glu Pro Asp Pro Val Arg Ile Lys Ser Lys Arg Ser
           210                 215                 220
   tct ttt tct tct tct aac gag aac gga gct ccg aag aag agg aga acg       779
   Ser Phe Ser Ser Ser Asn Glu Asn Gly Ala Pro Lys Lys Arg Arg Thr
       225                 230                 235
   gtg gcc gcc ggt ggt gga atg gat aag gga ttg acg gtg aag tgc gag       827
   Val Ala Ala Gly Gly Gly Met Asp Lys Gly Leu Thr Val Lys Cys Glu
   240                 245                 250                 255
   gtt gtt gaa gtg gca cgt ggc gat cgt tta ttg gtt tta taa               869
   Val Val Glu Val Ala Arg Gly Asp Arg Leu Leu Val Leu
                   260                 265
   ttttgatttt tctttgttgg atgattatat gattcttcaa aaaagaagaa cgttaataaa      929
   aaaattcgtt tattattaaa aaaaaaaaaa aaaaa                                  964
<210> 10
   <211> 268
   <212> PRT
   <213> Arabidopsis thaliana
<400> 10
   Met Ser Met Thr Ala Asp Ser Gln Ser Asp Tyr Ala Phe Leu Glu Ser
   1               5                   10                  15
   Ile Arg Arg His Leu Leu Gly Glu Ser Glu Pro Ile Leu Ser Glu Ser
               20                  25                  30
   Thr Ala Ser Ser Val Thr Gln Ser Cys Val Thr Gly Gln Ser Ile Lys
           35                  40                  45
   Pro Val Tyr Gly Arg Asn Pro Ser Phe Ser Lys Leu Tyr Pro Cys Phe
       50                  55                  60
   Thr Glu Ser Trp Gly Asp Leu Pro Leu Lys Glu Asn Asp Ser Glu Asp
   65                  70                  75                  80
   Met Leu Val Tyr Gly Ile Leu Asn Asp Ala Phe His Gly Gly Trp Glu
                   85                  90                  95
   Pro Ser Ser Ser Ser Ser Asp Glu Asp Arg Ser Ser Phe Pro Ser Val
               100                 105                 110
   Lys Ile Glu Thr Pro Glu Ser Phe Ala Ala Val Asp Ser Val Pro Val
           115                 120                 125
   Lys Lys Glu Lys Thr Ser Pro Val Ser Ala Ala Val Thr Ala Ala Lys
       130                 135                 140
   Gly Lys His Tyr Arg Gly Val Arg Gln Arg Pro Trp Gly Lys Phe Ala
   145                 150                 155                 160
   Ala Glu Ile Arg Asp Pro Ala Lys Asn Gly Ala Arg Val Trp Leu Gly
                   165                 170                 175
   Thr Phe Glu Thr Ala Glu Asp Ala Ala Leu Ala Tyr Asp Arg Ala Ala
               180                 185                 190
   Phe Arg Met Arg Gly Ser Arg Ala Leu Leu Asn Phe Pro Leu Arg Val
           195                 200                 205
   Asn Ser Gly Glu Pro Asp Pro Val Arg Ile Lys Ser Lys Arg Ser Ser
       210                 215                 220
   Phe Ser Ser Ser Asn Glu Asn Gly Ala Pro Lys Lys Arg Arg Thr Val
   225                 230                 235                 240
   Ala Ala Gly Gly Gly Met Asp Lys Gly Leu Thr Val Lys Cys Glu Val
                   245                 250                 255
   Val Glu Val Ala Arg Gly Asp Arg Leu Leu Val Leu
               260                 265
<210> 11
   <211> 1571
   <212> DNA
   <213> Arabidopsis thaliana
<220>
   <221> CDS
   <222> (428)..(1402)
   <223> G869
<400> 11
   aggaacagtg aaaggttcgg ttttttgggt ttcgatctga taatcaacaa gaaaaaaggg      60
   tttgatttat gtcggctggg tttgaatcga ctgtgatttt gtctttgatt catatctctt     120
   ctccgatttc atcatcatct tccccatcat cgtcgtcttt gaaatcttgt cttctcaacg     180
   ctcttcactt ctgctgtaat aagcagaggc ttgttctgga gactccttct ctttccatgc     240
   gcttaagacc caaaaggact tgttctagtg ttgaagtctt tgggggtttt cacataaagc      300
   agcaaaagtt ttcttttttc atagttcgct gagagttttg agttttgata ccaaaaaagt     360
   tttgaccttt tagagtgatt ttttgttctt tctgttttct gggtattttt gaggagtggg     420
   tttaaca atg gtt gcg att aga aag gaa cag tct ttg agt ggt gtt agt       469
           Met Val Ala Ile Arg Lys Glu Gln Ser Leu Ser Gly Val Ser
           1               5                   10
   agc gag att aag aag aga gct aag aga aac act cta tcg tcc ctt cct       517
   Ser Glu Ile Lys Lys Arg Ala Lys Arg Asn Thr Leu Ser Ser Leu Pro
   15                  20                  25                  30
   caa gaa acc caa cct ttg agg aaa gtc cgt att att gtg aat gat cct      565
   Gln Glu Thr Gln Pro Leu Arg Lys Val Arg Ile Ile Val Asn Asp Pro
                   35                  40                  45
   tat gct act gat gat tcc tct agt gat gag gaa gag ctt aag gtt cct      613
   Tyr Ala Thr Asp Asp Ser Ser Ser Asp Glu Glu Glu Leu Lys Val Pro
               50                  55                  60
   aag cca agg aaa atg aaa cgt atc gtt cgt gag att aac ttt cct tct      661
   Lys Pro Arg Lys Met Lys Arg Ile Val Arg Glu Ile Asn Phe Pro Ser
           65                  70                  75
   atg gaa gtt tct gaa cag cct tct gag agt tct tct cag gac agt act      709
   Met Glu Val Ser Glu Gln Pro Ser Glu Ser Ser Ser Gln Asp Ser Thr
       80                  85                  90
   aaa act gat ggc aag ata gct gtg tca gct tct cct gct gtt cct agg      757
   Lys Thr Asp Gly Lys Ile Ala Val Ser Ala Ser Pro Ala Val Pro Arg
   95                  100                 105                 110
   aag aag cct gtt ggt gtt agg caa agg aaa tgg ggg aaa tgg gct gct      805
   Lys Lys Pro Val Gly Val Arg Gln Arg Lys Trp Gly Lys Trp Ala Ala
                   115                 120                 125
   gag att aga gat cct att aag aaa act agg act tgg ttg ggt act ttt      853
   Glu Ile Arg Asp Pro Ile Lys Lys Thr Arg Thr Trp Leu Gly Thr Phe
               130                 135                 140
   gat act ctt gaa gaa gct gct aaa gct tat gat gct aag aag ctt gag      901
   Asp Thr Leu Glu Glu Ala Ala Lys Ala Tyr Asp Ala Lys Lys Leu Glu
           145                 150                 155
   ttt gat gct att gtt gct gga aat gtg tcc act act aaa cgt gat gtt      949
   Phe Asp Ala Ile Val Ala Gly Asn Val Ser Thr Thr Lys Arg Asp Val
       160                 165                 170
   tct tca tct gag act agc caa tgc tct cgt tct tca cct gtt gtt cct      997
   Ser Ser Ser Glu Thr Ser Gln Cys Ser Arg Ser Ser Pro Val Val Pro
   175                 180                 185                 190
   gtt gag caa gat gac act tct gca tca gct ctc act tgt gtc aac aac     1045
   Val Glu Gln Asp Asp Thr Ser Ala Ser Ala Leu Thr Cys Val Asn Asn
                   195                 200                 205
   cct gat gac gtc tcg acc gtt gct cca act gct cca act cca aat gtt     1093
   Pro Asp Asp Val Ser Thr Val Ala Pro Thr Ala Pro Thr Pro Asn Val
               210                 215                 220
   cct gct ggt gga aac aag gaa acg ttg ttc gat ttc gac ttt act aat     1141
   Pro Ala Gly Gly Asn Lys Glu Thr Leu Phe Asp Phe Asp Phe Thr Asn
           225                 230                 235
   cta cag atc cct gat ttt ggt ttc ttg gca gag gag caa caa gac cta     1189
   Leu Gln Ile Pro Asp Phe Gly Phe Leu Ala Glu Glu Gln Gln Asp Leu
       240                 245                 250
   gac ttc gat tgt ttc ctc gcg gat gat cag ttt gat gat ttc ggc ttg     1237
   Asp Phe Asp Cys Phe Leu Ala Asp Asp Gln Phe Asp Asp Phe Gly Leu
   255                 260                 265                 270
   ctt gat gac att caa gga ttc gaa gat aac ggt cca agt gcg tta cca     1285
   Leu Asp Asp Ile Gln Gly Phe Glu Asp Asn Gly Pro Ser Ala Leu Pro
                   275                 280                 285
   gat ttc gac ttt gcg gat gtt gaa gat ctt cag cta gct gac tct agt     1333
   Asp Phe Asp Phe Ala Asp Val Glu Asp Leu Gln Leu Ala Asp Ser Ser
               290                 295                 300
   ttc ggt ttc ctt gat caa ctt gct cct atc aac atc tct tgc cca tta     1381
   Phe Gly Phe Leu Asp Gln Leu Ala Pro Ile Asn Ile Ser Cys Pro Leu
           305                 310                 315
   aaa agt ttt gca gct tca tag gatcttgctt agtaatgtta agtgagaaga        1432
   Lys Ser Phe Ala Ala Ser
       320
   gtgttttgtt ttttcgttta tgctttagta atttaagaca tacaaaagtg tgtgttccgg   1492
   attgtagtaa gatcttaaga cataaagccg ggttttgcaa ttaggaatcg agttttaatg   1552
   aagttttagt ttatgtttg                                                1571
<210> 12
   <211> 324
   <212> PRT
   <213> Arabidopsis thaliana
<400> 12
   Met Val Ala Ile Arg Lys Glu Gln Ser Leu Ser Gly Val Ser Ser Glu
   1               5                   10                  15
   Ile Lys Lys Arg Ala Lys Arg Asn Thr Leu Ser Ser Leu Pro Gln Glu
               20                  25                  30
   Thr Gln Pro Leu Arg Lys Val Arg Ile Ile Val Asn Asp Pro Tyr Ala
           35                  40                  45
   Thr Asp Asp Ser Ser Ser Asp Glu Glu Glu Leu Lys Val Pro Lys Pro
       50                  55                  60
   Arg Lys Met Lys Arg Ile Val Arg Glu Ile Asn Phe Pro Ser Met Glu
   65                  70                  75                  80
   Val Ser Glu Gln Pro Ser Glu Ser Ser Ser Gln Asp Ser Thr Lys Thr
                   85                  90                  95
   Asp Gly Lys Ile Ala Val Ser Ala Ser Pro Ala Val Pro Arg Lys Lys
               100                 105                 110
   Pro Val Gly Val Arg Gln Arg Lys Trp Gly Lys Trp Ala Ala Glu Ile
           115                 120                 125
   Arg Asp Pro Ile Lys Lys Thr Arg Thr Trp Leu Gly Thr Phe Asp Thr
       130                 135                 140
   Leu Glu Glu Ala Ala Lys Ala Tyr Asp Ala Lys Lys Leu Glu Phe Asp
   145                 150                 155                 160
   Ala Ile Val Ala Gly Asn Val Ser Thr Thr Lys Arg Asp Val Ser Ser
                   165                 170                 175
   Ser Glu Thr Ser Gln Cys Ser Arg Ser Ser Pro Val Val Pro Val Glu
               180                 185                 190
   Gln Asp Asp Thr Ser Ala Ser Ala Leu Thr Cys Val Asn Asn Pro Asp
           195                 200                 205
   Asp Val Ser Thr Val Ala Pro Thr Ala Pro Thr Pro Asn Val Pro Ala
       210                 215                 220
   Gly Gly Asn Lys Glu Thr Leu Phe Asp Phe Asp Phe Thr Asn Leu Gln
   225 230 235 240
   Ile Pro Asp Phe Gly Phe Leu Ala Glu Glu Gln Gln Asp Leu Asp Phe
   245 250 255
   Asp Cys Phe Leu Ala Asp Asp Gln Phe Asp Asp Phe Gly Leu Leu Asp
   260 265 270
   Asp Ile Gln Gly Phe Glu Asp Asn Gly Pro Ser Ala Leu Pro Asp Phe
   275 280 285
   Asp Phe Ala Asp Val Glu Asp Leu Gln Leu Ala Asp Ser Ser Phe Gly
   290 295 300
   Phe Leu Asp Gln Leu Ala Pro Ile Asn Ile Ser Cys Pro Leu Lys Ser
   305 310 315 320
   Phe Ala Ala Ser
<210> 13
   <211> 920
   <212> DNA
   <213> Arabidopsis thaliana
<220>
   <221> CDS
   <222> (1)..(852)
   <223> G237
<400> 13
   atg gcg aag acg aaa tat gga gag aga cat agg aaa ggg tta tgg tca       48
   Met Ala Lys Thr Lys Tyr Gly Glu Arg His Arg Lys Gly Leu Trp Ser
   1               5                   10                  15
   cct gaa gaa gac gag aag cta agg agc ttc atc ctc tct tat ggc cat       96
   Pro Glu Glu Asp Glu Lys Leu Arg Ser Phe Ile Leu Ser Tyr Gly His
               20                  25                  30
   tct tgc tgg acc act gtt ccc atc aaa gct ggg tta caa agg aat ggg      144
   Ser Cys Trp Thr Thr Val Pro Ile Lys Ala Gly Leu Gln Arg Asn Gly
           35                  40                  45
   aag agc tgc aga tta aga tgg att aat tac cta aga cca ggg tta aag      192
   Lys Ser Cys Arg Leu Arg Trp Ile Asn Tyr Leu Arg Pro Gly Leu Lys
       50                  55                  60
   agg gat atg att agt gca gaa gaa gaa gag act atc ttg acg ttt cat      240
   Arg Asp Met Ile Ser Ala Glu Glu Glu Glu Thr Ile Leu Thr Phe His
   65                  70                  75                  80
   tct ccc ttg ggt aac aag tgg tcg caa ata gct aaa ttc tta ccg gga      288
   Ser Pro Leu Gly Asn Lys Trp Ser Gln Ile Ala Lys Phe Leu Pro Gly
                   85                  90                  95
   aga aca gac aat gag ata aag aac tat tgg cac tct cat ttg aaa aag      336
   Arg Thr Asp Asn Glu Ile Lys Asn Tyr Trp His Ser His Leu Lys Lys
               100                 105                 110
   aaa tgg ctc aag tct cag agc tta caa gat gca aaa tct att tcc cct      384
   Lys Trp Leu Lys Ser Gln Ser Leu Gln Asp Ala Lys Ser Ile Ser Pro
           115                 120                 125
   cct tcg tct tca tca tca tca ctt gtt gct tgt gga gaa aga aat ccg      432
   Pro Ser Ser Ser Ser Ser Ser Leu Val Ala Cys Gly Glu Arg Asn Pro
       130                 135                 140
   gaa acc ttg atc tcg aat cac gtg ttc tcc ctc cag aga ctt cta gag      480
   Glu Thr Leu Ile Ser Asn His Val Phe Ser Leu Gln Arg Leu Leu Glu
   145                 150                 155                 160
   aac aaa tct tca tct ccc tca caa gaa agc aac gga aat aac agc cat      528
   Asn Lys Ser Ser Ser Pro Ser Gln Glu Ser Asn Gly Asn Asn Ser His
                   165                 170                 175
   caa tgt tct tct gct cct gag att cca agg ctt ttc ttc tct gaa tgg      576
   Gln Cys Ser Ser Ala Pro Glu Ile Pro Arg Leu Phe Phe Ser Glu Trp
               180                 185                 190
   ctt tct tct tca tat ccc cac acc gat tat tcc tct gag ttt acc gac      624
   Leu Ser Ser Ser Tyr Pro His Thr Asp Tyr Ser Ser Glu Phe Thr Asp
           195                 200                 205
   tct aag cac agt caa gct cca aat gtc gaa gag act ctc tca gct tat      672
   Ser Lys His Ser Gln Ala Pro Asn Val Glu Glu Thr Leu Ser Ala Tyr
       210                 215                 220
   gaa gaa atg ggt gat gtt gat cag ttc cat tac aac gaa atg atg atc      720
   Glu Glu Met Gly Asp Val Asp Gln Phe His Tyr Asn Glu Met Met Ile
   225                 230                 235                 240
   aac aac agc aac tgg act ctt aac gac att gtg ttt ggt tcc aaa tgt      768
   Asn Asn Ser Asn Trp Thr Leu Asn Asp Ile Val Phe Gly Ser Lys Cys
                   245                 250                 255
   aag aag cag gag cat cat att tat aga gag gct tca gat tgt aat tct      816
   Lys Lys Gln Glu His His Ile Tyr Arg Glu Ala Ser Asp Cys Asn Ser
               260                 265                 270
   tct gct gaa ttc ttt tct cca cca aca acg acg taa attgcgttta           862
   Ser Ala Glu Phe Phe Ser Pro Pro Thr Thr Thr
           275                 280
   ttgtaatgta aatcaaattt ctaaggcaaa accggaaaaa aaaaaaaaaa aaaaaaaa       920
<210> 14
   <211> 283
   <212> PRT
   <213> Arabidopsis thaliana
<400> 14
   Met Ala Lys Thr Lys Tyr Gly Glu Arg His Arg Lys Gly Leu Trp Ser
   1               5                   10                  15
   Pro Glu Glu Asp Glu Lys Leu Arg Ser Phe Ile Leu Ser Tyr Gly His
               20                  25                  30
   Ser Cys Trp Thr Thr Val Pro Ile Lys Ala Gly Leu Gln Arg Asn Gly
           35                  40                  45
   Lys Ser Cys Arg Leu Arg Trp Ile Asn Tyr Leu Arg Pro Gly Leu Lys
       50                  55                  60
   Arg Asp Met Ile Ser Ala Glu Glu Glu Glu Thr Ile Leu Thr Phe His
   65                  70                  75                  80
   Ser Pro Leu Gly Asn Lys Trp Ser Gln Ile Ala Lys Phe Leu Pro Gly
                   85                  90                  95
   Arg Thr Asp Asn Glu Ile Lys Asn Tyr Trp His Ser His Leu Lys Lys
               100                 105                 110
   Lys Trp Leu Lys Ser Gln Ser Leu Gln Asp Ala Lys Ser Ile Ser Pro
           115                 120                 125
   Pro Ser Ser Ser Ser Ser Ser Leu Val Ala Cys Gly Glu Arg Asn Pro
       130                 135                 140
   Glu Thr Leu Ile Ser Asn His Val Phe Ser Leu Gln Arg Leu Leu Glu
   145                 150                 155                 160
   Asn Lys Ser Ser Ser Pro Ser Gln Glu Ser Asn Gly Asn Asn Ser His
                   165                 170                 175
   Gln Cys Ser Ser Ala Pro Glu Ile Pro Arg Leu Phe Phe Ser Glu Trp
               180                 185                 190
   Leu Ser Ser Ser Tyr Pro His Thr Asp Tyr Ser Ser Glu Phe Thr Asp
           195                 200                 205
   Ser Lys His Ser Gln Ala Pro Asn Val Glu Glu Thr Leu Ser Ala Tyr
       210                 215                 220
   Glu Glu Met Gly Asp Val Asp Gln Phe His Tyr Asn Glu Met Met Ile
   225                 230                 235                 240
   Asn Asn Ser Asn Trp Thr Leu Asn Asp Ile Val Phe Gly Ser Lys Cys
                   245                 250                 255
   Lys Lys Gln Glu His His Ile Tyr Arg Glu Ala Ser Asp Cys Asn Ser
               260                 265                 270
   Ser Ala Glu Phe Phe Ser Pro Pro Thr Thr Thr
           275                 280
<210> 15
   <211> 1302
   <212> DNA
   <213> Arabidopsis thaliana
<220>
   <221> CDS
   <222> (331)..(1149)
   <223> G409
<400> 15
   tgtctctctc tctggctctc tttctcttaa cgtgatcata acgtgattcg aaaattggat      60
   atagataggt ttcttgttgg atcttgatcc ctctggaaaa ggaggggaga atagcagttc     120
   atgatgggat tttgtatctg cccgttggag tcacctgcga gattactatg gagtacaagc     180
   ttcttccgcc ataagatcat gatcttctaa tccttcctac ttcttcccat ctttttaatc     240
   atcttctcgc tatctctgct tcctctttct ctctgtttcc tctttctcag aactcagaag     300
   tagttgttgt tttatttctg ttgatcaaaa atg gaa tcc aat tcg ttt ttc ttc      354
                                    Met Glu Ser Asn Ser Phe Phe Phe
                                    1               5
   gat cca tct gct tca cac ggc aac agc atg ttc ttc ctt ggg aat ctc       402
   Asp Pro Ser Ala Ser His Gly Asn Ser Met Phe Phe Leu Gly Asn Leu
       10                  15                  20
   aat ccc gtc gtc caa gga gga gga gca aga tcg atg atg aac atg gag       450
   Asn Pro Val Val Gln Gly Gly Gly Ala Arg Ser Met Met Asn Met Glu
   25                  30                  35                  40
   gaa act tcg aag cga agg ccc ttc ttt agc tcc cct gag gat ctc tac      498
   Glu Thr Ser Lys Arg Arg Pro Phe Phe Ser Ser Pro Glu Asp Leu Tyr
                   45                  50                  55
   gac gat gac ttt tac gac gac cag ttg cct gaa aag aag cgt cgc ctc      546
   Asp Asp Asp Phe Tyr Asp Asp Gln Leu Pro Glu Lys Lys Arg Arg Leu
               60                  65                  70
   act acc gaa caa gtg cat ctg ctg gag aaa agc ttc gag aca gag aac      594
   Thr Thr Glu Gln Val His Leu Leu Glu Lys Ser Phe Glu Thr Glu Asn
           75                  80                  85
   aag cta gag cct gaa cgc aag act cag ctt gcc aag aag ctt ggt cta      642
   Lys Leu Glu Pro Glu Arg Lys Thr Gln Leu Ala Lys Lys Leu Gly Leu
       90                  95                  100
   cag cca agg caa gtg gct gtc tgg ttt cag aat cgc cga gct cgt tgg      690
   Gln Pro Arg Gln Val Ala Val Trp Phe Gln Asn Arg Arg Ala Arg Trp
   105                 110                 115                 120
   aaa aca aaa cag ctt gag aga gac tac gat ctt ctc aag tcc act tac      738
   Lys Thr Lys Gln Leu Glu Arg Asp Tyr Asp Leu Leu Lys Ser Thr Tyr
                   125                 130                 135
   gac caa ctt ctt tct aac tac gac tcc atc gtc atg gac aac gat aag      786
   Asp Gln Leu Leu Ser Asn Tyr Asp Ser Ile Val Met Asp Asn Asp Lys
               140                 145                 150
   ctc aga tcc gag gtt act tcc ctg acc gaa aag ctt cag ggc aaa caa      834
   Leu Arg Ser Glu Val Thr Ser Leu Thr Glu Lys Leu Gln Gly Lys Gln
           155                 160                 165
   gag aca gct aat gaa cca cct ggt caa gtg ccc gaa cca aac caa ctt      882
   Glu Thr Ala Asn Glu Pro Pro Gly Gln Val Pro Glu Pro Asn Gln Leu
       170                 175                 180
   gat ccg gtt tac att aat gcg gca gca atc aaa acc gag gac cgg tta      930
   Asp Pro Val Tyr Ile Asn Ala Ala Ala Ile Lys Thr Glu Asp Arg Leu
   185                 190                 195                 200
   agt tca ggg agc gtt ggg agc gcg gta cta gac gac gac gca cct caa      978
   Ser Ser Gly Ser Val Gly Ser Ala Val Leu Asp Asp Asp Ala Pro Gln
                   205                 210                 215
   cta cta gac agc tgt gac tct tac ttc cca agc atc gta ccc atc caa     1026
   Leu Leu Asp Ser Cys Asp Ser Tyr Phe Pro Ser Ile Val Pro Ile Gln
               220                 225                 230
   gac aac agc aac gcc agt gat cat gac aat gac cgg agc tgt ttc gcc     1074
   Asp Asn Ser Asn Ala Ser Asp His Asp Asn Asp Arg Ser Cys Phe Ala
           235                 240                 245
   gac gtc ttt gtg ccc acc act tca ccg tcg cac gat cat cac ggt gaa     1122
   Asp Val Phe Val Pro Thr Thr Ser Pro Ser His Asp His His Gly Glu
       250                 255                 260
   tca ttg gct ttc tgg gga tgg cct tag aaaaccactc tgataataaa           1169
   Ser Leu Ala Phe Trp Gly Trp Pro
   265                 270
   tgtgtgttta tttaagttca agagtcatct tcttgttgtt tccatgttga cgataattgt   1229
   tgactcgtgg aataattccg ctgttcaacg gtatttttat cagttgcatt atatgctttt   1289
   atgaaaaaaa aaa                                                       1302
<210> 16
   <211> 272
   <212> PRT
   <213> Arabidopsis thaliana
<400> 16
   Met Glu Ser Asn Ser Phe Phe Phe Asp Pro Ser Ala Ser His Gly Asn
   1               5                   10                  15
   Ser Met Phe Phe Leu Gly Asn Leu Asn Pro Val Val Gln Gly Gly Gly
               20                  25                  30
   Ala Arg Ser Met Met Asn Met Glu Glu Thr Ser Lys Arg Arg Pro Phe
           35                  40                  45
   Phe Ser Ser Pro Glu Asp Leu Tyr Asp Asp Asp Phe Tyr Asp Asp Gln
       50                  55                  60
   Leu Pro Glu Lys Lys Arg Arg Leu Thr Thr Glu Gln Val His Leu Leu
   65                  70                  75                  80
   Glu Lys Ser Phe Glu Thr Glu Asn Lys Leu Glu Pro Glu Arg Lys Thr
                   85                  90                  95
   Gln Leu Ala Lys Lys Leu Gly Leu Gln Pro Arg Gln Val Ala Val Trp
               100                 105                 110
   Phe Gln Asn Arg Arg Ala Arg Trp Lys Thr Lys Gln Leu Glu Arg Asp
           115                 120                 125
   Tyr Asp Leu Leu Lys Ser Thr Tyr Asp Gln Leu Leu Ser Asn Tyr Asp
       130                 135                 140
   Ser Ile Val Met Asp Asn Asp Lys Leu Arg Ser Glu Val Thr Ser Leu
   145                 150                 155                 160
   Thr Glu Lys Leu Gln Gly Lys Gln Glu Thr Ala Asn Glu Pro Pro Gly
                   165                 170                 175
   Gln Val Pro Glu Pro Asn Gln Leu Asp Pro Val Tyr Ile Asn Ala Ala
               180                 185                 190
   Ala Ile Lys Thr Glu Asp Arg Leu Ser Ser Gly Ser Val Gly Ser Ala
           195                 200                 205
   Val Leu Asp Asp Asp Ala Pro Gln Leu Leu Asp Ser Cys Asp Ser Tyr
       210                 215                 220
   Phe Pro Ser Ile Val Pro Ile Gln Asp Asn Ser Asn Ala Ser Asp His
   225                 230                 235                 240
   Asp Asn Asp Arg Ser Cys Phe Ala Asp Val Phe Val Pro Thr Thr Ser
                   245                 250                 255
   Pro Ser His Asp His His Gly Glu Ser Leu Ala Phe Trp Gly Trp Pro
               260                 265                 270
<210> 17
   <211> 2545
   <212> DNA
   <213> Arabidopsis thaliana
<220>
   <221> CDS
   <222> (103)..(2322)
   <223> G418
<400> 17
   acatatgttt taaattcttt gtctgaatct tacaggatcc gagagagaga gctctggaac      60
   gatattaaca tatatcatga agaaaaagat tgaagtattg at atg gga ata act        114
                                                  Met Gly Ile Thr
                                                  1
   aaa act tct cct aat act aca att ctc ttg aag act ttt cac aat aat       162
   Lys Thr Ser Pro Asn Thr Thr Ile Leu Leu Lys Thr Phe His Asn Asn
   5                   10                  15                  20
   tct atg tcc caa gat tat cat cat cat cat cat cat aat caa cac caa       210
   Ser Met Ser Gln Asp Tyr His His His His His His Asn Gln His Gln
                   25                  30                  35
   gga ggt atc ttc aac ttc tct aat gga ttc gac cga tca gat tct ccc       258
   Gly Gly Ile Phe Asn Phe Ser Asn Gly Phe Asp Arg Ser Asp Ser Pro
               40                  45                  50
   aat tta aca act cag cag aag caa gag cat caa agg gta gag atg gac       306
   Asn Leu Thr Thr Gln Gln Lys Gln Glu His Gln Arg Val Glu Met Asp
           55                  60                  65
   gag gaa tct tca gtc gcc gga ggt agg att ccg gtc tac gaa tca gcc       354
   Glu Glu Ser Ser Val Ala Gly Gly Arg Ile Pro Val Tyr Glu Ser Ala
       70                  75                  80
   ggt atg tta tcc gaa atg ttt aat ttc ccc gga agc agc ggt gga gga       402
   Gly Met Leu Ser Glu Met Phe Asn Phe Pro Gly Ser Ser Gly Gly Gly
   85                  90                  95                  100
   aga gat ctc gac ctc ggc caa tct ttc cgg tca aat agg cag ttg ctt       450
   Arg Asp Leu Asp Leu Gly Gln Ser Phe Arg Ser Asn Arg Gln Leu Leu
                   105                 110                 115
   gag gag caa cat cag aat att ccg gct atg aat gct acg gat tca gcc       498
   Glu Glu Gln His Gln Asn Ile Pro Ala Met Asn Ala Thr Asp Ser Ala
               120                 125                 130
   acc gcc acc gca gcc gcc atg cag tta ttc ttg atg aat cca ccg cca       546
   Thr Ala Thr Ala Ala Ala Met Gln Leu Phe Leu Met Asn Pro Pro Pro
           135                 140                 145
   ccg caa caa cca ccg tct ccg tca tcc aca act tcc cca agg agc cac       594
   Pro Gln Gln Pro Pro Ser Pro Ser Ser Thr Thr Ser Pro Arg Ser His
       150                 155                 160
   cac aat tct tca act ctt cac atg tta ctt cca agt cca tcc acc aac       642
   His Asn Ser Ser Thr Leu His Met Leu Leu Pro Ser Pro Ser Thr Asn
   165                 170                 175                 180
   aca act cac cat cag aac tac act aat cat atg tct atg cat cag ctt       690
   Thr Thr His His Gln Asn Tyr Thr Asn His Met Ser Met His Gln Leu
                   185                 190                 195
   cca cat cag cat cac caa cag ata tcg acg tgg cag tct tct ccc gat       738
   Pro His Gln His His Gln Gln Ile Ser Thr Trp Gln Ser Ser Pro Asp
               200                 205                 210
   cat cat cat cat cat cac aac agc caa acg gag att ggg acc gtc cac       786
   His His His His His His Asn Ser Gln Thr Glu Ile Gly Thr Val His
           215                 220                 225
   gtg gaa aac agc gga gga cac gga gga caa ggc ttg tcc tta tct ctc       834
   Val Glu Asn Ser Gly Gly His Gly Gly Gln Gly Leu Ser Leu Ser Leu
       230                 235                 240
   tca tcg tct tta gag gct gca gca aaa gcg gaa gag tat aga aac att       882
   Ser Ser Ser Leu Glu Ala Ala Ala Lys Ala Glu Glu Tyr Arg Asn Ile
   245                 250                 255                 260
   tac tac gga gcc aat tct tct aac gca tca cct cat cat caa tac aat      930
   Tyr Tyr Gly Ala Asn Ser Ser Asn Ala Ser Pro His His Gln Tyr Asn
                   265                 270                 275
   caa ttc aag act ctt ctt gct aat tct tct caa cat cac cat caa gta      978
   Gln Phe Lys Thr Leu Leu Ala Asn Ser Ser Gln His His His Gln Val
               280                 285                 290
   tta aac caa ttc cga tca tct ccg gct gct tct tcc tct tcc atg gca     1026
   Leu Asn Gln Phe Arg Ser Ser Pro Ala Ala Ser Ser Ser Ser Met Ala
           295                 300                 305
   gcg gtc aat atc tta aga aac tcg agg tac aca acg gcc gcg caa gag     1074
   Ala Val Asn Ile Leu Arg Asn Ser Arg Tyr Thr Thr Ala Ala Gln Glu
       310                 315                 320
   ttg ttg gaa gag ttt tgt agt gtt gga aga gga ttt ttg aag aag aac     1122
   Leu Leu Glu Glu Phe Cys Ser Val Gly Arg Gly Phe Leu Lys Lys Asn
   325                 330                 335                 340
   aaa ctt ggg aac agc tca aac cct aat act tgc ggt ggt gat ggt ggt     1170
   Lys Leu Gly Asn Ser Ser Asn Pro Asn Thr Cys Gly Gly Asp Gly Gly
                   345                 350                 355
   ggc agc tct cct tcg tcg gcc gga gca aac aag gag cat cct cct tta     1218
   Gly Ser Ser Pro Ser Ser Ala Gly Ala Asn Lys Glu His Pro Pro Leu
               360                 365                 370
   tcg gcg tct gat cgg att gag cat caa aga agg aaa gtg aaa cta ctc     1266
   Ser Ala Ser Asp Arg Ile Glu His Gln Arg Arg Lys Val Lys Leu Leu
           375                 380                 385
   acc atg ctt gaa gag gtg gac cga cgg tac aac cat tac tgc gag caa     1314
   Thr Met Leu Glu Glu Val Asp Arg Arg Tyr Asn His Tyr Cys Glu Gln
       390                 395                 400
   atg cag atg gtt gtg aac tct ttc gac ata gta atg ggc cac ggt gcg     1362
   Met Gln Met Val Val Asn Ser Phe Asp Ile Val Met Gly His Gly Ala
   405                 410                 415                 420
   gca tta ccg tac acc gca ttg gct caa aaa gct atg tca aga cat ttt     1410
   Ala Leu Pro Tyr Thr Ala Leu Ala Gln Lys Ala Met Ser Arg His Phe
                   425                 430                 435
   aga tgc ctt aaa gat gca gtt gcg gct cag ctt aag cag agt tgc gaa     1458
   Arg Cys Leu Lys Asp Ala Val Ala Ala Gln Leu Lys Gln Ser Cys Glu
               440                 445                 450
   ctt ctt ggg gac aaa gat gca gcg gga atc tct tct tcc ggg tta aca     1506
   Leu Leu Gly Asp Lys Asp Ala Ala Gly Ile Ser Ser Ser Gly Leu Thr
           455                 460                 465
   aaa ggt gaa act ccg cgt ttg cgt ttg cta gag caa agt ttg cgt cag     1554
   Lys Gly Glu Thr Pro Arg Leu Arg Leu Leu Glu Gln Ser Leu Arg Gln
       470                 475                 480
   caa cgt gcg ttt cat caa atg ggt atg atg gaa caa gaa gct tgg cgg     1602
   Gln Arg Ala Phe His Gln Met Gly Met Met Glu Gln Glu Ala Trp Arg
   485                 490                 495                 500
   cca caa cgc ggt ttg cct gaa cgc tcc gtc aat ata ctt aga gct tgg     1650
   Pro Gln Arg Gly Leu Pro Glu Arg Ser Val Asn Ile Leu Arg Ala Trp
                   505                 510                 515
   ctc ttc gaa cat ttc ctt cac ccg tat cca agt gat gca gat aaa cac     1698
   Leu Phe Glu His Phe Leu His Pro Tyr Pro Ser Asp Ala Asp Lys His
               520                 525                 530
   cta ttg gct cga cag act ggt tta tcc aga aat cag gta tca aat tgg     1746
   Leu Leu Ala Arg Gln Thr Gly Leu Ser Arg Asn Gln Val Ser Asn Trp
           535                 540                 545
   ttc ata aat gct agg gtt cgt tta tgg aaa cca atg gtg gaa gaa atg     1794
   Phe Ile Asn Ala Arg Val Arg Leu Trp Lys Pro Met Val Glu Glu Met
           550                 555                 560
   tac caa caa gaa tca aaa gaa aga gaa aga gaa gag gaa tta gaa gag      1842
   Tyr Gln Gln Glu Ser Lys Glu Arg Glu Arg Glu Glu Glu Leu Glu Glu
   565                 570                 575                 580
   aac gaa gaa gat caa gaa aca aaa aac agc aac gac gac aag agc aca      1890
   Asn Glu Glu Asp Gln Glu Thr Lys Asn Ser Asn Asp Asp Lys Ser Thr
                   585                 590                 595
   aaa tcc aac aac aat gaa agc aac ttc act gcc gtt cgg acc act tca      1938
   Lys Ser Asn Asn Asn Glu Ser Asn Phe Thr Ala Val Arg Thr Thr Ser
               600                 605                 610
   caa act cca acg aca acc gca cca gac gca tca gac gca gac gca gca      1986
   Gln Thr Pro Thr Thr Thr Ala Pro Asp Ala Ser Asp Ala Asp Ala Ala
           615                 620                 625
   gta gcg aca ggc cac cgt cta aga tcc aac att aat gct tac gaa aac      2034
   Val Ala Thr Gly His Arg Leu Arg Ser Asn Ile Asn Ala Tyr Glu Asn
       630                 635                 640
   gac gct tca tca ctt cta ctc cct tcc tct tat tcc aac gcc gcc gct      2082
   Asp Ala Ser Ser Leu Leu Leu Pro Ser Ser Tyr Ser Asn Ala Ala Ala
   645                 650                 655                 660
   cct gcc gct gtt tct gac gac ttg aat tct cgt tac ggt ggc tca gac      2130
   Pro Ala Ala Val Ser Asp Asp Leu Asn Ser Arg Tyr Gly Gly Ser Asp
                   665                 670                 675
   gcg ttt tcc gcc gtt gcc acg tgt caa caa agt gta ggt ggg ttc gat      2178
   Ala Phe Ser Ala Val Ala Thr Cys Gln Gln Ser Val Gly Gly Phe Asp
               680                 685                 690
   gat gct gac atg gat ggt gtt aac gtt ata agg ttt ggg aca aac cct      2226
   Asp Ala Asp Met Asp Gly Val Asn Val Ile Arg Phe Gly Thr Asn Pro
           695                 700                 705
   act ggt gac gtg tct ctc acg ctt ggt tta cgc cac gct gga aac atg      2274
   Thr Gly Asp Val Ser Leu Thr Leu Gly Leu Arg His Ala Gly Asn Met
       710                 715                 720
   cct gac aaa gac gct tct ttc tgc gtt aga gag ttt ggg ggt ttt tag      2322
   Pro Asp Lys Asp Ala Ser Phe Cys Val Arg Glu Phe Gly Gly Phe
   725                 730                 735
   tttgcttttg tcactccatt taattaatta attatagttt tccattctta cttattttaa    2382
   ttgaaaatct atttttgtct cttaaaagtc caaacaatac attagtctag ccctcctctg    2442
   cttttttttt tctatctcgt gaagagaaga aaacgatacg taaatccctt cgaaaactaa    2502
   tgtacgttgt acgacttatt gttttcataa aaaaaaaaaa aaa                      2545
<210> 18
   <211> 739
   <212> PRT
   <213> Arabidopsis thaliana
<400> 18
   Met Gly Ile Thr Lys Thr Ser Pro Asn Thr Thr Ile Leu Leu Lys Thr
   1               5                   10                  15
   Phe His Asn Asn Ser Met Ser Gln Asp Tyr His His His His His His
               20                  25                  30
   Asn Gln His Gln Gly Gly Ile Phe Asn Phe Ser Asn Gly Phe Asp Arg
           35                  40                  45
   Ser Asp Ser Pro Asn Leu Thr Thr Gln Gln Lys Gln Glu His Gln Arg
   50 55 60
   Val Glu Met Asp Glu Glu Ser Ser Val Ala Gly Gly Arg Ile Pro Val
   65 70 75 80
   Tyr Glu Ser Ala Gly Met Leu Ser Glu Met Phe Asn Phe Pro Gly Ser
   85 90 95
   Ser Gly Gly Gly Arg Asp Leu Asp Leu Gly Gln Ser Phe Arg Ser Asn
   100 105 110
   Arg Gln Leu Leu Glu Glu Gln His Gln Asn Ile Pro Ala Met Asn Ala
   115 120 125
   Thr Asp Ser Ala Thr Ala Thr Ala Ala Ala Met Gln Leu Phe Leu Met
   130 135 140
   Asn Pro Pro Pro Pro Gln Gln Pro Pro Ser Pro Ser Ser Thr Thr Ser
   145 150 155 160
   Pro Arg Ser His His Asn Ser Ser Thr Leu His Met Leu Leu Pro Ser
   165 170 175
   Pro Ser Thr Asn Thr Thr His His Gln Asn Tyr Thr Asn His Met Ser
   180 185 190
   Met His Gln Leu Pro His Gln His His Gln Gln Ile Ser Thr Trp Gln
   195 200 205
   Ser Ser Pro Asp His His His His His His Asn Ser Gln Thr Glu Ile
   210 215 220
   Gly Thr Val His Val Glu Asn Ser Gly Gly His Gly Gly Gln Gly Leu
   225 230 235 240
   Ser Leu Ser Leu Ser Ser Ser Leu Glu Ala Ala Ala Lys Ala Glu Glu
   245 250 255
   Tyr Arg Asn Ile Tyr Tyr Gly Ala Asn Ser Ser Asn Ala Ser Pro His
   260 265 270
   His Gln Tyr Asn Gln Phe Lys Thr Leu Leu Ala Asn Ser Ser Gln His
   275 280 285
   His His Gln Val Leu Asn Gln Phe Arg Ser Ser Pro Ala Ala Ser Ser
   290 295 300
   Ser Ser Met Ala Ala Val Asn Ile Leu Arg Asn Ser Arg Tyr Thr Thr
   305 310 315 320
   Ala Ala Gln Glu Leu Leu Glu Glu Phe Cys Ser Val Gly Arg Gly Phe
   325 330 335
   Leu Lys Lys Asn Lys Leu Gly Asn Ser Ser Asn Pro Asn Thr Cys Gly
   340 345 350
   Gly Asp Gly Gly Gly Ser Ser Pro Ser Ser Ala Gly Ala Asn Lys Glu
   355 360 365
   His Pro Pro Leu Ser Ala Ser Asp Arg Ile Glu His Gln Arg Arg Lys
   370 375 380
   Val Lys Leu Leu Thr Met Leu Glu Glu Val Asp Arg Arg Tyr Asn His
   385 390 395 400
   Tyr Cys Glu Gln Met Gln Met Val Val Asn Ser Phe Asp Ile Val Met
   405 410 415
   Gly His Gly Ala Ala Leu Pro Tyr Thr Ala Leu Ala Gln Lys Ala Met
   420 425 430
   Ser Arg His Phe Arg Cys Leu Lys Asp Ala Val Ala Ala Gln Leu Lys
   435 440 445
   Gln Ser Cys Glu Leu Leu Gly Asp Lys Asp Ala Ala Gly Ile Ser Ser
   450 455 460
   Ser Gly Leu Thr Lys Gly Glu Thr Pro Arg Leu Arg Leu Leu Glu Gln
   465 470 475 480
   Ser Leu Arg Gln Gln Arg Ala Phe His Gln Met Gly Met Met Glu Gln
   485 490 495
   Glu Ala Trp Arg Pro Gln Arg Gly Leu Pro Glu Arg Ser Val Asn Ile
   500 505 510
   Leu Arg Ala Trp Leu Phe Glu His Phe Leu His Pro Tyr Pro Ser Asp
   515 520 525
   Ala Asp Lys His Leu Leu Ala Arg Gln Thr Gly Leu Ser Arg Asn Gln
   530 535 540
   Val Ser Asn Trp Phe Ile Asn Ala Arg Val Arg Leu Trp Lys Pro Met
   545 550 555 560
   Val Glu Glu Met Tyr Gln Gln Glu Ser Lys Glu Arg Glu Arg Glu Glu
   565 570 575
   Glu Leu Glu Glu Asn Glu Glu Asp Gln Glu Thr Lys Asn Ser Asn Asp
   580 585 590
   Asp Lys Ser Thr Lys Ser Asn Asn Asn Glu Ser Asn Phe Thr Ala Val
   595 600 605
   Arg Thr Thr Ser Gln Thr Pro Thr Thr Thr Ala Pro Asp Ala Ser Asp
   610 615 620
   Ala Asp Ala Ala Val Ala Thr Gly His Arg Leu Arg Ser Asn Ile Asn
   625 630 635 640
   Ala Tyr Glu Asn Asp Ala Ser Ser Leu Leu Leu Pro Ser Ser Tyr Ser
   645 650 655
   Asn Ala Ala Ala Pro Ala Ala Val Ser Asp Asp Leu Asn Ser Arg Tyr
               660                 665                 670
   Gly Gly Ser Asp Ala Phe Ser Ala Val Ala Thr Cys Gln Gln Ser Val
           675                 680                 685
   Gly Gly Phe Asp Asp Ala Asp Met Asp Gly Val Asn Val Ile Arg Phe
       690                 695                 700
   Gly Thr Asn Pro Thr Gly Asp Val Ser Leu Thr Leu Gly Leu Arg His
   705                 710                 715                 720
   Ala Gly Asn Met Pro Asp Lys Asp Ala Ser Phe Cys Val Arg Glu Phe
                   725                 730                 735
   Gly Gly Phe
<210> 19
   <211> 1240
   <212> DNA
   <213> Arabidopsis thaliana
<220>
   <221> CDS
   <222> (88)..(1020)
   <223> G591
<400> 19
   gtaaatctct ctttgaaggt tcctaactcg ttaatcgtaa ctcacagtga ctcgttcgag      60
   tcaaagtctc tgtctttagc tcaaacc atg gct agt aac aac cct cac gac aac     114
                                 Met Ala Ser Asn Asn Pro His Asp Asn
                                 1               5
   ctt tct gac caa act cct tct gat gat ttc ttc gag caa atc ctc ggc       162
   Leu Ser Asp Gln Thr Pro Ser Asp Asp Phe Phe Glu Gln Ile Leu Gly
   10                  15                  20                  25
   ctt cct aac ttc tca gcc tct tct gcc gcc ggt tta tct gga gtt gac       210
   Leu Pro Asn Phe Ser Ala Ser Ser Ala Ala Gly Leu Ser Gly Val Asp
                   30                  35                  40
   gga gga tta ggt ggt gga gca ccg cct atg atg ctg cag ttg ggt tcc       258
   Gly Gly Leu Gly Gly Gly Ala Pro Pro Met Met Leu Gln Leu Gly Ser
               45                  50                  55
   gga gaa gaa gga agt cac atg ggt ggc tta gga gga agt gga cca act       306
   Gly Glu Glu Gly Ser His Met Gly Gly Leu Gly Gly Ser Gly Pro Thr
           60                  65                  70
   ggg ttt cac aat cag atg ttt cct ttg ggg tta agt ctt gat caa ggg       354
   Gly Phe His Asn Gln Met Phe Pro Leu Gly Leu Ser Leu Asp Gln Gly
       75                  80                  85
   aaa gga cct ggg ttt ctt aga cct gaa gga gga cat gga agt ggg aaa       402
   Lys Gly Pro Gly Phe Leu Arg Pro Glu Gly Gly His Gly Ser Gly Lys
   90                  95                  100                 105
   aga ttc tca gat gat gtt gtt gat aat cga tgt tct tct atg aaa cct       450
   Arg Phe Ser Asp Asp Val Val Asp Asn Arg Cys Ser Ser Met Lys Pro
                   110                 115                 120
   gtt ttc cac ggg cag cct atg caa cag cca cct cca tcg gcc cca cat       498
   Val Phe His Gly Gln Pro Met Gln Gln Pro Pro Pro Ser Ala Pro His
               125                 130                 135
   cag cct act tca atc cgt ccc agg gtt cga gct agg cgt ggt cag gct        546
   Gln Pro Thr Ser Ile Arg Pro Arg Val Arg Ala Arg Arg Gly Gln Ala
           140                 145                 150
   act gat cca cat agc atc gct gag cgg cta cgt aga gaa aga ata gca        594
   Thr Asp Pro His Ser Ile Ala Glu Arg Leu Arg Arg Glu Arg Ile Ala
       155                 160                 165
   gaa cgg atc agg gcg ctg cag gaa ctt gta cct act gtg aac aag acc        642
   Glu Arg Ile Arg Ala Leu Gln Glu Leu Val Pro Thr Val Asn Lys Thr
   170                 175                 180                 185
   gat aga gct gct atg atc gat gag att gtc gat tat gta aag ttt ctc        690
   Asp Arg Ala Ala Met Ile Asp Glu Ile Val Asp Tyr Val Lys Phe Leu
                   190                 195                 200
   agg ctc caa gtc aag gtt ttg agc atg aac cga ctt ggt gga gcc ggt        738
   Arg Leu Gln Val Lys Val Leu Ser Met Asn Arg Leu Gly Gly Ala Gly
               205                 210                 215
   gcg gtt gct cca ctt gtt act gat atg cct ctt tca tca tca gtt gag        786
   Ala Val Ala Pro Leu Val Thr Asp Met Pro Leu Ser Ser Ser Val Glu
           220                 225                 230
   gat gaa acg ggt gag ggt gga agg act ccg caa cca gcg tgg gag aaa        834
   Asp Glu Thr Gly Glu Gly Gly Arg Thr Pro Gln Pro Ala Trp Glu Lys
       235                 240                 245
   tgg tct aac gat ggg act gaa cgt caa gtg gct aaa ctg atg gaa gag        882
   Trp Ser Asn Asp Gly Thr Glu Arg Gln Val Ala Lys Leu Met Glu Glu
   250                 255                 260                 265
   aac gtt gga gcc gcg atg cag ctt ctt caa tca aag gct ctt tgt atg        930
   Asn Val Gly Ala Ala Met Gln Leu Leu Gln Ser Lys Ala Leu Cys Met
                   270                 275                 280
   atg cca atc tca ttg gca atg gca att tac cat tct caa cct ccg gat        978
   Met Pro Ile Ser Leu Ala Met Ala Ile Tyr His Ser Gln Pro Pro Asp
               285                 290                 295
   aca tct tca gtg gtc aag cct gag aac aat cct cca cag tag               1020
   Thr Ser Ser Val Val Lys Pro Glu Asn Asn Pro Pro Gln
           300                 305                 310
   gatttctgca ataaagagtt tgtacagcta atccaactgt ccaacatggg tttttcttct     1080
   gctctaatga ctctggtttc ttctctcctc tctcaccgac ttgaaaggta aaaaagtgaa     1140
   aaaggctttg tagatggaat caatgtagga tttgcagtag agggcaaaaa aatgtcatat     1200
   agctcaattg atcaagtctt aaaaaaaaaa aaaaaaaaaa                           1240
<210> 20
   <211> 310
   <212> PRT
   <213> Arabidopsis thaliana
<400> 20
   Met Ala Ser Asn Asn Pro His Asp Asn Leu Ser Asp Gln Thr Pro Ser
   1               5                   10                  15
   Asp Asp Phe Phe Glu Gln Ile Leu Gly Leu Pro Asn Phe Ser Ala Ser
               20                  25                  30
   Ser Ala Ala Gly Leu Ser Gly Val Asp Gly Gly Leu Gly Gly Gly Ala
           35                  40                  45
   Pro Pro Met Met Leu Gln Leu Gly Ser Gly Glu Glu Gly Ser His Met
       50                  55                  60
   Gly Gly Leu Gly Gly Ser Gly Pro Thr Gly Phe His Asn Gln Met Phe
   65                  70                  75                  80
   Pro Leu Gly Leu Ser Leu Asp Gln Gly Lys Gly Pro Gly Phe Leu Arg
                   85                  90                  95
   Pro Glu Gly Gly His Gly Ser Gly Lys Arg Phe Ser Asp Asp Val Val
               100                 105                 110
   Asp Asn Arg Cys Ser Ser Met Lys Pro Val Phe His Gly Gln Pro Met
           115                 120                 125
   Gln Gln Pro Pro Pro Ser Ala Pro His Gln Pro Thr Ser Ile Arg Pro
       130                 135                 140
   Arg Val Arg Ala Arg Arg Gly Gln Ala Thr Asp Pro His Ser Ile Ala
   145                 150                 155                 160
   Glu Arg Leu Arg Arg Glu Arg Ile Ala Glu Arg Ile Arg Ala Leu Gln
                   165                 170                 175
   Glu Leu Val Pro Thr Val Asn Lys Thr Asp Arg Ala Ala Met Ile Asp
               180                 185                 190
   Glu Ile Val Asp Tyr Val Lys Phe Leu Arg Leu Gln Val Lys Val Leu
           195                 200                 205
   Ser Met Asn Arg Leu Gly Gly Ala Gly Ala Val Ala Pro Leu Val Thr
       210                 215                 220
   Asp Met Pro Leu Ser Ser Ser Val Glu Asp Glu Thr Gly Glu Gly Gly
   225                 230                 235                 240
   Arg Thr Pro Gln Pro Ala Trp Glu Lys Trp Ser Asn Asp Gly Thr Glu
                   245                 250                 255
   Arg Gln Val Ala Lys Leu Met Glu Glu Asn Val Gly Ala Ala Met Gln
               260                 265                 270
   Leu Leu Gln Ser Lys Ala Leu Cys Met Met Pro Ile Ser Leu Ala Met
           275                 280                 285
   Ala Ile Tyr His Ser Gln Pro Pro Asp Thr Ser Ser Val Val Lys Pro
       290                 295                 300
   Glu Asn Asn Pro Pro Gln
   305                 310
<210> 21
   <211> 1179
   <212> DNA
   <213> Arabidopsis thaliana
<220>
   <221> CDS
   <222> (109)..(966)
   <223> G525
<400> 21
   cttctctctt ctcaaaaacc cttccctctt cgtctccaaa caacaacaaa cacaacaaca      60
   acaaaaatct tacaagaaga tcatttttag aaaccctatt aggataaa atg gat tac      117
                                                        Met Asp Tyr
                                                        1
   gag gca tca aga atc gtc gaa atg gta gaa gat gaa gaa cat ata gat      165
   Glu Ala Ser Arg Ile Val Glu Met Val Glu Asp Glu Glu His Ile Asp
       5                   10                  15
   cta cca cca gga ttc aga ttt cac cct act gat gaa gaa ctc ata act      213
   Leu Pro Pro Gly Phe Arg Phe His Pro Thr Asp Glu Glu Leu Ile Thr
   20                  25                  30                  35
   cac tac ctc aaa cca aag gtt ttc aac act ttc ttc tct gct act gcc      261
   His Tyr Leu Lys Pro Lys Val Phe Asn Thr Phe Phe Ser Ala Thr Ala
                   40                  45                  50
   att ggt gaa gtt gat ctc aac aag att gag cct tgg gac tta cca tgg      309
   Ile Gly Glu Val Asp Leu Asn Lys Ile Glu Pro Trp Asp Leu Pro Trp
               55                  60                  65
   aag gct aag atg gga gaa aaa gaa tgg tat ttc ttc tgt gtg aga gac      357
   Lys Ala Lys Met Gly Glu Lys Glu Trp Tyr Phe Phe Cys Val Arg Asp
           70                  75                  80
   cgg aaa tac ccg acc ggt tta agg aca aac cgg gcg aca gaa gcc ggt      405
   Arg Lys Tyr Pro Thr Gly Leu Arg Thr Asn Arg Ala Thr Glu Ala Gly
       85                  90                  95
   tat tgg aaa gcc aca gga aaa gac aaa gag ata ttc aag gga aaa tca      453
   Tyr Trp Lys Ala Thr Gly Lys Asp Lys Glu Ile Phe Lys Gly Lys Ser
   100                 105                 110                 115
   ctt gtg ggt atg aag aaa act ttg gtt ttc tat aaa gga aga gct cct      501
   Leu Val Gly Met Lys Lys Thr Leu Val Phe Tyr Lys Gly Arg Ala Pro
                   120                 125                 130
   aaa gga gtt aaa acc aat tgg gtt atg cat gaa tat cgt tta gaa ggc      549
   Lys Gly Val Lys Thr Asn Trp Val Met His Glu Tyr Arg Leu Glu Gly
               135                 140                 145
   aaa tat tgt att gaa aat ctt ccc caa aca gct aag aac gaa tgg gtt      597
   Lys Tyr Cys Ile Glu Asn Leu Pro Gln Thr Ala Lys Asn Glu Trp Val
           150                 155                 160
   ata tgt cgt gtt ttc caa aaa cgt gcc gat ggt aca aag gtt cca atg      645
   Ile Cys Arg Val Phe Gln Lys Arg Ala Asp Gly Thr Lys Val Pro Met
       165                 170                 175
   tca atg ctt gat cca cac att aac cga atg gaa cca gcc ggt tta cct      693
   Ser Met Leu Asp Pro His Ile Asn Arg Met Glu Pro Ala Gly Leu Pro
   180                 185                 190                 195
   tcg tta atg gat tgt tct caa cga gac tcc ttc acc ggt tcg tcg tct      741
   Ser Leu Met Asp Cys Ser Gln Arg Asp Ser Phe Thr Gly Ser Ser Ser
                   200                 205                 210
   cac gtg acc tgc ttc tcc gac caa gaa acc gaa gac aaa aga ctt gtc      789
   His Val Thr Cys Phe Ser Asp Gln Glu Thr Glu Asp Lys Arg Leu Val
               215                 220                 225
   cac gag tcc aaa gac ggt ttt ggt tct ctg ttt tac tcg gat cct ctg      837
   His Glu Ser Lys Asp Gly Phe Gly Ser Leu Phe Tyr Ser Asp Pro Leu
           230                 235                 240
   ttt tta caa gac aat tat tcg cta atg aag ctg ttg ctt gac ggt caa      885
   Phe Leu Gln Asp Asn Tyr Ser Leu Met Lys Leu Leu Leu Asp Gly Gln
       245                 250                 255
   gaa act caa ttc tcc ggc aaa cct ttc gac ggt cgt gat tcg tcc ggt      933
   Glu Thr Gln Phe Ser Gly Lys Pro Phe Asp Gly Arg Asp Ser Ser Gly
   260                 265                 270                 275
   aca gaa gaa ttg gat tgc gtt tgg aat ttc tga gttgtataag ttatgttgta    986
   Thr Glu Glu Leu Asp Cys Val Trp Asn Phe
                   280                 285
   gacttgtagt agtcatgtgt tcgtgtgtgt gaatgaatat tcttgttaca tttttttgta   1046
   aaaaaggaga aaaaaatatg ctagaaagtc aattgctttt gttatgtagc attagtgttt   1106
   tttatgtact caatagactt cctaattaaa taaaaatctt aatttatttg ccaaaaaaaa   1166
   aaaaaaaaaa aaa                                                       1179
<210> 22
   <211> 285
   <212> PRT
   <213> Arabidopsis thaliana
<400> 22
   Met Asp Tyr Glu Ala Ser Arg Ile Val Glu Met Val Glu Asp Glu Glu
   1               5                   10                  15
   His Ile Asp Leu Pro Pro Gly Phe Arg Phe His Pro Thr Asp Glu Glu
               20                  25                  30
   Leu Ile Thr His Tyr Leu Lys Pro Lys Val Phe Asn Thr Phe Phe Ser
           35                  40                  45
   Ala Thr Ala Ile Gly Glu Val Asp Leu Asn Lys Ile Glu Pro Trp Asp
       50                  55                  60
   Leu Pro Trp Lys Ala Lys Met Gly Glu Lys Glu Trp Tyr Phe Phe Cys
   65                  70                  75                  80
   Val Arg Asp Arg Lys Tyr Pro Thr Gly Leu Arg Thr Asn Arg Ala Thr
                   85                  90                  95
   Glu Ala Gly Tyr Trp Lys Ala Thr Gly Lys Asp Lys Glu Ile Phe Lys
               100                 105                 110
   Gly Lys Ser Leu Val Gly Met Lys Lys Thr Leu Val Phe Tyr Lys Gly
           115                 120                 125
   Arg Ala Pro Lys Gly Val Lys Thr Asn Trp Val Met His Glu Tyr Arg
       130                 135                 140
   Leu Glu Gly Lys Tyr Cys Ile Glu Asn Leu Pro Gln Thr Ala Lys Asn
   145                 150                 155                 160
   Glu Trp Val Ile Cys Arg Val Phe Gln Lys Arg Ala Asp Gly Thr Lys
                   165                 170                 175
   Val Pro Met Ser Met Leu Asp Pro His Ile Asn Arg Met Glu Pro Ala
               180                 185                 190
   Gly Leu Pro Ser Leu Met Asp Cys Ser Gln Arg Asp Ser Phe Thr Gly
           195                 200                 205
   Ser Ser Ser His Val Thr Cys Phe Ser Asp Gln Glu Thr Glu Asp Lys
       210                 215                 220
   Arg Leu Val His Glu Ser Lys Asp Gly Phe Gly Ser Leu Phe Tyr Ser
   225                 230                 235                 240
   Asp Pro Leu Phe Leu Gln Asp Asn Tyr Ser Leu Met Lys Leu Leu Leu
                   245                 250                 255
   Asp Gly Gln Glu Thr Gln Phe Ser Gly Lys Pro Phe Asp Gly Arg Asp
               260                 265                 270
   Ser Ser Gly Thr Glu Glu Leu Asp Cys Val Trp Asn Phe
           275                 280                 285
<210> 23
   <211> 890
   <212> DNA
   <213> Arabidopsis thaliana
<220>
   <221> CDS
   <222> (55)..(738)
   <223> G545
<400> 23
   gcaaccttca aactaaaact cgagagacaa gaaatcctca gaatctttaa ctta atg      57
                                                               Met
                                                               1
   gcg ctc gag gct ctt aca tca cca aga tta gct tct ccg att cct cct     105
   Ala Leu Glu Ala Leu Thr Ser Pro Arg Leu Ala Ser Pro Ile Pro Pro
               5                   10                  15
   ttg ttc gaa gat tct tca gtc ttc cat gga gtc gag cac tgg aca aag     153
   Leu Phe Glu Asp Ser Ser Val Phe His Gly Val Glu His Trp Thr Lys
           20                  25                  30
   ggt aag cga tct aag aga tca aga tcc gat ttc cac cac caa aac ctc     201
   Gly Lys Arg Ser Lys Arg Ser Arg Ser Asp Phe His His Gln Asn Leu
       35                  40                  45
   act gag gaa gag tat cta gct ttt tgc ctc atg ctt ctc gct cgc gac     249
   Thr Glu Glu Glu Tyr Leu Ala Phe Cys Leu Met Leu Leu Ala Arg Asp
   50                  55                  60                  65
   aac cgt cag cct cct cct cct ccg gcg gtg gag aag ttg agc tac aag     297
   Asn Arg Gln Pro Pro Pro Pro Pro Ala Val Glu Lys Leu Ser Tyr Lys
                   70                  75                  80
   tgt agc gtc tgc gac aag acg ttc tct tct tac caa gct ctc ggt ggt     345
   Cys Ser Val Cys Asp Lys Thr Phe Ser Ser Tyr Gln Ala Leu Gly Gly
               85                  90                  95
   cac aag gca agc cac cgt aag aac tta tca cag act ctc tcc ggc gga     393
   His Lys Ala Ser His Arg Lys Asn Leu Ser Gln Thr Leu Ser Gly Gly
           100                 105                 110
   gga gat gat cat tca acc tcg tcg gcg aca acc aca tcc gcc gtg act     441
   Gly Asp Asp His Ser Thr Ser Ser Ala Thr Thr Thr Ser Ala Val Thr
       115                 120                 125
   act gga agt ggg aaa tca cac gtt tgc acc atc tgt aac aag tct ttt     489
   Thr Gly Ser Gly Lys Ser His Val Cys Thr Ile Cys Asn Lys Ser Phe
   130                 135                 140                 145
   cct tcc ggt caa gct ctc ggc gga cac aag cgg tgc cac tac gaa gga     537
   Pro Ser Gly Gln Ala Leu Gly Gly His Lys Arg Cys His Tyr Glu Gly
                   150                 155                 160
   aac aac aac atc aac act agt agc gtg tcc aac tcc gaa ggt gcg ggg     585
   Asn Asn Asn Ile Asn Thr Ser Ser Val Ser Asn Ser Glu Gly Ala Gly
               165                 170                 175
   tcc act agc cac gtt agc agt agc cac cgt ggg ttt gac ctc aac atc      633
   Ser Thr Ser His Val Ser Ser Ser His Arg Gly Phe Asp Leu Asn Ile
           180                 185                 190
   cct ccg atc cct gaa ttc tcg atg gtc aac gga gac gac gaa gtc atg      681
   Pro Pro Ile Pro Glu Phe Ser Met Val Asn Gly Asp Asp Glu Val Met
       195                 200                 205
   agc cct atg ccg gcg aag aag cct cgg ttt gac ttt ccg gtc aaa ctt      729
   Ser Pro Met Pro Ala Lys Lys Pro Arg Phe Asp Phe Pro Val Lys Leu
   210                 215                 220                 225
   caa ctt taa ggaaatttac ttagacgata agatttcgtt tgtatactgt              778
   Gln Leu
   tgagagttgt gtaggaattt gttgactgta cataccaaat tggactttga ctgattccaa    838
   ttcttcttgt tctttcattt taaaaattat taaaccgatt ctttaccaca aa            890
<210> 24
   <211> 227
   <212> PRT
   <213> Arabidopsis thaliana
<400> 24
   Met Ala Leu Glu Ala Leu Thr Ser Pro Arg Leu Ala Ser Pro Ile Pro
   1               5                   10                  15
   Pro Leu Phe Glu Asp Ser Ser Val Phe His Gly Val Glu His Trp Thr
               20                  25                  30
   Lys Gly Lys Arg Ser Lys Arg Ser Arg Ser Asp Phe His His Gln Asn
           35                  40                  45
   Leu Thr Glu Glu Glu Tyr Leu Ala Phe Cys Leu Met Leu Leu Ala Arg
       50                  55                  60
   Asp Asn Arg Gln Pro Pro Pro Pro Pro Ala Val Glu Lys Leu Ser Tyr
   65                  70                  75                  80
   Lys Cys Ser Val Cys Asp Lys Thr Phe Ser Ser Tyr Gln Ala Leu Gly
                   85                  90                  95
   Gly His Lys Ala Ser His Arg Lys Asn Leu Ser Gln Thr Leu Ser Gly
               100                 105                 110
   Gly Gly Asp Asp His Ser Thr Ser Ser Ala Thr Thr Thr Ser Ala Val
           115                 120                 125
   Thr Thr Gly Ser Gly Lys Ser His Val Cys Thr Ile Cys Asn Lys Ser
       130                 135                 140
   Phe Pro Ser Gly Gln Ala Leu Gly Gly His Lys Arg Cys His Tyr Glu
   145                 150                 155                 160
   Gly Asn Asn Asn Ile Asn Thr Ser Ser Val Ser Asn Ser Glu Gly Ala
                   165                 170                 175
   Gly Ser Thr Ser His Val Ser Ser Ser His Arg Gly Phe Asp Leu Asn
               180                 185                 190
   Ile Pro Pro Ile Pro Glu Phe Ser Met Val Asn Gly Asp Asp Glu Val
           195                 200                 205
   Met Ser Pro Met Pro Ala Lys Lys Pro Arg Phe Asp Phe Pro Val Lys
       210                 215                 220
   Leu Gln Leu
   225
<210> 25
   <211> 1126
   <212> DNA
   <213> Arabidopsis thaliana
<220>
   <221> CDS
   <222> (282)..(920)
   <223> G865
<400> 25
   atccccactt gttgttcatc accaagccaa gctccatgtc ctagtcactc cacagattcc    60
   ctatcatcat caattcgttt caaacttagt tcctttcaaa gtcttgtaca tatatacaca   120
   cacacctatt attctcttgg tgtgtttgtg tgttacatat acgtgtgagt acatactttg   180
   ttgtaaaagt ggatcggagg tatggaaagg gaccggttcc accggaaaca tcggcggcgg   240
   cggatgataa ttcgtcttgg aacgagactg atgtcaccgc c atg gtc tcc gct ctc   296
                                                 Met Val Ser Ala Leu
                                                 1               5
   agc cgt gtc ata gag aat ccg aca gac ccg ccg gtc aaa caa gag ctt    344
   Ser Arg Val Ile Glu Asn Pro Thr Asp Pro Pro Val Lys Gln Glu Leu
                   10                  15                  20
   gat aaa tcg gat caa cat caa cca gac caa gat caa cca aga aga aga    392
   Asp Lys Ser Asp Gln His Gln Pro Asp Gln Asp Gln Pro Arg Arg Arg
               25                  30                  35
   cac tat aga ggc gta agg cag aga cca tgg ggt aaa tgg gcg gca gaa    440
   His Tyr Arg Gly Val Arg Gln Arg Pro Trp Gly Lys Trp Ala Ala Glu
           40                  45                  50
   atc cgc gat cca aag aaa gca gcc cgt gtc tgg ctc ggg act ttc gag    488
   Ile Arg Asp Pro Lys Lys Ala Ala Arg Val Trp Leu Gly Thr Phe Glu
       55                  60                  65
   acg gca gag gaa gct gct tta gcc tat gac cga gct gcc ctc aaa ttc    536
   Thr Ala Glu Glu Ala Ala Leu Ala Tyr Asp Arg Ala Ala Leu Lys Phe
   70                  75                  80                  85
   aaa ggc acc aag gct aaa ctg aac ttc cct gaa cgg gtc caa ggc cct    584
   Lys Gly Thr Lys Ala Lys Leu Asn Phe Pro Glu Arg Val Gln Gly Pro
                   90                  95                  100
   act acc acc aca acc att tct cat gca cca aga gga gtt agt gaa tcc    632
   Thr Thr Thr Thr Thr Ile Ser His Ala Pro Arg Gly Val Ser Glu Ser
               105                 110                 115
   atg aac tca cct cct cct cga cct ggt cca cct tca act act act act    680
   Met Asn Ser Pro Pro Pro Arg Pro Gly Pro Pro Ser Thr Thr Thr Thr
           120                 125                 130
   tcg tgg cca atg act tat aac cag gac ata ctt caa tac gct cag ttg    728
   Ser Trp Pro Met Thr Tyr Asn Gln Asp Ile Leu Gln Tyr Ala Gln Leu
       135                 140                 145
   ctt acg agt aac aat gag gtt gat tta tca tac tac acg tcg act ctc    776
   Leu Thr Ser Asn Asn Glu Val Asp Leu Ser Tyr Tyr Thr Ser Thr Leu
   150                 155                 160                 165
   ttc agt caa cct ttt tca acg cct tct tca tct tct tct tcc tcc caa    824
   Phe Ser Gln Pro Phe Ser Thr Pro Ser Ser Ser Ser Ser Ser Ser Gln
                   170                 175                 180
   cag acg cag caa cag cag cta caa caa caa caa cag cag cgt gaa gaa    872
   Gln Thr Gln Gln Gln Gln Leu Gln Gln Gln Gln Gln Gln Arg Glu Glu
               185                 190                 195
   gaa gag aag aat tat ggt tac aat tat tat aac tac cca aga gaa taa    920
   Glu Glu Lys Asn Tyr Gly Tyr Asn Tyr Tyr Asn Tyr Pro Arg Glu
           200                 205                 210
   tctaattatt attgttggtc gaatcagttt tataaatagc tatcatagtt tcatttttgg   980
   tttccgtaac ctttgttgca tggaaaatat gaatgaacga gggacatgtg taacaatttg  1040
   tttgtgtttc gtaaatgtta gttgtatttg gatttgctga agtttgattt tctgagcata  1100
   aatcatttga cggtcaaaaa aaaaaa                                      1126
<210> 26
   <211> 212
   <212> PRT
   <213> Arabidopsis thaliana
<400> 26
   Met Val Ser Ala Leu Ser Arg Val Ile Glu Asn Pro Thr Asp Pro Pro
   1               5                   10                  15
   Val Lys Gln Glu Leu Asp Lys Ser Asp Gln His Gln Pro Asp Gln Asp
               20                  25                  30
   Gln Pro Arg Arg Arg His Tyr Arg Gly Val Arg Gln Arg Pro Trp Gly
           35                  40                  45
   Lys Trp Ala Ala Glu Ile Arg Asp Pro Lys Lys Ala Ala Arg Val Trp
       50                  55                  60
   Leu Gly Thr Phe Glu Thr Ala Glu Glu Ala Ala Leu Ala Tyr Asp Arg
   65                  70                  75                  80
   Ala Ala Leu Lys Phe Lys Gly Thr Lys Ala Lys Leu Asn Phe Pro Glu
                   85                  90                  95
   Arg Val Gln Gly Pro Thr Thr Thr Thr Thr Ile Ser His Ala Pro Arg
               100                 105                 110
   Gly Val Ser Glu Ser Met Asn Ser Pro Pro Pro Arg Pro Gly Pro Pro
           115                 120                 125
   Ser Thr Thr Thr Thr Ser Trp Pro Met Thr Tyr Asn Gln Asp Ile Leu
       130                 135                 140
   Gln Tyr Ala Gln Leu Leu Thr Ser Asn Asn Glu Val Asp Leu Ser Tyr
   145                 150                 155                 160
   Tyr Thr Ser Thr Leu Phe Ser Gln Pro Phe Ser Thr Pro Ser Ser Ser
                   165                 170                 175
   Ser Ser Ser Ser Gln Gln Thr Gln Gln Gln Gln Leu Gln Gln Gln Gln
               180                 185                 190
   Gln Gln Arg Glu Glu Glu Glu Lys Asn Tyr Gly Tyr Asn Tyr Tyr Asn
           195                 200                 205
   Tyr Pro Arg Glu
       210
<210> 27
   <211> 1152
   <212> DNA
   <213> Arabidopsis thaliana
<220>
   <221> CDS
   <222> (76)..(1008)
   <223> G881
<400> 27
   gtgaccgaag aaagcaaatt gagactacgc accaactagt cctttggttt gtatcttaag      60
   ataaaggttt ctttt atg gac ggt tct tcg ttt ctc gac atc tct ctc gat      111
                    Met Asp Gly Ser Ser Phe Leu Asp Ile Ser Leu Asp
                    1               5                   10
   ctc aac acc aat cct ttc tcc gca aaa ctt ccg aag aag gag gtc tca       159
   Leu Asn Thr Asn Pro Phe Ser Ala Lys Leu Pro Lys Lys Glu Val Ser
           15                  20                  25
   gtt ttg gct tct act cac tta aag agg aaa tgg ttg gag caa gac gag       207
   Val Leu Ala Ser Thr His Leu Lys Arg Lys Trp Leu Glu Gln Asp Glu
       30                  35                  40
   agc gca agt gag tta cga gag gag cta aac aga gtt aat tca gag aac       255
   Ser Ala Ser Glu Leu Arg Glu Glu Leu Asn Arg Val Asn Ser Glu Asn
   45                  50                  55                  60
   aag aag cta aca gag atg tta gct aga gtc tgt gag agc tac aac gaa       303
   Lys Lys Leu Thr Glu Met Leu Ala Arg Val Cys Glu Ser Tyr Asn Glu
                   65                  70                  75
   cta cat aat cat ttg gag aag ctt cag agt cgc cag agc cct gaa atc       351
   Leu His Asn His Leu Glu Lys Leu Gln Ser Arg Gln Ser Pro Glu Ile
               80                  85                  90
   gag cag acc gat ata ccg ata aag aaa aga aaa caa gac ccg gat gag       399
   Glu Gln Thr Asp Ile Pro Ile Lys Lys Arg Lys Gln Asp Pro Asp Glu
           95                  100                 105
   ttc tta ggc ttt cct att gga ctc agt agt gga aaa act gag aac agc       447
   Phe Leu Gly Phe Pro Ile Gly Leu Ser Ser Gly Lys Thr Glu Asn Ser
       110                 115                 120
   tcc agc aac gaa gat cat cat cat cat cat cag caa cat gag cag aaa       495
   Ser Ser Asn Glu Asp His His His His His Gln Gln His Glu Gln Lys
   125                 130                 135                 140
   aat cag ctt ctt tca tgt aaa aga cca gtc act gat agc ttc aac aaa       543
   Asn Gln Leu Leu Ser Cys Lys Arg Pro Val Thr Asp Ser Phe Asn Lys
                   145                 150                 155
   gca aaa gtt tcg act gtc tac gtg cct act gaa aca tcg gac aca agc       591
   Ala Lys Val Ser Thr Val Tyr Val Pro Thr Glu Thr Ser Asp Thr Ser
               160                 165                 170
   ttg aca gtt aaa gat gga ttt caa tgg agg aaa tac gga caa aag gtt       639
   Leu Thr Val Lys Asp Gly Phe Gln Trp Arg Lys Tyr Gly Gln Lys Val
           175                 180                 185
   aca aga gac aac ccg tca cct aga gct tac ttt aga tgc tcg ttt gca      687
   Thr Arg Asp Asn Pro Ser Pro Arg Ala Tyr Phe Arg Cys Ser Phe Ala
       190                 195                 200
   ccg tct tgt cca gta aaa aag aag gta caa cgc agc gca gag gat cca      735
   Pro Ser Cys Pro Val Lys Lys Lys Val Gln Arg Ser Ala Glu Asp Pro
   205                 210                 215                 220
   tct tta ctt gta gcg aca tac gaa ggg acg cat aac cac ttg ggt cca      783
   Ser Leu Leu Val Ala Thr Tyr Glu Gly Thr His Asn His Leu Gly Pro
                   225                 230                 235
   aat gct tct gaa ggg gat gct aca agc cag ggt ggg tca agc aca gtg      831
   Asn Ala Ser Glu Gly Asp Ala Thr Ser Gln Gly Gly Ser Ser Thr Val
               240                 245                 250
   act ttg gat ctg gtt aat ggc tgt cat aga cta gcg ttg gag aaa aac      879
   Thr Leu Asp Leu Val Asn Gly Cys His Arg Leu Ala Leu Glu Lys Asn
           255                 260                 265
   gaa agg gat aat acg atg caa gag gtt ctg att caa caa atg gcg tca      927
   Glu Arg Asp Asn Thr Met Gln Glu Val Leu Ile Gln Gln Met Ala Ser
       270                 275                 280
   tcg tta aca aaa gat tcg aaa ttt aca gct gct ctt gct gct gct ata      975
   Ser Leu Thr Lys Asp Ser Lys Phe Thr Ala Ala Leu Ala Ala Ala Ile
   285                 290                 295                 300
   tct ggg agg tta atg gag caa tct aga aca tga acgtttttag tgaatgtatt   1028
   Ser Gly Arg Leu Met Glu Gln Ser Arg Thr
                   305                 310
   gtttttgttt gtttagaatg attcttcgtt ttcgaattgt gtctttcgat taggagataa   1088
   aagatgtata taaatattat aagtagatga agaaatcgta taagtaaaaa aaaaaaaaaa   1148
   aaaa                                                                1152
<210> 28
   <211> 310
   <212> PRT
   <213> Arabidopsis thaliana
<400> 28
   Met Asp Gly Ser Ser Phe Leu Asp Ile Ser Leu Asp Leu Asn Thr Asn
   1               5                   10                  15
   Pro Phe Ser Ala Lys Leu Pro Lys Lys Glu Val Ser Val Leu Ala Ser
               20                  25                  30
   Thr His Leu Lys Arg Lys Trp Leu Glu Gln Asp Glu Ser Ala Ser Glu
           35                  40                  45
   Leu Arg Glu Glu Leu Asn Arg Val Asn Ser Glu Asn Lys Lys Leu Thr
       50                  55                  60
   Glu Met Leu Ala Arg Val Cys Glu Ser Tyr Asn Glu Leu His Asn His
   65                  70                  75                  80
   Leu Glu Lys Leu Gln Ser Arg Gln Ser Pro Glu Ile Glu Gln Thr Asp
                   85                  90                  95
   Ile Pro Ile Lys Lys Arg Lys Gln Asp Pro Asp Glu Phe Leu Gly Phe
               100                 105                 110
   Pro Ile Gly Leu Ser Ser Gly Lys Thr Glu Asn Ser Ser Ser Asn Glu
               115                 120                 125
   Asp His His His His His Gln Gln His Glu Gln Lys Asn Gln Leu Leu
       130                 135                 140
   Ser Cys Lys Arg Pro Val Thr Asp Ser Phe Asn Lys Ala Lys Val Ser
   145                 150                 155                 160
   Thr Val Tyr Val Pro Thr Glu Thr Ser Asp Thr Ser Leu Thr Val Lys
                   165                 170                 175
   Asp Gly Phe Gln Trp Arg Lys Tyr Gly Gln Lys Val Thr Arg Asp Asn
               180                 185                 190
   Pro Ser Pro Arg Ala Tyr Phe Arg Cys Ser Phe Ala Pro Ser Cys Pro
           195                 200                 205
   Val Lys Lys Lys Val Gln Arg Ser Ala Glu Asp Pro Ser Leu Leu Val
       210                 215                 220
   Ala Thr Tyr Glu Gly Thr His Asn His Leu Gly Pro Asn Ala Ser Glu
   225                 230                 235                 240
   Gly Asp Ala Thr Ser Gln Gly Gly Ser Ser Thr Val Thr Leu Asp Leu
                   245                 250                 255
   Val Asn Gly Cys His Arg Leu Ala Leu Glu Lys Asn Glu Arg Asp Asn
               260                 265                 270
   Thr Met Gln Glu Val Leu Ile Gln Gln Met Ala Ser Ser Leu Thr Lys
           275                 280                 285
   Asp Ser Lys Phe Thr Ala Ala Leu Ala Ala Ala Ile Ser Gly Arg Leu
       290                 295                 300
   Met Glu Gln Ser Arg Thr
   305                 310
<210> 29
   <211> 1276
   <212> DNA
   <213> Arabidopsis thaliana
<220>
   <221> CDS
   <222> (47)..(1150)
   <223> G896
<400> 29
   taatccgatt cgtcttcatc tgattccctc ccttccgaga ataata atg tac ccg          55
                                                      Met Tyr Pro
                                                      1
   cca cct ccc tca agc atc tac gct cct ccg atg ctg gtg aat tgc tcc        103
   Pro Pro Pro Ser Ser Ile Tyr Ala Pro Pro Met Leu Val Asn Cys Ser
       5                   10                  15
   ggt tgc cgg acg cct ctc cag ctc cca tcc ggc gcc cga tct att cgc        151
   Gly Cys Arg Thr Pro Leu Gln Leu Pro Ser Gly Ala Arg Ser Ile Arg
   20                  25                  30                  35
   tgc gct ctc tgc cag gct gtt act cat atc gcc gac cct cgc acc gcc      199
   Cys Ala Leu Cys Gln Ala Val Thr His Ile Ala Asp Pro Arg Thr Ala
                   40                  45                  50
   cct cct ccg caa cct tcc tcc gcc cct tct ccg cct ccc caa atc cac      247
   Pro Pro Pro Gln Pro Ser Ser Ala Pro Ser Pro Pro Pro Gln Ile His
               55                  60                  65
   gcg cct ccc ggt cag ctg cct cac ccc cat ggc agg aag agg gcc gtg      295
   Ala Pro Pro Gly Gln Leu Pro His Pro His Gly Arg Lys Arg Ala Val
           70                  75                  80
   atc tgt ggc atc tcg tat cgt ttc tct cgc cac gag ctc aaa ggc tgc      343
   Ile Cys Gly Ile Ser Tyr Arg Phe Ser Arg His Glu Leu Lys Gly Cys
       85                  90                  95
   atc aac gac gcc aag tgc atg cgt cac ctt ctc atc aac aaa ttc aaa      391
   Ile Asn Asp Ala Lys Cys Met Arg His Leu Leu Ile Asn Lys Phe Lys
   100                 105                 110                 115
   ttc tcc cca gat tca att ctc atg ctt acc gag gaa gaa act gat cca      439
   Phe Ser Pro Asp Ser Ile Leu Met Leu Thr Glu Glu Glu Thr Asp Pro
                   120                 125                 130
   tat cgt atc ccg acc aag caa aac atg agg atg gca ttg tat tgg ctc      487
   Tyr Arg Ile Pro Thr Lys Gln Asn Met Arg Met Ala Leu Tyr Trp Leu
               135                 140                 145
   gta cag gga tgc aca gca ggc gac tca ctt gtc ttc cac tac tct ggt      535
   Val Gln Gly Cys Thr Ala Gly Asp Ser Leu Val Phe His Tyr Ser Gly
           150                 155                 160
   cat ggt tcg cgt caa aga aac tac aac ggt gat gaa gtt gat ggc tat      583
   His Gly Ser Arg Gln Arg Asn Tyr Asn Gly Asp Glu Val Asp Gly Tyr
       165                 170                 175
   gat gaa aca ctc tgt cct ctg gat ttt gaa act cag ggg atg att gta      631
   Asp Glu Thr Leu Cys Pro Leu Asp Phe Glu Thr Gln Gly Met Ile Val
   180                 185                 190                 195
   gac gat gag atc aac gca acc att gta cgc cct ctt cca cat ggt gtc      679
   Asp Asp Glu Ile Asn Ala Thr Ile Val Arg Pro Leu Pro His Gly Val
                   200                 205                 210
   aag ctc cat tca att atc gat gct tgc cat agt ggt acc gtt ctg gat      727
   Lys Leu His Ser Ile Ile Asp Ala Cys His Ser Gly Thr Val Leu Asp
               215                 220                 225
   tta ccc ttc cta tgc aga atg aac aga gct ggg cag tat gtg tgg gag      775
   Leu Pro Phe Leu Cys Arg Met Asn Arg Ala Gly Gln Tyr Val Trp Glu
           230                 235                 240
   gat cat cgg cct agg tca ggt ttg tgg aaa gga act gct ggt gga gaa      823
   Asp His Arg Pro Arg Ser Gly Leu Trp Lys Gly Thr Ala Gly Gly Glu
       245                 250                 255
   gcc att tca att agt gga tgt gat gat gat cag act tcg gcc gac aca      871
   Ala Ile Ser Ile Ser Gly Cys Asp Asp Asp Gln Thr Ser Ala Asp Thr
   260                 265                 270                 275
   tca gcg ctg tcg aag atc acg tct acg ggt gct atg act ttc tgt ttt      919
   Ser Ala Leu Ser Lys Ile Thr Ser Thr Gly Ala Met Thr Phe Cys Phe
                   280                 285                 290
   att caa gca att gaa cgc agc gca caa ggc aca acc tat gga agc ctt      967
   Ile Gln Ala Ile Glu Arg Ser Ala Gln Gly Thr Thr Tyr Gly Ser Leu
               295                 300                 305
   ctg aat tct atg cgc acc aca ata agg aat aca ggg aat gat ggt ggt     1015
   Leu Asn Ser Met Arg Thr Thr Ile Arg Asn Thr Gly Asn Asp Gly Gly
           310                 315                 320
   ggt agt ggt gga gtt gtg acg act gtg ctg agc atg ctt ctg aca ggg     1063
   Gly Ser Gly Gly Val Val Thr Thr Val Leu Ser Met Leu Leu Thr Gly
       325                 330                 335
   gga agt gcg att ggg gga tta aga cag gag cct caa ctg act gct tgc      1111
   Gly Ser Ala Ile Gly Gly Leu Arg Gln Glu Pro Gln Leu Thr Ala Cys
   340                 345                 350                 355
   caa aca ttc gat gtc tat gca aag cct ttc act ctc tag taaaggacaa       1160
   Gln Thr Phe Asp Val Tyr Ala Lys Pro Phe Thr Leu
                   360                 365
   gtcacttttt atgtatagcg agtgtgattt gagaatccgt ccatataacc accttttgtt   1220
   tcttattttt atttttcttt caaaagaata aaggaaaaca ttgatttggt gattcg       1276
<210> 30
   <211> 367
   <212> PRT
   <213> Arabidopsis thaliana
<400> 30
   Met Tyr Pro Pro Pro Pro Ser Ser Ile Tyr Ala Pro Pro Met Leu Val
   1               5                   10                  15
   Asn Cys Ser Gly Cys Arg Thr Pro Leu Gln Leu Pro Ser Gly Ala Arg
               20                  25                  30
   Ser Ile Arg Cys Ala Leu Cys Gln Ala Val Thr His Ile Ala Asp Pro
           35                  40                  45
   Arg Thr Ala Pro Pro Pro Gln Pro Ser Ser Ala Pro Ser Pro Pro Pro
       50                  55                  60
   Gln Ile His Ala Pro Pro Gly Gln Leu Pro His Pro His Gly Arg Lys
   65                  70                  75                  80
   Arg Ala Val Ile Cys Gly Ile Ser Tyr Arg Phe Ser Arg His Glu Leu
                   85                  90                  95
   Lys Gly Cys Ile Asn Asp Ala Lys Cys Met Arg His Leu Leu Ile Asn
               100                 105                 110
   Lys Phe Lys Phe Ser Pro Asp Ser Ile Leu Met Leu Thr Glu Glu Glu
           115                 120                 125
   Thr Asp Pro Tyr Arg Ile Pro Thr Lys Gln Asn Met Arg Met Ala Leu
       130                 135                 140
   Tyr Trp Leu Val Gln Gly Cys Thr Ala Gly Asp Ser Leu Val Phe His
   145                 150                 155                 160
   Tyr Ser Gly His Gly Ser Arg Gln Arg Asn Tyr Asn Gly Asp Glu Val
                   165                 170                 175
   Asp Gly Tyr Asp Glu Thr Leu Cys Pro Leu Asp Phe Glu Thr Gln Gly
               180                 185                 190
   Met Ile Val Asp Asp Glu Ile Asn Ala Thr Ile Val Arg Pro Leu Pro
           195                 200                 205
   His Gly Val Lys Leu His Ser Ile Ile Asp Ala Cys His Ser Gly Thr
   210 215 220
   Val Leu Asp Leu Pro Phe Leu Cys Arg Met Asn Arg Ala Gly Gln Tyr
   225 230 235 240
   Val Trp Glu Asp His Arg Pro Arg Ser Gly Leu Trp Lys Gly Thr Ala
   245 250 255
   Gly Gly Glu Ala Ile Ser Ile Ser Gly Cys Asp Asp Asp Gln Thr Ser
   260 265 270
   Ala Asp Thr Ser Ala Leu Ser Lys Ile Thr Ser Thr Gly Ala Met Thr
   275 280 285
   Phe Cys Phe Ile Gln Ala Ile Glu Arg Ser Ala Gln Gly Thr Thr Tyr
   290 295 300
   Gly Ser Leu Leu Asn Ser Met Arg Thr Thr Ile Arg Asn Thr Gly Asn
   305 310 315 320
   Asp Gly Gly Gly Ser Gly Gly Val Val Thr Thr Val Leu Ser Met Leu
   325 330 335
   Leu Thr Gly Gly Ser Ala Ile Gly Gly Leu Arg Gln Glu Pro Gln Leu
   340 345 350
   Thr Ala Cys Gln Thr Phe Asp Val Tyr Ala Lys Pro Phe Thr Leu
   355 360 365
<210> 31
   <211> 726
   <212> DNA
   <213> Arabidopsis thaliana
<220>
   <221> CDS
   <222> (1)..(726)
   <223> G378
<400> 31
   atg gcc tcg tca tca tca tca tct tat aga ttc caa tct ggg tct tac      48
   Met Ala Ser Ser Ser Ser Ser Ser Tyr Arg Phe Gln Ser Gly Ser Tyr
   1               5                   10                  15
   cct ctt tcg tca agt cct tct ctt ggg aat ttc gtc gaa cgc att aaa      96
   Pro Leu Ser Ser Ser Pro Ser Leu Gly Asn Phe Val Glu Arg Ile Lys
               20                  25                  30
   gac gct tgt cat ttc ctt gtc tct gct gtt ttg ggt acc att atc tcc      144
   Asp Ala Cys His Phe Leu Val Ser Ala Val Leu Gly Thr Ile Ile Ser
           35                  40                  45
   gcg atc ttg acc ttc ttc ttc gca cta gtg ggc aca ttg cta ggg gca      192
   Ala Ile Leu Thr Phe Phe Phe Ala Leu Val Gly Thr Leu Leu Gly Ala
       50                  55                  60
   ctt aca gga gct ttg ata ggt caa gaa act gag agt ggt ttc att aga      240
   Leu Thr Gly Ala Leu Ile Gly Gln Glu Thr Glu Ser Gly Phe Ile Arg
   65                  70                  75                  80
   gga gca gca att gga gcc att tcg gga gct gtt ttc tct atc gag gtc      288
   Gly Ala Ala Ile Gly Ala Ile Ser Gly Ala Val Phe Ser Ile Glu Val
                   85                  90                  95
   ttt gaa tca tct ctg gat ctc tgg aaa tcc gat gag tcg ggt ttc gga      336
   Phe Glu Ser Ser Leu Asp Leu Trp Lys Ser Asp Glu Ser Gly Phe Gly
               100                 105                 110
   tgt ttt ctc tac ttg att gat gtc att gtt agt ctt cta agc ggg aga      384
   Cys Phe Leu Tyr Leu Ile Asp Val Ile Val Ser Leu Leu Ser Gly Arg
           115                 120                 125
   ctt gta cga gag cgc att ggt cct gca atg cta agt gca gtg caa agt      432
   Leu Val Arg Glu Arg Ile Gly Pro Ala Met Leu Ser Ala Val Gln Ser
       130                 135                 140
   caa atg gga gct gtg gat aca gct ttt gat gat cac aca agc ctt ttt      480
   Gln Met Gly Ala Val Asp Thr Ala Phe Asp Asp His Thr Ser Leu Phe
   145                 150                 155                 160
   gat aca gga ggc tca aaa gga ttg aca gga gac ctt gtt gag aaa atc      528
   Asp Thr Gly Gly Ser Lys Gly Leu Thr Gly Asp Leu Val Glu Lys Ile
                   165                 170                 175
   cca aag atg aca atc act ggc aac aat aac act gat gct tct gag aac      576
   Pro Lys Met Thr Ile Thr Gly Asn Asn Asn Thr Asp Ala Ser Glu Asn
               180                 185                 190
   aca gac tca tgt tct gtt tgt ctt cag gat ttc cag ctc ggt gaa aca      624
   Thr Asp Ser Cys Ser Val Cys Leu Gln Asp Phe Gln Leu Gly Glu Thr
           195                 200                 205
   gtt aga agc ttg cct cat tgt cat cac atg ttt cac tta cct tgc ata      672
   Val Arg Ser Leu Pro His Cys His His Met Phe His Leu Pro Cys Ile
       210                 215                 220
   gac aat tgg ctc ctt aga cac ggt tct tgc ccg atg tgt aga cgt gat      720
   Asp Asn Trp Leu Leu Arg His Gly Ser Cys Pro Met Cys Arg Arg Asp
   225                 230                 235                 240
   att taa                                                              726
   Ile
<210> 32
   <211> 241
   <212> PRT
   <213> Arabidopsis thaliana
<400> 32
   Met Ala Ser Ser Ser Ser Ser Ser Tyr Arg Phe Gln Ser Gly Ser Tyr
   1               5                   10                  15
   Pro Leu Ser Ser Ser Pro Ser Leu Gly Asn Phe Val Glu Arg Ile Lys
               20                  25                  30
   Asp Ala Cys His Phe Leu Val Ser Ala Val Leu Gly Thr Ile Ile Ser
           35                  40                  45
   Ala Ile Leu Thr Phe Phe Phe Ala Leu Val Gly Thr Leu Leu Gly Ala
       50                  55                  60
   Leu Thr Gly Ala Leu Ile Gly Gln Glu Thr Glu Ser Gly Phe Ile Arg
   65                  70                  75                  80
   Gly Ala Ala Ile Gly Ala Ile Ser Gly Ala Val Phe Ser Ile Glu Val
                   85                  90                  95
   Phe Glu Ser Ser Leu Asp Leu Trp Lys Ser Asp Glu Ser Gly Phe Gly
               100                 105                 110
   Cys Phe Leu Tyr Leu Ile Asp Val Ile Val Ser Leu Leu Ser Gly Arg
           115                 120                 125
   Leu Val Arg Glu Arg Ile Gly Pro Ala Met Leu Ser Ala Val Gln Ser
       130                 135                 140
   Gln Met Gly Ala Val Asp Thr Ala Phe Asp Asp His Thr Ser Leu Phe
   145                 150                 155                 160
   Asp Thr Gly Gly Ser Lys Gly Leu Thr Gly Asp Leu Val Glu Lys Ile
                   165                 170                 175
   Pro Lys Met Thr Ile Thr Gly Asn Asn Asn Thr Asp Ala Ser Glu Asn
               180                 185                 190
   Thr Asp Ser Cys Ser Val Cys Leu Gln Asp Phe Gln Leu Gly Glu Thr
           195                 200                 205
   Val Arg Ser Leu Pro His Cys His His Met Phe His Leu Pro Cys Ile
       210                 215                 220
   Asp Asn Trp Leu Leu Arg His Gly Ser Cys Pro Met Cys Arg Arg Asp
   225                 230                 235                 240
   Ile
<210> 33
   <211> 1370
   <212> DNA
   <213> Arabidopsis thaliana
<220>
   <221> CDS
   <222> (184)..(969)
   <223> G569
<400> 33
   gtcgacccac gcgtccgggt ttttctttta tcctcttatc gctaatctgg agctctatat      60
   agactataaa gggtttttga ttgattcggg agctcgagat ttgacttctt ttagctgatt     120
   cggcaagttt gtatctagaa aggatcgatt ggtgaggtca atagtggttg gtgggtttta     180
   gta atg gaa gac ggt gag ctt gat ttc tcc aat cag gaa gtg ttt tcg       228
       Met Glu Asp Gly Glu Leu Asp Phe Ser Asn Gln Glu Val Phe Ser
       1               5                   10                  15
   agt tcg gag atg ggt gaa tta cca cct agc aat tgt tcg atg gat agt       276
   Ser Ser Glu Met Gly Glu Leu Pro Pro Ser Asn Cys Ser Met Asp Ser
                   20                  25                  30
   ttc ttt gat ggg ctt tta atg gat act aat gct gct tgt acc cac act       324
   Phe Phe Asp Gly Leu Leu Met Asp Thr Asn Ala Ala Cys Thr His Thr
               35                  40                  45
   cac acc tgt aac ccc act gga cca gag aac act cat act cac acg tgc       372
   His Thr Cys Asn Pro Thr Gly Pro Glu Asn Thr His Thr His Thr Cys
           50                  55                  60
   ttc cat gtc cac acc aag att ctc ccg gat gag agc gat gaa aaa gtt       420
   Phe His Val His Thr Lys Ile Leu Pro Asp Glu Ser Asp Glu Lys Val
       65                  70                  75
   tct act gat gat aca gct gag tct tgt ggg aag aag ggt gaa aag aga      468
   Ser Thr Asp Asp Thr Ala Glu Ser Cys Gly Lys Lys Gly Glu Lys Arg
   80                  85                  90                  95
   cct ttg gga aac cgg gaa gcg gtt aga aag tat aga gag aag aag aag      516
   Pro Leu Gly Asn Arg Glu Ala Val Arg Lys Tyr Arg Glu Lys Lys Lys
                   100                 105                 110
   gct aaa gct gct tct ttg gag gat gag gtt gca agg ctt agg gcg gtg      564
   Ala Lys Ala Ala Ser Leu Glu Asp Glu Val Ala Arg Leu Arg Ala Val
               115                 120                 125
   aat cag cag ctg gtg aag agg ttg caa aat cag gct acc ttg gaa gct      612
   Asn Gln Gln Leu Val Lys Arg Leu Gln Asn Gln Ala Thr Leu Glu Ala
           130                 135                 140
   gag gtt tcg agg ctt aag tgt ttg ctt gtg gat ttg aga gga aga ata      660
   Glu Val Ser Arg Leu Lys Cys Leu Leu Val Asp Leu Arg Gly Arg Ile
       145                 150                 155
   gat gga gag att gga tct ttt cct tat cag aaa cct atg gct gca aat      708
   Asp Gly Glu Ile Gly Ser Phe Pro Tyr Gln Lys Pro Met Ala Ala Asn
   160                 165                 170                 175
   att cct tct ttc tcg cac atg atg aat cct tgt aat gta caa tgt gat      756
   Ile Pro Ser Phe Ser His Met Met Asn Pro Cys Asn Val Gln Cys Asp
                   180                 185                 190
   gat gaa gtt tat tgc cct cag aat gtg ttt gga gtg aat agc caa gaa      804
   Asp Glu Val Tyr Cys Pro Gln Asn Val Phe Gly Val Asn Ser Gln Glu
               195                 200                 205
   ggt gcc tcg atc aat gac caa ggg tta agt ggt tgt gat ttt gat cag      852
   Gly Ala Ser Ile Asn Asp Gln Gly Leu Ser Gly Cys Asp Phe Asp Gln
           210                 215                 220
   cta caa tgc atg gct aat cag aac tta aat gga aat gga aac gga tca      900
   Leu Gln Cys Met Ala Asn Gln Asn Leu Asn Gly Asn Gly Asn Gly Ser
       225                 230                 235
   ttc agc aac gtc aat aca tct gtc tcg aat aag aga aaa ggt ggg cat      948
   Phe Ser Asn Val Asn Thr Ser Val Ser Asn Lys Arg Lys Gly Gly His
   240                 245                 250                 255
   cgt gca tca aga gca gtt tga agcatcatca agcttgtact atctatttcc         999
   Arg Ala Ser Arg Ala Val
                   260
   accagcatag atattgtatt ccaaataagt tgtagagttc agctgcagga tcagcttcgc    1059
   tcagctttga ggggttggtg gtgtggtctt tctttgtggc acgagtgaga tctatggaca    1119
   gaacccagat ttagtagtag tagaggcagg atttcgactt ccactaacca tcatgttgct    1179
   tggtgaagaa caaggtatgc ccatgaagca cactgttttg tacattgagc ttgaggggct    1239
   gtctctgatc tagccttact gtaacattgc aacgttctca caattgtgat cccaagttgc    1299
   tttgttgact taaatgtgat aatatagctt aacttttact tgaaaaaaaa aaaaaaaaaa    1359
   aaaaaaaaaa a                                                         1370
<210> 34
   <211> 261
   <212> PRT
   <213> Arabidopsis thaliana
<400> 34
   Met Glu Asp Gly Glu Leu Asp Phe Ser Asn Gln Glu Val Phe Ser Ser
   1 5 10 15
   Ser Glu Met Gly Glu Leu Pro Pro Ser Asn Cys Ser Met Asp Ser Phe
   20 25 30
   Phe Asp Gly Leu Leu Met Asp Thr Asn Ala Ala Cys Thr His Thr His
   35 40 45
   Thr Cys Asn Pro Thr Gly Pro Glu Asn Thr His Thr His Thr Cys Phe
   50 55 60
   His Val His Thr Lys Ile Leu Pro Asp Glu Ser Asp Glu Lys Val Ser
   65 70 75 80
   Thr Asp Asp Thr Ala Glu Ser Cys Gly Lys Lys Gly Glu Lys Arg Pro
   85 90 95
   Leu Gly Asn Arg Glu Ala Val Arg Lys Tyr Arg Glu Lys Lys Lys Ala
   100 105 110
   Lys Ala Ala Ser Leu Glu Asp Glu Val Ala Arg Leu Arg Ala Val Asn
   115 120 125
   Gln Gln Leu Val Lys Arg Leu Gln Asn Gln Ala Thr Leu Glu Ala Glu
   130 135 140
   Val Ser Arg Leu Lys Cys Leu Leu Val Asp Leu Arg Gly Arg Ile Asp
   145 150 155 160
   Gly Glu Ile Gly Ser Phe Pro Tyr Gln Lys Pro Met Ala Ala Asn Ile
   165 170 175
   Pro Ser Phe Ser His Met Met Asn Pro Cys Asn Val Gln Cys Asp Asp
   180 185 190
   Glu Val Tyr Cys Pro Gln Asn Val Phe Gly Val Asn Ser Gln Glu Gly
   195 200 205
   Ala Ser Ile Asn Asp Gln Gly Leu Ser Gly Cys Asp Phe Asp Gln Leu
   210 215 220
   Gln Cys Met Ala Asn Gln Asn Leu Asn Gly Asn Gly Asn Gly Ser Phe
   225 230 235 240
   Ser Asn Val Asn Thr Ser Val Ser Asn Lys Arg Lys Gly Gly His Arg
   245 250 255
   Ala Ser Arg Ala Val
   260
<210> 35
   <211> 1638
   <212> DNA
   <213> Arabidopsis thaliana
<220>
   <221> CDS
   <222> (267)..(1259)
   <223> G558
<400> 35
   ggaatttcgg atcgtgtctc tctctgtttc tttgtttcaa tccgatttcg aatcaagccc     60
   tttacttgtg caccttcaag atttcgtttt ttccagcgcc cagaatgctc cgggtgacca    120
   acatttgttc ctgattcatt tcctattggt tcgtattgtc tgtgcacaca agagaaattt    180
   caagaagttg ttactaaaag agaggccaca agtggatatt gtctttgtta tcaagtgtta    240
   gtacagaaaa gtggtgagaa agtaat atg gct gat acc agt ccg aga act gat     293
                                Met Ala Asp Thr Ser Pro Arg Thr Asp
                                1               5
   gtc tca aca gat gac gac aca gat cat cct gat ctt ggg tcg gag gga      341
   Val Ser Thr Asp Asp Asp Thr Asp His Pro Asp Leu Gly Ser Glu Gly
   10                  15                  20                  25
   gca cta gtg aat act gct gct tct gat tcg agt gac cga tcg aag gga      389
   Ala Leu Val Asn Thr Ala Ala Ser Asp Ser Ser Asp Arg Ser Lys Gly
                   30                  35                  40
   aag atg gat caa aag act ctt cgt agg ctt gct caa aac cgt gag gca      437
   Lys Met Asp Gln Lys Thr Leu Arg Arg Leu Ala Gln Asn Arg Glu Ala
               45                  50                  55
   gca agg aaa agc aga ttg agg aag aag gct tat gtt cag cag cta gag      485
   Ala Arg Lys Ser Arg Leu Arg Lys Lys Ala Tyr Val Gln Gln Leu Glu
           60                  65                  70
   aac agc cgc ttg aaa cta acc cag ctt gag cag gag ctg caa aga gca      533
   Asn Ser Arg Leu Lys Leu Thr Gln Leu Glu Gln Glu Leu Gln Arg Ala
       75                  80                  85
   aga cag cag ggc gtc ttc att tca ggc aca gga gac cag gcc cat tct      581
   Arg Gln Gln Gly Val Phe Ile Ser Gly Thr Gly Asp Gln Ala His Ser
   90                  95                  100                 105
   act ggt gga aat ggt gct ttg gcg ttt gat gct gaa cat tca cgg tgg      629
   Thr Gly Gly Asn Gly Ala Leu Ala Phe Asp Ala Glu His Ser Arg Trp
                   110                 115                 120
   ttg gaa gaa aag aac aag caa atg aac gag ctg agg tct gct ctg aat      677
   Leu Glu Glu Lys Asn Lys Gln Met Asn Glu Leu Arg Ser Ala Leu Asn
               125                 130                 135
   gcg cat gca ggt gat tct gag ctt cga ata ata gtc gat ggt gtg atg      725
   Ala His Ala Gly Asp Ser Glu Leu Arg Ile Ile Val Asp Gly Val Met
           140                 145                 150
   gct cac tat gag gag ctt ttc agg ata aag agc aat gca gct aag aat      773
   Ala His Tyr Glu Glu Leu Phe Arg Ile Lys Ser Asn Ala Ala Lys Asn
       155                 160                 165
   gat gtc ttt cac ttg cta tct ggc atg tgg aaa aca cca gct gag aga      821
   Asp Val Phe His Leu Leu Ser Gly Met Trp Lys Thr Pro Ala Glu Arg
   170                 175                 180                 185
   tgt ttc ttg tgg ctc ggt gga ttt cgt tca tcc gaa ctt cta aag ctt      869
   Cys Phe Leu Trp Leu Gly Gly Phe Arg Ser Ser Glu Leu Leu Lys Leu
                   190                 195                 200
   ctg gcg aat cag ttg gag cca atg aca gag aga cag ttg atg ggc ata      917
   Leu Ala Asn Gln Leu Glu Pro Met Thr Glu Arg Gln Leu Met Gly Ile
               205                 210                 215
   aat aac ctg caa cag aca tcg cag cag gct gaa gat gct ttg tct caa      965
   Asn Asn Leu Gln Gln Thr Ser Gln Gln Ala Glu Asp Ala Leu Ser Gln
           220                 225                 230
   ggg atg gag agc tta caa cag tca cta gct gat act tta tcg agc ggg     1013
   Gly Met Glu Ser Leu Gln Gln Ser Leu Ala Asp Thr Leu Ser Ser Gly
       235                 240                 245
   act ctt ggt tca agt tca tca ggg aat gtc gca agc tac atg ggt cag     1061
   Thr Leu Gly Ser Ser Ser Ser Gly Asn Val Ala Ser Tyr Met Gly Gln
   250                 255                 260                 265
   atg gcc atg gca atg gga aag tta ggt aca ctc gaa gga ttt atc cgc      1109
   Met Ala Met Ala Met Gly Lys Leu Gly Thr Leu Glu Gly Phe Ile Arg
                   270                 275                 280
   cag gct gat aat ttg aga cta caa aca ttg caa cag atg ata aga gta      1157
   Gln Ala Asp Asn Leu Arg Leu Gln Thr Leu Gln Gln Met Ile Arg Val
               285                 290                 295
   tta aca acg aga cag tca gca cgt gct cta ctt gca ata cac gat tac      1205
   Leu Thr Thr Arg Gln Ser Ala Arg Ala Leu Leu Ala Ile His Asp Tyr
           300                 305                 310
   ttc tca cgg cta cga gct cta agc tcc tta tgg ctt gct cga ccc aga      1253
   Phe Ser Arg Leu Arg Ala Leu Ser Ser Leu Trp Leu Ala Arg Pro Arg
       315                 320                 325
   gag tga aactgtattt tggtcacatg tcagctgtac aaaatccata tggacacaaa      1309
   Glu
   330
   accaggagag actattaatc aacacttgtc agattcttct taccaaatcc atcaacaaat   1369
   aagcaaattt ctgggaaaca aaagactctt tgtatgtagg tttcttctac atggttgtgg   1429
   taattcatgt tgttttagtt gtagtcatca gtttttaatt tagcatttga aaagttcaat   1489
   gttgtttata tagcatcttc gattatctta gaaaggttat tgaattttgt ttttttttgt    1549
   tacttttgtg tgtggtaaag gtgttttaac cttgcaactt ctgtactgta atcatttaac   1609
   aatattaaga tgttctattt gagttttgt                                      1638
<210> 36
   <211> 330
   <212> PRT
   <213> Arabidopsis thaliana
<400> 36
   Met Ala Asp Thr Ser Pro Arg Thr Asp Val Ser Thr Asp Asp Asp Thr
   1               5                   10                  15
   Asp His Pro Asp Leu Gly Ser Glu Gly Ala Leu Val Asn Thr Ala Ala
               20                  25                  30
   Ser Asp Ser Ser Asp Arg Ser Lys Gly Lys Met Asp Gln Lys Thr Leu
           35                  40                  45
   Arg Arg Leu Ala Gln Asn Arg Glu Ala Ala Arg Lys Ser Arg Leu Arg
       50                  55                  60
   Lys Lys Ala Tyr Val Gln Gln Leu Glu Asn Ser Arg Leu Lys Leu Thr
   65                  70                  75                  80
   Gln Leu Glu Gln Glu Leu Gln Arg Ala Arg Gln Gln Gly Val Phe Ile
                   85                  90                  95
   Ser Gly Thr Gly Asp Gln Ala His Ser Thr Gly Gly Asn Gly Ala Leu
               100                 105                 110
   Ala Phe Asp Ala Glu His Ser Arg Trp Leu Glu Glu Lys Asn Lys Gln
           115                 120                 125
   Met Asn Glu Leu Arg Ser Ala Leu Asn Ala His Ala Gly Asp Ser Glu
       130                 135                 140
   Leu Arg Ile Ile Val Asp Gly Val Met Ala His Tyr Glu Glu Leu Phe
   145                 150                 155                 160
   Arg Ile Lys Ser Asn Ala Ala Lys Asn Asp Val Phe His Leu Leu Ser
                   165                 170                 175
   Gly Met Trp Lys Thr Pro Ala Glu Arg Cys Phe Leu Trp Leu Gly Gly
               180                 185                 190
   Phe Arg Ser Ser Glu Leu Leu Lys Leu Leu Ala Asn Gln Leu Glu Pro
           195                 200                 205
   Met Thr Glu Arg Gln Leu Met Gly Ile Asn Asn Leu Gln Gln Thr Ser
       210                 215                 220
   Gln Gln Ala Glu Asp Ala Leu Ser Gln Gly Met Glu Ser Leu Gln Gln
   225                 230                 235                 240
   Ser Leu Ala Asp Thr Leu Ser Ser Gly Thr Leu Gly Ser Ser Ser Ser
                   245                 250                 255
   Gly Asn Val Ala Ser Tyr Met Gly Gln Met Ala Met Ala Met Gly Lys
               260                 265                 270
   Leu Gly Thr Leu Glu Gly Phe Ile Arg Gln Ala Asp Asn Leu Arg Leu
           275                 280                 285
   Gln Thr Leu Gln Gln Met Ile Arg Val Leu Thr Thr Arg Gln Ser Ala
       290                 295                 300
   Arg Ala Leu Leu Ala Ile His Asp Tyr Phe Ser Arg Leu Arg Ala Leu
   305                 310                 315                 320
   Ser Ser Leu Trp Leu Ala Arg Pro Arg Glu
                   325                 330
<210> 37
   <211> 436
   <212> DNA
   <213> Arabidopsis thaliana
<220>
   <221> CDS
   <222> (83)..(313)
   <223> G1396
<400> 37
   tcgacctcgt ttcctttcct cctctcttcc taccattagt acgttactgg agctgatctc      60
   acgtatattt tggatcgtaa tc atg gac ggc gaa gat ttt gcc gga aag gcg      112
                            Met Asp Gly Glu Asp Phe Ala Gly Lys Ala
                            1               5                   10
   gct gct gaa gcc aag gga ttg aac ccg gga tta atc gtg ctg ctt gtt      160
   Ala Ala Glu Ala Lys Gly Leu Asn Pro Gly Leu Ile Val Leu Leu Val
                   15                  20                  25
   gtt gga ggt ccg ctt ctt gtg ttc cta atc gcc aac tac gtg ctt tac      208
   Val Gly Gly Pro Leu Leu Val Phe Leu Ile Ala Asn Tyr Val Leu Tyr
               30                  35                  40
   gtt tat gct cag aag aac cta cct cca agg aag aag aag ccc gtt tcc      256
   Val Tyr Ala Gln Lys Asn Leu Pro Pro Arg Lys Lys Lys Pro Val Ser
           45                  50                  55
   aaa aag aag ctc aag cgg gag aag cta aag caa gga gtc cct gtc cct      304
   Lys Lys Lys Leu Lys Arg Glu Lys Leu Lys Gln Gly Val Pro Val Pro
       60                  65                  70
   gga gaa taa aagccagctt aagcttcctt cacttgtgcc tccttcaaag              353
   Gly Glu
   75
   cggtttttgt tcggttacca aatttcaccc ttgcgggttt ttttcttcct ttacttctgt      413
   catgaggatt atctttgagg cct                                             436
<210> 38
   <211> 76
   <212> PRT
   <213> Arabidopsis thaliana
<400> 38
   Met Asp Gly Glu Asp Phe Ala Gly Lys Ala Ala Ala Glu Ala Lys Gly
   1               5                   10                  15
   Leu Asn Pro Gly Leu Ile Val Leu Leu Val Val Gly Gly Pro Leu Leu
               20                  25                  30
   Val Phe Leu Ile Ala Asn Tyr Val Leu Tyr Val Tyr Ala Gln Lys Asn
           35                  40                  45
   Leu Pro Pro Arg Lys Lys Lys Pro Val Ser Lys Lys Lys Leu Lys Arg
       50                  55                  60
   Glu Lys Leu Lys Gln Gly Val Pro Val Pro Gly Glu
   65                  70                  75
<210> 39
   <211> 1470
   <212> DNA
   <213> Arabidopsis thaliana
<220>
   <221> CDS
   <222> (280)..(1317)
   <223> G265
<400> 39
   ctttggtctt ggaagccaaa tcaaaccttt ccttcaatcc tcaaattttc gaaaattttc      60
   tcttttgctt tacgttctct caattcttat ttgtaagaaa gtttgttcct ttaatcaatc     120
   aaatcaaaga gacttttgaa gattgtttcc caatttgcgt caatcgggat cgagtcaaat     180
   ctgaaatctt ctccactcat catctgacta taagacttaa tcaagggact ttttgttcgg     240
   gtttggtttt aaacgtcttg gattcgaagt ggttaaggt atg gat gaa aat aat       294
                                             Met Asp Glu Asn Asn
                                             1               5
   gga ggt tca agc tca ctt cca cct ttc ctt act aaa aca tat gaa atg      342
   Gly Gly Ser Ser Ser Leu Pro Pro Phe Leu Thr Lys Thr Tyr Glu Met
                   10                  15                  20
   gtt gat gat tct tct tct gac tcg gtc gtt gct tgg agc gaa aac aac      390
   Val Asp Asp Ser Ser Ser Asp Ser Val Val Ala Trp Ser Glu Asn Asn
               25                  30                  35
   aaa agc ttc atc gtc aag aat cca gca gag ttt tca aga gac ctt ctt        438
   Lys Ser Phe Ile Val Lys Asn Pro Ala Glu Phe Ser Arg Asp Leu Leu
           40                  45                  50
   ccg aga ttc ttc aag cat aag aat ttc tca agt ttc atc cgt cag ctt        486
   Pro Arg Phe Phe Lys His Lys Asn Phe Ser Ser Phe Ile Arg Gln Leu
       55                  60                  65
   aat aca tat ggt ttt cga aaa gta gat cct gag aaa tgg gaa ttc ttg        534
   Asn Thr Tyr Gly Phe Arg Lys Val Asp Pro Glu Lys Trp Glu Phe Leu
   70                  75                  80                  85
   aat gat gat ttt gtt aga ggt cga cct tac ctt atg aag aac att cat        582
   Asn Asp Asp Phe Val Arg Gly Arg Pro Tyr Leu Met Lys Asn Ile His
                   90                  95                  100
   aga cga aaa ccg gtt cat agc cac tcg tta gtg aat cta caa gcg caa        630
   Arg Arg Lys Pro Val His Ser His Ser Leu Val Asn Leu Gln Ala Gln
               105                 110                 115
   aat cct ttg acg gaa tca gaa aga cgg agc atg gag gat cag ata gaa        678
   Asn Pro Leu Thr Glu Ser Glu Arg Arg Ser Met Glu Asp Gln Ile Glu
           120                 125                 130
   aga ctg aaa aat gag aaa gaa ggc ctt ctt gcg gag tta cag aac caa        726
   Arg Leu Lys Asn Glu Lys Glu Gly Leu Leu Ala Glu Leu Gln Asn Gln
       135                 140                 145
   gag caa gaa cgg aaa gag ttt gag ctg caa gta acg aca ttg aaa gat        774
   Glu Gln Glu Arg Lys Glu Phe Glu Leu Gln Val Thr Thr Leu Lys Asp
   150                 155                 160                 165
   cgg tta caa cat atg gaa caa cat cag aaa tca ata gtg gca tat gtt        822
   Arg Leu Gln His Met Glu Gln His Gln Lys Ser Ile Val Ala Tyr Val
                   170                 175                 180
   tca cag gtt ttg gga aaa cca gga ctt tca cta aac ctc gaa aac cat        870
   Ser Gln Val Leu Gly Lys Pro Gly Leu Ser Leu Asn Leu Glu Asn His
               185                 190                 195
   gag aga aga aaa aga aga ttt caa gag aac tct ctt cct cca agc agt        918
   Glu Arg Arg Lys Arg Arg Phe Gln Glu Asn Ser Leu Pro Pro Ser Ser
           200                 205                 210
   tca cac ata gaa cag gtc gaa aag tta gaa tct tcg cta acg ttt tgg        966
   Ser His Ile Glu Gln Val Glu Lys Leu Glu Ser Ser Leu Thr Phe Trp
       215                 220                 225
   gag aat ctt gta tcg gaa tca tgc gag aag agc ggt ttg cag tca tca       1014
   Glu Asn Leu Val Ser Glu Ser Cys Glu Lys Ser Gly Leu Gln Ser Ser
   230                 235                 240                 245
   agc atg gat cat gat gca gct gag tca agt cta agt att ggc gat aca       1062
   Ser Met Asp His Asp Ala Ala Glu Ser Ser Leu Ser Ile Gly Asp Thr
                   250                 255                 260
   cga ccc aaa tca tcg aag att gat atg aac tca gag ccg ccc gtt acc       1110
   Arg Pro Lys Ser Ser Lys Ile Asp Met Asn Ser Glu Pro Pro Val Thr
               265                 270                 275
   gtt act gcg cct gct cca aaa aca ggc gtt aac gat gac ttt tgg gaa       1158
   Val Thr Ala Pro Ala Pro Lys Thr Gly Val Asn Asp Asp Phe Trp Glu
           280                 285                 290
   caa tgt ttg aca gag aac cct gga tca acc gag caa caa gaa gtt cag       1206
   Gln Cys Leu Thr Glu Asn Pro Gly Ser Thr Glu Gln Gln Glu Val Gln
       295                 300                 305
   tca gag aga aga gat gtc ggt aat gat aat aat ggt aat aag att gga       1254
   Ser Glu Arg Arg Asp Val Gly Asn Asp Asn Asn Gly Asn Lys Ile Gly
   310                 315                 320                 325
   aat caa agg acg tat tgg tgg aat tca ggg aat gta aat aac att aca       1302
   Asn Gln Arg Thr Tyr Trp Trp Asn Ser Gly Asn Val Asn Asn Ile Thr
                   330                 335                 340
   gag aaa gct tct tga catgaatgag gtttttgtaa aatagttttc ttttggttcc     1357
   Glu Lys Ala Ser
               345
   actgagatta ttgtatgtgt tcattattta ttactctgtt tctgtaaaaa caaatctctc   1417
   tattgtttga ggcaggagtg acataaatgc atatgcagaa ttggtttcaa aaa           1470
<210> 40
   <211> 345
   <212> PRT
   <213> Arabidopsis thaliana
<400> 40
   Met Asp Glu Asn Asn Gly Gly Ser Ser Ser Leu Pro Pro Phe Leu Thr
   1               5                   10                  15
   Lys Thr Tyr Glu Met Val Asp Asp Ser Ser Ser Asp Ser Val Val Ala
               20                  25                  30
   Trp Ser Glu Asn Asn Lys Ser Phe Ile Val Lys Asn Pro Ala Glu Phe
           35                  40                  45
   Ser Arg Asp Leu Leu Pro Arg Phe Phe Lys His Lys Asn Phe Ser Ser
       50                  55                  60
   Phe Ile Arg Gln Leu Asn Thr Tyr Gly Phe Arg Lys Val Asp Pro Glu
   65                  70                  75                  80
   Lys Trp Glu Phe Leu Asn Asp Asp Phe Val Arg Gly Arg Pro Tyr Leu
                   85                  90                  95
   Met Lys Asn Ile His Arg Arg Lys Pro Val His Ser His Ser Leu Val
               100                 105                 110
   Asn Leu Gln Ala Gln Asn Pro Leu Thr Glu Ser Glu Arg Arg Ser Met
           115                 120                 125
   Glu Asp Gln Ile Glu Arg Leu Lys Asn Glu Lys Glu Gly Leu Leu Ala
       130                 135                 140
   Glu Leu Gln Asn Gln Glu Gln Glu Arg Lys Glu Phe Glu Leu Gln Val
   145                 150                 155                 160
   Thr Thr Leu Lys Asp Arg Leu Gln His Met Glu Gln His Gln Lys Ser
                   165                 170                 175
   Ile Val Ala Tyr Val Ser Gln Val Leu Gly Lys Pro Gly Leu Ser Leu
               180                 185                 190
   Asn Leu Glu Asn His Glu Arg Arg Lys Arg Arg Phe Gln Glu Asn Ser
           195                 200                 205
   Leu Pro Pro Ser Ser Ser His Ile Glu Gln Val Glu Lys Leu Glu Ser
       210                 215                 220
   Ser Leu Thr Phe Trp Glu Asn Leu Val Ser Glu Ser Cys Glu Lys Ser
   225                 230                 235                 240
   Gly Leu Gln Ser Ser Ser Met Asp His Asp Ala Ala Glu Ser Ser Leu
                   245                 250                 255
   Ser Ile Gly Asp Thr Arg Pro Lys Ser Ser Lys Ile Asp Met Asn Ser
               260                 265                 270
   Glu Pro Pro Val Thr Val Thr Ala Pro Ala Pro Lys Thr Gly Val Asn
           275                 280                 285
   Asp Asp Phe Trp Glu Gln Cys Leu Thr Glu Asn Pro Gly Ser Thr Glu
       290                 295                 300
   Gln Gln Glu Val Gln Ser Glu Arg Arg Asp Val Gly Asn Asp Asn Asn
   305                 310                 315                 320
   Gly Asn Lys Ile Gly Asn Gln Arg Thr Tyr Trp Trp Asn Ser Gly Asn
                   325                 330                 335
   Val Asn Asn Ile Thr Glu Lys Ala Ser
               340                 345
<210> 41
   <211> 913
   <212> DNA
   <213> Arabidopsis thaliana
<220>
   <221> CDS
   <222> (52)..(783)
   <223> G1006
<400> 41
   gataaatcaa tcaacaaaac aaaaaaaact ctatagttag tttctctgaa a atg tac      57
                                                            Met Tyr
                                                            1
   gga cag tgc aat ata gaa tcc gac tac gct ttg ttg gag tcg ata aca      105
   Gly Gln Cys Asn Ile Glu Ser Asp Tyr Ala Leu Leu Glu Ser Ile Thr
           5                   10                  15
   cgt cac ttg cta gga gga gga gga gag aac gag ctg cga ctc aat gag      153
   Arg His Leu Leu Gly Gly Gly Gly Glu Asn Glu Leu Arg Leu Asn Glu
       20                  25                  30
   tca aca ccg agt tcg tgt ttc aca gag agt tgg gga ggt ttg cca ttg      201
   Ser Thr Pro Ser Ser Cys Phe Thr Glu Ser Trp Gly Gly Leu Pro Leu
   35                  40                  45                  50
   aaa gag aat gat tca gag gac atg ttg gtg tac gga ctc ctc aaa gat      249
   Lys Glu Asn Asp Ser Glu Asp Met Leu Val Tyr Gly Leu Leu Lys Asp
                   55                  60                  65
   gcc ttc cat ttt gac acg tca tca tcg gac ttg agc tgt ctt ttt gat      297
   Ala Phe His Phe Asp Thr Ser Ser Ser Asp Leu Ser Cys Leu Phe Asp
               70                  75                  80
   ttt ccg gcg gtt aaa gtc gag cca act gag aac ttt acg gcg atg gag      345
   Phe Pro Ala Val Lys Val Glu Pro Thr Glu Asn Phe Thr Ala Met Glu
           85                  90                  95
   gag aaa cca aag aaa gcg ata ccg gtt acg gag acg gca gtg aag gcg      393
   Glu Lys Pro Lys Lys Ala Ile Pro Val Thr Glu Thr Ala Val Lys Ala
       100                 105                 110
   aag cat tac aga gga gtg agg cag aga ccg tgg ggg aaa ttc gcg gcg      441
   Lys His Tyr Arg Gly Val Arg Gln Arg Pro Trp Gly Lys Phe Ala Ala
   115                 120                 125                 130
   gag ata cgt gat ccg gcg aag aat gga gct agg gtt tgg tta ggg acg      489
   Glu Ile Arg Asp Pro Ala Lys Asn Gly Ala Arg Val Trp Leu Gly Thr
                   135                 140                 145
   ttt gag acg gcg gaa gat gcg gct tta gct tac gat ata gct gct ttt      537
   Phe Glu Thr Ala Glu Asp Ala Ala Leu Ala Tyr Asp Ile Ala Ala Phe
               150                 155                 160
   agg atg cgt ggt tcc cgc gct tta ttg aat ttt ccg ttg agg gtt aat      585
   Arg Met Arg Gly Ser Arg Ala Leu Leu Asn Phe Pro Leu Arg Val Asn
           165                 170                 175
   tcc ggt gaa cct gac ccg gtt cgg atc acg tct aag aga tct tct tcg      633
   Ser Gly Glu Pro Asp Pro Val Arg Ile Thr Ser Lys Arg Ser Ser Ser
       180                 185                 190
   tcg tcg tcg tcg tcg tcc tct tct acg tcg tcg tct gaa aac ggg aag      681
   Ser Ser Ser Ser Ser Ser Ser Ser Thr Ser Ser Ser Glu Asn Gly Lys
   195                 200                 205                 210
   ttg aaa cga agg aga aaa gca gag aat ctg acg tcg gag gtg gtg cag      729
   Leu Lys Arg Arg Arg Lys Ala Glu Asn Leu Thr Ser Glu Val Val Gln
                   215                 220                 225
   gtg aag tgt gag gtt ggt gat gag aca cgt gtt gat gag tta ttg gtt      777
   Val Lys Cys Glu Val Gly Asp Glu Thr Arg Val Asp Glu Leu Leu Val
               230                 235                 240
   tca taa gtttgatctt gtgtgttttg tagttgaata gttttgctat aaatgttgag      833
   Ser
   gcaccaagta aaagtgttcc cgtgatgtaa attagttact aaacagagcc atatatcttc    893
   aatcaaaaaa aaaaaaaaaa                                                913
<210> 42
   <211> 243
   <212> PRT
   <213> Arabidopsis thaliana
<400> 42
   Met Tyr Gly Gln Cys Asn Ile Glu Ser Asp Tyr Ala Leu Leu Glu Ser
   1               5                   10                  15
   Ile Thr Arg His Leu Leu Gly Gly Gly Gly Glu Asn Glu Leu Arg Leu
               20                  25                  30
   Asn Glu Ser Thr Pro Ser Ser Cys Phe Thr Glu Ser Trp Gly Gly Leu
           35                  40                  45
   Pro Leu Lys Glu Asn Asp Ser Glu Asp Met Leu Val Tyr Gly Leu Leu
       50                  55                  60
   Lys Asp Ala Phe His Phe Asp Thr Ser Ser Ser Asp Leu Ser Cys Leu
   65                  70                  75                  80
   Phe Asp Phe Pro Ala Val Lys Val Glu Pro Thr Glu Asn Phe Thr Ala
                   85                  90                  95
   Met Glu Glu Lys Pro Lys Lys Ala Ile Pro Val Thr Glu Thr Ala Val
               100                 105                 110
   Lys Ala Lys His Tyr Arg Gly Val Arg Gln Arg Pro Trp Gly Lys Phe
           115                 120                 125
   Ala Ala Glu Ile Arg Asp Pro Ala Lys Asn Gly Ala Arg Val Trp Leu
       130                 135                 140
   Gly Thr Phe Glu Thr Ala Glu Asp Ala Ala Leu Ala Tyr Asp Ile Ala
   145                 150                 155                 160
   Ala Phe Arg Met Arg Gly Ser Arg Ala Leu Leu Asn Phe Pro Leu Arg
                   165                 170                 175
   Val Asn Ser Gly Glu Pro Asp Pro Val Arg Ile Thr Ser Lys Arg Ser
               180                 185                 190
   Ser Ser Ser Ser Ser Ser Ser Ser Ser Ser Thr Ser Ser Ser Glu Asn
           195                 200                 205
   Gly Lys Leu Lys Arg Arg Arg Lys Ala Glu Asn Leu Thr Ser Glu Val
       210                 215                 220
   Val Gln Val Lys Cys Glu Val Gly Asp Glu Thr Arg Val Asp Glu Leu
   225                 230                 235                 240
   Leu Val Ser
<210> 43
   <211> 912
   <212> DNA
   <213> Arabidopsis thaliana
<220>
   <221> CDS
   <222> (53)..(859)
   <223> G1309
<400> 43
   cgtcgacctc ttaattaaga cgacttgaga gagaaagaaa gatacgtgga ag atg acc      58
                                                             Met Thr
                                                             1
   aaa tct gga gag aga cca aaa cag aga cag agg aaa ggg tta tgg tca      106
   Lys Ser Gly Glu Arg Pro Lys Gln Arg Gln Arg Lys Gly Leu Trp Ser
           5                   10                  15
   cct gaa gaa gac cag aag ctc aag agt ttc atc ctc tct cgt ggc cat      154
   Pro Glu Glu Asp Gln Lys Leu Lys Ser Phe Ile Leu Ser Arg Gly His
       20                  25                  30
   gct tgc tgg acc act gtt ccc atc cta gct gga ttg caa agg aat ggg      202
   Ala Cys Trp Thr Thr Val Pro Ile Leu Ala Gly Leu Gln Arg Asn Gly
   35                  40                  45                  50
   aaa agc tgc aga tta agg tgg att aat tac cta aga cca gga cta aag      250
   Lys Ser Cys Arg Leu Arg Trp Ile Asn Tyr Leu Arg Pro Gly Leu Lys
                   55                  60                  65
   agg ggg tcg ttt agt gaa gaa gaa gaa gag acc atc ttg act tta cat      298
   Arg Gly Ser Phe Ser Glu Glu Glu Glu Glu Thr Ile Leu Thr Leu His
               70                  75                  80
   tct tcc ttg ggt aac aag tgg tct cgg att gca aaa tat tta ccg gga      346
   Ser Ser Leu Gly Asn Lys Trp Ser Arg Ile Ala Lys Tyr Leu Pro Gly
           85                  90                  95
   aga aca gac aac gag att aag aac tat tgg cat tcc tat ctg aag aag      394
   Arg Thr Asp Asn Glu Ile Lys Asn Tyr Trp His Ser Tyr Leu Lys Lys
       100                 105                 110
   aga tgg ctc aaa tct caa cca caa ctc aaa agc caa ata tca gac ctc      442
   Arg Trp Leu Lys Ser Gln Pro Gln Leu Lys Ser Gln Ile Ser Asp Leu
   115                 120                 125                 130
   aca gaa tct cct tct tca cta ctt tct tgc ggg aaa aga aat ctg gaa      490
   Thr Glu Ser Pro Ser Ser Leu Leu Ser Cys Gly Lys Arg Asn Leu Glu
                   135                 140                 145
   acc gaa acc cta gat cac gtg atc tcc ttc cag aaa ttt tca gag aat      538
   Thr Glu Thr Leu Asp His Val Ile Ser Phe Gln Lys Phe Ser Glu Asn
               150                 155                 160
   cca act tca tca cca tcc aaa gaa agc aac aac aac atg atc atg aac      586
   Pro Thr Ser Ser Pro Ser Lys Glu Ser Asn Asn Asn Met Ile Met Asn
           165                 170                 175
   aac agt aat aac ttg cct aaa ctg ttc ttc tct gag tgg atc agt tct      634
   Asn Ser Asn Asn Leu Pro Lys Leu Phe Phe Ser Glu Trp Ile Ser Ser
       180                 185                 190
   tca aat cca cac atc gat tac tcc tct gct ttt aca gat tcc aag cac      682
   Ser Asn Pro His Ile Asp Tyr Ser Ser Ala Phe Thr Asp Ser Lys His
   195                 200                 205                 210
   att aat gaa act caa gat caa atc aat gaa gag gaa gtg atg atg atc      730
   Ile Asn Glu Thr Gln Asp Gln Ile Asn Glu Glu Glu Val Met Met Ile
                   215                 220                 225
   aat aac aac aac tac tct tca ctt gag gat gtc atg ctc cgt aca gat      778
   Asn Asn Asn Asn Tyr Ser Ser Leu Glu Asp Val Met Leu Arg Thr Asp
               230                 235                 240
   ttt ttg cag cct gat cat gaa tat gca aat tat tat tct tct gga gat      826
   Phe Leu Gln Pro Asp His Glu Tyr Ala Asn Tyr Tyr Ser Ser Gly Asp
           245                 250                 255
   ttc ttc atc aac agt gac caa aat tat gtc taa gaagagtgaa tatgatcgta    879
   Phe Phe Ile Asn Ser Asp Gln Asn Tyr Val
       260                 265
   agaggaacat aagctagtta cttgtgttac agc                                  912
<210> 44
   <211> 268
   <212> PRT
   <213> Arabidopsis thaliana
<400> 44
   Met Thr Lys Ser Gly Glu Arg Pro Lys Gln Arg Gln Arg Lys Gly Leu
   1               5                   10                  15
   Trp Ser Pro Glu Glu Asp Gln Lys Leu Lys Ser Phe Ile Leu Ser Arg
               20                  25                  30
   Gly His Ala Cys Trp Thr Thr Val Pro Ile Leu Ala Gly Leu Gln Arg
           35                  40                  45
   Asn Gly Lys Ser Cys Arg Leu Arg Trp Ile Asn Tyr Leu Arg Pro Gly
       50                  55                  60
   Leu Lys Arg Gly Ser Phe Ser Glu Glu Glu Glu Glu Thr Ile Leu Thr
   65                  70                  75                  80
   Leu His Ser Ser Leu Gly Asn Lys Trp Ser Arg Ile Ala Lys Tyr Leu
                   85                  90                  95
   Pro Gly Arg Thr Asp Asn Glu Ile Lys Asn Tyr Trp His Ser Tyr Leu
               100                 105                 110
   Lys Lys Arg Trp Leu Lys Ser Gln Pro Gln Leu Lys Ser Gln Ile Ser
           115                 120                 125
   Asp Leu Thr Glu Ser Pro Ser Ser Leu Leu Ser Cys Gly Lys Arg Asn
       130                 135                 140
   Leu Glu Thr Glu Thr Leu Asp His Val Ile Ser Phe Gln Lys Phe Ser
   145                 150                 155                 160
   Glu Asn Pro Thr Ser Ser Pro Ser Lys Glu Ser Asn Asn Asn Met Ile
                   165                 170                 175
   Met Asn Asn Ser Asn Asn Leu Pro Lys Leu Phe Phe Ser Glu Trp Ile
               180                 185                 190
   Ser Ser Ser Asn Pro His Ile Asp Tyr Ser Ser Ala Phe Thr Asp Ser
           195                 200                 205
   Lys His Ile Asn Glu Thr Gln Asp Gln Ile Asn Glu Glu Glu Val Met
       210                 215                 220
   Met Ile Asn Asn Asn Asn Tyr Ser Ser Leu Glu Asp Val Met Leu Arg
   225                 230                 235                 240
   Thr Asp Phe Leu Gln Pro Asp His Glu Tyr Ala Asn Tyr Tyr Ser Ser
                   245                 250                 255
   Gly Asp Phe Phe Ile Asn Ser Asp Gln Asn Tyr Val
               260                 265
<210> 45
   <211> 1575
   <212> DNA
   <213> Arabidopsis thaliana
<220>
   <221> CDS
   <222> (1)..(1575)
   <223> G2550
<400> 45
   atg gct gtg tat tac cct aat agt gtc ggc atg caa tct ctt tac caa      48
   Met Ala Val Tyr Tyr Pro Asn Ser Val Gly Met Gln Ser Leu Tyr Gln
   1               5                   10                  15
   gaa tcc att tac ctc aac gaa caa caa caa caa caa caa caa gct tct      96
   Glu Ser Ile Tyr Leu Asn Glu Gln Gln Gln Gln Gln Gln Gln Ala Ser
               20                  25                  30
   tct tcc tct gct gca tct ttc tcc gag att gtt tcc ggt gat gtt cga      144
   Ser Ser Ser Ala Ala Ser Phe Ser Glu Ile Val Ser Gly Asp Val Arg
           35                  40                  45
   aac aac gag atg gta ttt atc cca cca aca agc gac gta gcc gtc aac      192
   Asn Asn Glu Met Val Phe Ile Pro Pro Thr Ser Asp Val Ala Val Asn
        50                  55                  60
   gga aac gta acg gtg tca agt aac gat cta agc ttt cac ggt gga gga      240
   Gly Asn Val Thr Val Ser Ser Asn Asp Leu Ser Phe His Gly Gly Gly
   65                  70                  75                  80
   ctt tct tta agt ctt ggt aat cag atc cag tca gct gtc tct gtt tct      288
   Leu Ser Leu Ser Leu Gly Asn Gln Ile Gln Ser Ala Val Ser Val Ser
                   85                  90                  95
   ccg ttt cag tat cat tac cag aat ctt tcg aac caa ttg agt tac aat      336
   Pro Phe Gln Tyr His Tyr Gln Asn Leu Ser Asn Gln Leu Ser Tyr Asn
               100                 105                 110
   aat ctt aat cct tct act atg tct gat gag aat ggg aag agc ttg agt      384
   Asn Leu Asn Pro Ser Thr Met Ser Asp Glu Asn Gly Lys Ser Leu Ser
           115                 120                 125
   gtt cat cag cat cac tct gat caa att tta cct tcc tct gtt tac aac      432
   Val His Gln His His Ser Asp Gln Ile Leu Pro Ser Ser Val Tyr Asn
       130                 135                 140
   aac aat ggt aat aat ggt gtt gga ttc tac aac aat tac cgt tac gag      480
   Asn Asn Gly Asn Asn Gly Val Gly Phe Tyr Asn Asn Tyr Arg Tyr Glu
   145                 150                 155                 160
   aca tca ggg ttt gtg agt agt gta ctg aga tct cgt tac ctt aaa cca      528
   Thr Ser Gly Phe Val Ser Ser Val Leu Arg Ser Arg Tyr Leu Lys Pro
                   165                 170                 175
   aca caa caa ttg ctt gat gaa gtt gtt agt gta agg aaa gat ttg aaa      576
   Thr Gln Gln Leu Leu Asp Glu Val Val Ser Val Arg Lys Asp Leu Lys
               180                 185                 190
   ttg ggg aat aag aag atg aag aat gat aaa ggt caa gac ttt cac aat      624
   Leu Gly Asn Lys Lys Met Lys Asn Asp Lys Gly Gln Asp Phe His Asn
           195                 200                 205
   ggg tct agt gat aac att aca gaa gat gat aaa tct caa tcg cag gag      672
   Gly Ser Ser Asp Asn Ile Thr Glu Asp Asp Lys Ser Gln Ser Gln Glu
       210                 215                 220
   ttg tct cct tca gaa cgt cag gag cta cag agc aag aag agc aag ctt      720
   Leu Ser Pro Ser Glu Arg Gln Glu Leu Gln Ser Lys Lys Ser Lys Leu
   225                 230                 235                 240
   tta aca atg gtg gat gag gta gat aaa agg tat aac caa tac cat cat      768
   Leu Thr Met Val Asp Glu Val Asp Lys Arg Tyr Asn Gln Tyr His His
                   245                 250                 255
   caa atg gaa gct tta gca tcg tct ttc gag atg gta aca ggt ctt gga      816
   Gln Met Glu Ala Leu Ala Ser Ser Phe Glu Met Val Thr Gly Leu Gly
               260                 265                 270
   gca gct aag cct tac aca tcc gta gct ctg aat aga atc tct cgc cat      864
   Ala Ala Lys Pro Tyr Thr Ser Val Ala Leu Asn Arg Ile Ser Arg His
           275                 280                 285
   ttc cgc tgt tta cgc gac gcg ata aaa gaa cag att cag gtg atc aga      912
   Phe Arg Cys Leu Arg Asp Ala Ile Lys Glu Gln Ile Gln Val Ile Arg
       290                 295                 300
   ggg aag ctt ggg gag aga gag act tct gat gaa caa gga gag agg ata      960
   Gly Lys Leu Gly Glu Arg Glu Thr Ser Asp Glu Gln Gly Glu Arg Ile
   305                 310                 315                 320
   ccg cgt ctt agg tac tta gat caa cgg ttg aga caa cag aga gct ttg     1008
   Pro Arg Leu Arg Tyr Leu Asp Gln Arg Leu Arg Gln Gln Arg Ala Leu
                   325                 330                 335
   cat caa caa ctt gga atg gtt aga cca gct tgg aga cca caa aga ggc     1056
   His Gln Gln Leu Gly Met Val Arg Pro Ala Trp Arg Pro Gln Arg Gly
               340                 345                 350
   tta cct gaa aac tct gtc tct ata ctt cga gct tgg ctc ttt gag cat      1104
   Leu Pro Glu Asn Ser Val Ser Ile Leu Arg Ala Trp Leu Phe Glu His
           355                     360                     365
   ttc ctt cat cca tat cct aaa gaa tca gag aaa atc atg ctt tca aag      1152
   Phe Leu His Pro Tyr Pro Lys Glu Ser Glu Lys Ile Met Leu Ser Lys
       370                     375                     380
   cag aca gga cta tcg aaa aac cag gtt gca aat tgg ttt att aac gcg      1200
   Gln Thr Gly Leu Ser Lys Asn Gln Val Ala Asn Trp Phe Ile Asn Ala
   385                     390                     395                 400
   aga gtt cga cta tgg aaa cca atg att gaa gag atg tat aaa gaa gag      1248
   Arg Val Arg Leu Trp Lys Pro Met Ile Glu Glu Met Tyr Lys Glu Glu
                   405                     410                     415
   ttt gga gaa tca gca gag tta ctc tct aac tct aat caa gac acc aaa      1296
   Phe Gly Glu Ser Ala Glu Leu Leu Ser Asn Ser Asn Gln Asp Thr Lys
               420                     425                     430
   aaa atg cag gaa aca tct cag ctc aaa cac gaa gac tct tcg tct tcg      1344
   Lys Met Gln Glu Thr Ser Gln Leu Lys His Glu Asp Ser Ser Ser Ser
           435                     440                     445
   caa caa cag aat cag gga aac aac aac aac aac atc cca tat aca tct      1392
   Gln Gln Gln Asn Gln Gly Asn Asn Asn Asn Asn Ile Pro Tyr Thr Ser
       450                     455                     460
   gat gca gaa caa aac cta gtc ttt gca gat cct aaa cca gac cgt gct      1440
   Asp Ala Glu Gln Asn Leu Val Phe Ala Asp Pro Lys Pro Asp Arg Ala
   465                     470                     475                 480
   act act gga gat tac gac agc ttg atg aac tat cat ggg ttt ggt att      1488
   Thr Thr Gly Asp Tyr Asp Ser Leu Met Asn Tyr His Gly Phe Gly Ile
                   485                     490                     495
   gat gat tac aat cgt tac gtt ggc ctt gga aac caa caa gat ggc aga      1536
   Asp Asp Tyr Asn Arg Tyr Val Gly Leu Gly Asn Gln Gln Asp Gly Arg
               500                     505                     510
   tat tct aat ccc cat caa tta cac gac ttt gtt gtc taa                  1575
   Tyr Ser Asn Pro His Gln Leu His Asp Phe Val Val
           515                     520
<210> 46
   <211> 524
   <212> PRT
   <213> Arabidopsis thaliana
<400> 46
   Met Ala Val Tyr Tyr Pro Asn Ser Val Gly Met Gln Ser Leu Tyr Gln
   1               5                   10                  15
   Glu Ser Ile Tyr Leu Asn Glu Gln Gln Gln Gln Gln Gln Gln Ala Ser
               20                  25                  30
   Ser Ser Ser Ala Ala Ser Phe Ser Glu Ile Val Ser Gly Asp Val Arg
           35                  40                  45
   Asn Asn Glu Met Val Phe Ile Pro Pro Thr Ser Asp Val Ala Val Asn
       50                  55                  60
   Gly Asn Val Thr Val Ser Ser Asn Asp Leu Ser Phe His Gly Gly Gly
   65                  70                  75                  80
   Leu Ser Leu Ser Leu Gly Asn Gln Ile Gln Ser Ala Val Ser Val Ser
                   85                  90                  95
   Pro Phe Gln Tyr His Tyr Gln Asn Leu Ser Asn Gln Leu Ser Tyr Asn
               100                 105                 110
   Asn Leu Asn Pro Ser Thr Met Ser Asp Glu Asn Gly Lys Ser Leu Ser
           115                 120                 125
   Val His Gln His His Ser Asp Gln Ile Leu Pro Ser Ser Val Tyr Asn
       130                 135                 140
   Asn Asn Gly Asn Asn Gly Val Gly Phe Tyr Asn Asn Tyr Arg Tyr Glu
   145                 150                 155                 160
   Thr Ser Gly Phe Val Ser Ser Val Leu Arg Ser Arg Tyr Leu Lys Pro
                   165                 170                 175
   Thr Gln Gln Leu Leu Asp Glu Val Val Ser Val Arg Lys Asp Leu Lys
               180                 185                 190
   Leu Gly Asn Lys Lys Met Lys Asn Asp Lys Gly Gln Asp Phe His Asn
           195                 200                 205
   Gly Ser Ser Asp Asn Ile Thr Glu Asp Asp Lys Ser Gln Ser Gln Glu
       210                 215                 220
   Leu Ser Pro Ser Glu Arg Gln Glu Leu Gln Ser Lys Lys Ser Lys Leu
   225                 230                 235                 240
   Leu Thr Met Val Asp Glu Val Asp Lys Arg Tyr Asn Gln Tyr His His
                   245                 250                 255
   Gln Met Glu Ala Leu Ala Ser Ser Phe Glu Met Val Thr Gly Leu Gly
               260                 265                 270
   Ala Ala Lys Pro Tyr Thr Ser Val Ala Leu Asn Arg Ile Ser Arg His
           275                 280                 285
   Phe Arg Cys Leu Arg Asp Ala Ile Lys Glu Gln Ile Gln Val Ile Arg
       290                 295                 300
   Gly Lys Leu Gly Glu Arg Glu Thr Ser Asp Glu Gln Gly Glu Arg Ile
   305                 310                 315                 320
   Pro Arg Leu Arg Tyr Leu Asp Gln Arg Leu Arg Gln Gln Arg Ala Leu
                   325                 330                 335
   His Gln Gln Leu Gly Met Val Arg Pro Ala Trp Arg Pro Gln Arg Gly
               340                 345                 350
   Leu Pro Glu Asn Ser Val Ser Ile Leu Arg Ala Trp Leu Phe Glu His
           355                 360                 365
   Phe Leu His Pro Tyr Pro Lys Glu Ser Glu Lys Ile Met Leu Ser Lys
       370                 375                 380
   Gln Thr Gly Leu Ser Lys Asn Gln Val Ala Asn Trp Phe Ile Asn Ala
   385                 390                 395                 400
   Arg Val Arg Leu Trp Lys Pro Met Ile Glu Glu Met Tyr Lys Glu Glu
                   405                 410                 415
   Phe Gly Glu Ser Ala Glu Leu Leu Ser Asn Ser Asn Gln Asp Thr Lys
               420                 425                 430
   Lys Met Gln Glu Thr Ser Gln Leu Lys His Glu Asp Ser Ser Ser Ser
           435                 440                 445
   Gln Gln Gln Asn Gln Gly Asn Asn Asn Asn Asn Ile Pro Tyr Thr Ser
       450                 455                 460
   Asp Ala Glu Gln Asn Leu Val Phe Ala Asp Pro Lys Pro Asp Arg Ala
   465                 470                 475                 480
   Thr Thr Gly Asp Tyr Asp Ser Leu Met Asn Tyr His Gly Phe Gly Ile
                   485                 490                 495
   Asp Asp Tyr Asn Arg Tyr Val Gly Leu Gly Asn Gln Gln Asp Gly Arg
               500                 505                 510
   Tyr Ser Asn Pro His Gln Leu His Asp Phe Val Val
           515                 520
<210> 47
   <211> 1983
   <212> DNA
   <213> Arabidopsis thaliana
<220>
   <221> CDS
   <222> (73)..(1956)
   <223> G965
<400> 47
   gattctctgt gtatgtctga atccttacag gatccaagag ctttggaaaa aagatataat      60
   gaataacaag at atg ggt tta gct act aca act tct tct atg tca caa gat     111
                 Met Gly Leu Ala Thr Thr Thr Ser Ser Met Ser Gln Asp
                 1               5                   10
   tat cat cat cac caa gga atc ttt tcc ttc tct aat gga ttc cac cga      159
   Tyr His His His Gln Gly Ile Phe Ser Phe Ser Asn Gly Phe His Arg
       15                  20                  25
   tca tca tca acc act cat cag gag gaa gta gat gaa tcc gcc gtc gtc      207
   Ser Ser Ser Thr Thr His Gln Glu Glu Val Asp Glu Ser Ala Val Val
   30                  35                  40                  45
   tcc ggt gct caa att ccg gtt tat gaa acc gcc gga atg ttg tct gaa      255
   Ser Gly Ala Gln Ile Pro Val Tyr Glu Thr Ala Gly Met Leu Ser Glu
                   50                  55                  60
   atg ttt gct tac cct ggc gga ggt ggc ggc ggt tcc ggt gga gag att      303
   Met Phe Ala Tyr Pro Gly Gly Gly Gly Gly Gly Ser Gly Gly Glu Ile
               65                  70                  75
   ctt gat cag tct act aaa cag ttg cta gag caa caa aac cgt cac aac      351
   Leu Asp Gln Ser Thr Lys Gln Leu Leu Glu Gln Gln Asn Arg His Asn
           80                  85                  90
   aac aac aat aac tca act ctt cat atg tta tta cca aat cat cat caa      399
   Asn Asn Asn Asn Ser Thr Leu His Met Leu Leu Pro Asn His His Gln
       95                  100                 105
   ggt ttt gct ttc acc gac gaa aac act atg cag ccg cag caa caa caa        447
   Gly Phe Ala Phe Thr Asp Glu Asn Thr Met Gln Pro Gln Gln Gln Gln
   110                 115                 120                 125
   cac ttt aca tgg cca tct tcc tcc tcc gat cat cat caa aac cga gat        495
   His Phe Thr Trp Pro Ser Ser Ser Ser Asp His His Gln Asn Arg Asp
                   130                 135                 140
   atg atc gga acc gtc cac gtg gaa gga gga aag ggt ttg tct tta tct        543
   Met Ile Gly Thr Val His Val Glu Gly Gly Lys Gly Leu Ser Leu Ser
               145                 150                 155
   ctc tca tct tca tta gcc gca gct aaa gcc gag gaa tat aga agc att        591
   Leu Ser Ser Ser Leu Ala Ala Ala Lys Ala Glu Glu Tyr Arg Ser Ile
           160                 165                 170
   tat tgt gca gcc gtt gat gga act tct tct tct tct aac gca tcc gct        639
   Tyr Cys Ala Ala Val Asp Gly Thr Ser Ser Ser Ser Asn Ala Ser Ala
       175                 180                 185
   cat cat cat caa ttc aat cag ttc aag aat ctt ctt ctt gag aat tct        687
   His His His Gln Phe Asn Gln Phe Lys Asn Leu Leu Leu Glu Asn Ser
   190                 195                 200                 205
   tct tct caa cat cat cac cat caa gtt gtt gga cat ttt ggt tca tca        735
   Ser Ser Gln His His His His Gln Val Val Gly His Phe Gly Ser Ser
                   210                 215                 220
   tca tca tct ccc atg gcg gct tct tca tcc att gga ggg atc tac acg        783
   Ser Ser Ser Pro Met Ala Ala Ser Ser Ser Ile Gly Gly Ile Tyr Thr
               225                 230                 235
   ttg agg aat tcg aaa tat acg aaa ccg gct caa gag ttg ttg gaa gag        831
   Leu Arg Asn Ser Lys Tyr Thr Lys Pro Ala Gln Glu Leu Leu Glu Glu
           240                 245                 250
   ttt tgt agt gtt gga aga gga cat ttc aag aag aac aaa ctt agt agg        879
   Phe Cys Ser Val Gly Arg Gly His Phe Lys Lys Asn Lys Leu Ser Arg
       255                 260                 265
   aac aac tca aac cct aat act acc ggt gga gga gga ggc gga ggg tcc        927
   Asn Asn Ser Asn Pro Asn Thr Thr Gly Gly Gly Gly Gly Gly Gly Ser
   270                 275                 280                 285
   tcg tca tcg gcc gga aca gct aat gat agt cct cct ttg tct ccg gct        975
   Ser Ser Ser Ala Gly Thr Ala Asn Asp Ser Pro Pro Leu Ser Pro Ala
                   290                 295                 300
   gat cgg att gaa cat caa aga aga aaa gtc aag cta cta tct atg ctt       1023
   Asp Arg Ile Glu His Gln Arg Arg Lys Val Lys Leu Leu Ser Met Leu
               305                 310                 315
   gaa gag gtg gac cga cgg tac aac cac tac tgc gaa caa atg caa atg       1071
   Glu Glu Val Asp Arg Arg Tyr Asn His Tyr Cys Glu Gln Met Gln Met
           320                 325                 330
   gta gtg aac tca ttc gac caa gta atg ggt tac ggc gcg gcg gtt ccg       1119
   Val Val Asn Ser Phe Asp Gln Val Met Gly Tyr Gly Ala Ala Val Pro
       335                 340                 345
   tac acg aca tta gct caa aag gca atg tct agg cat ttc cgg tgt ttg       1167
   Tyr Thr Thr Leu Ala Gln Lys Ala Met Ser Arg His Phe Arg Cys Leu
   350                 355                 360                 365
   aaa gac gcg gta gcg gtt cag ctt aaa cgc agc tgt gag ctt cta ggg       1215
   Lys Asp Ala Val Ala Val Gln Leu Lys Arg Ser Cys Glu Leu Leu Gly
                   370                 375                 380
   gat aaa gag gcg gca ggg gct gca tcc tcg ggg tta acc aaa ggg gaa       1263
   Asp Lys Glu Ala Ala Gly Ala Ala Ser Ser Gly Leu Thr Lys Gly Glu
               385                 390                 395
   acg ccg cga ttg cgt ttg cta gag cag agt ttg cgt cag caa cga gcg       1311
   Thr Pro Arg Leu Arg Leu Leu Glu Gln Ser Leu Arg Gln Gln Arg Ala
               400                 405                 410
   tttcat cat atg ggt atg atg gag caa gag gca tgg aga ccg caa cgt      1359
   Phe His His Met Gly Met Met Glu Gln Glu Ala Trp Arg Pro Gln Arg
       415                 420                 425
   ggt ttg cct gaa cgc tcc gtt aat atc ctt aga gct tgg cta ttc gag      1407
   Gly Leu Pro Glu Arg Ser Val Asn Ile Leu Arg Ala Trp Leu Phe Glu
   430                 435                 440                 445
   cat ttt ctt aat ccg tac cca agc gat gct gat aag cac ctc tta gca      1455
   His Phe Leu Asn Pro Tyr Pro Ser Asp Ala Asp Lys His Leu Leu Ala
                   450                 455                 460
   cga cag act ggt tta tcc aga aat cag gtg tca aat tgg ttc ata aat      1503
   Arg Gln Thr Gly Leu Ser Arg Asn Gln Val Ser Asn Trp Phe Ile Asn
               465                 470                 475
   gct agg gtt cgc cta tgg aaa cca atg gtg gaa gag atg tat caa caa      1551
   Ala Arg Val Arg Leu Trp Lys Pro Met Val Glu Glu Met Tyr Gln Gln
           480                 485                 490
   gaa gca aaa gaa aga gaa gaa gca gaa gaa gaa aat gaa aat caa caa      1599
   Glu Ala Lys Glu Arg Glu Glu Ala Glu Glu Glu Asn Glu Asn Gln Gln
       495                 500                 505
   caa caa aga aga cag caa caa aca aac aac aac gac acg aaa ccc aac      1647
   Gln Gln Arg Arg Gln Gln Gln Thr Asn Asn Asn Asp Thr Lys Pro Asn
   510                 515                 520                 525
   aac aat gaa aac aac ttc act gtc ata acc gca caa act cca acg acg      1695
   Asn Asn Glu Asn Asn Phe Thr Val Ile Thr Ala Gln Thr Pro Thr Thr
                   530                 535                 540
   atg aca tcg aca cat cac gaa aac gac tct tca ttc ctc tct tcc gtc      1743
   Met Thr Ser Thr His His Glu Asn Asp Ser Ser Phe Leu Ser Ser Val
               545                 550                 555
   gcc gcc gct tct cac ggc ggt tca gac gcg ttc acc gtc gcc acg tgt      1791
   Ala Ala Ala Ser His Gly Gly Ser Asp Ala Phe Thr Val Ala Thr Cys
           560                 565                 570
   cag caa gac gtc agt gac ttc cac gtc gac gga gat ggt gtg aac gtc      1839
   Gln Gln Asp Val Ser Asp Phe His Val Asp Gly Asp Gly Val Asn Val
       575                 580                 585
   ata aga ttc ggg acc aaa cag act ggt gac gtg tct ctt acg ctt ggt      1887
   Ile Arg Phe Gly Thr Lys Gln Thr Gly Asp Val Ser Leu Thr Leu Gly
   590                 595                 600                 605
   cta cgc cac tct ggc aat att cct gat aag aac act tct ttc tcc gtt      1935
   Leu Arg His Ser Gly Asn Ile Pro Asp Lys Asn Thr Ser Phe Ser Val
                   610                 615                 620
   aga gac ttt gga gat ttt tag tcttctttgt ttctcaattt attcatc          1983
   Arg Asp Phe Gly Asp Phe
               625
<210> 48
   <211> 627
   <212> PRT
   <213> Arabidopsis thaliana
<400> 48
   Met Gly Leu Ala Thr Thr Thr Ser Ser Met Ser Gln Asp Tyr His His
   1               5                   10                  15
   His Gln Gly Ile Phe Ser Phe Ser Asn Gly Phe His Arg Ser Ser Ser
               20                  25                  30
   Thr Thr His Gln Glu Glu Val Asp Glu Ser Ala Val Val Ser Gly Ala
   35 40 45
   Gln Ile Pro Val Tyr Glu Thr Ala Gly Met Leu Ser Glu Met Phe Ala
   50 55 60
   Tyr Pro Gly Gly Gly Gly Gly Gly Ser Gly Gly Glu Ile Leu Asp Gln
   65 70 75 80
   Ser Thr Lys Gln Leu Leu Glu Gln Gln Asn Arg His Asn Asn Asn Asn
   85 90 95
   Asn Ser Thr Leu His Met Leu Leu Pro Asn His His Gln Gly Phe Ala
   100 105 110
   Phe Thr Asp Glu Asn Thr Met Gln Pro Gln Gln Gln Gln His Phe Thr
   115 120 125
   Trp Pro Ser Ser Ser Ser Asp His His Gln Asn Arg Asp Met Ile Gly
   130 135 140
   Thr Val His Val Glu Gly Gly Lys Gly Leu Ser Leu Ser Leu Ser Ser
   145 150 155 160
   Ser Leu Ala Ala Ala Lys Ala Glu Glu Tyr Arg Ser Ile Tyr Cys Ala
   165 170 175
   Ala Val Asp Gly Thr Ser Ser Ser Ser Asn Ala Ser Ala His His His
   180 185 190
   Gln Phe Asn Gln Phe Lys Asn Leu Leu Leu Glu Asn Ser Ser Ser Gln
   195 200 205
   His His His His Gln Val Val Gly His Phe Gly Ser Ser Ser Ser Ser
   210 215 220
   Pro Met Ala Ala Ser Ser Ser Ile Gly Gly Ile Tyr Thr Leu Arg Asn
   225 230 235 240
   Ser Lys Tyr Thr Lys Pro Ala Gln Glu Leu Leu Glu Glu Phe Cys Ser
   245 250 255
   Val Gly Arg Gly His Phe Lys Lys Asn Lys Leu Ser Arg Asn Asn Ser
   260 265 270
   Asn Pro Asn Thr Thr Gly Gly Gly Gly Gly Gly Gly Ser Ser Ser Ser
   275 280 285
   Ala Gly Thr Ala Asn Asp Ser Pro Pro Leu Ser Pro Ala Asp Arg Ile
   290 295 300
   Glu His Gln Arg Arg Lys Val Lys Leu Leu Ser Met Leu Glu Glu Val
   305 310 315 320
   Asp Arg Arg Tyr Asn His Tyr Cys Glu Gln Met Gln Met Val Val Asn
   325 330 335
   Ser Phe Asp Gln Val Met Gly Tyr Gly Ala Ala Val Pro Tyr Thr Thr
               340                 345                 350
   Leu Ala Gln Lys Ala Met Ser Arg His Phe Arg Cys Leu Lys Asp Ala
           355                 360                 365
   Val Ala Val Gln Leu Lys Arg Ser Cys Glu Leu Leu Gly Asp Lys Glu
       370                 375                 380
   Ala Ala Gly Ala Ala Ser Ser Gly Leu Thr Lys Gly Glu Thr Pro Arg
   385                 390                 395                 400
   Leu Arg Leu Leu Glu Gln Ser Leu Arg Gln Gln Arg Ala Phe His His
                   405                 410                 415
   Met Gly Met Met Glu Gln Glu Ala Trp Arg Pro Gln Arg Gly Leu Pro
               420                 425                 430
   Glu Arg Ser Val Asn Ile Leu Arg Ala Trp Leu Phe Glu His Phe Leu
           435                 440                 445
   Asn Pro Tyr Pro Ser Asp Ala Asp Lys His Leu Leu Ala Arg Gln Thr
       450                 455                 460
   Gly Leu Ser Arg Asn Gln Val Ser Asn Trp Phe Ile Asn Ala Arg Val
   465                 470                 475                 480
   Arg Leu Trp Lys Pro Met Val Glu Glu Met Tyr Gln Gln Glu Ala Lys
                   485                 490                 495
   Glu Arg Glu Glu Ala Glu Glu Glu Asn Glu Asn Gln Gln Gln Gln Arg
               500                 505                 510
   Arg Gln Gln Gln Thr Asn Asn Asn Asp Thr Lys Pro Asn Asn Asn Glu
           515                 520                 525
   Asn Asn Phe Thr Val Ile Thr Ala Gln Thr Pro Thr Thr Met Thr Ser
       530                 535                 540
   Thr His His Glu Asn Asp Ser Ser Phe Leu Ser Ser Val Ala Ala Ala
   545                 550                 555                 560
   Ser His Gly Gly Ser Asp Ala Phe Thr Val Ala Thr Cys Gln Gln Asp
                   565                 570                 575
   Val Ser Asp Phe His Val Asp Gly Asp Gly Val Asn Val Ile Arg Phe
               580                 585                 590
   Gly Thr Lys Gln Thr Gly Asp Val Ser Leu Thr Leu Gly Leu Arg His
           595                 600                 605
   Ser Gly Asn Ile Pro Asp Lys Asn Thr Ser Phe Ser Val Arg Asp Phe
       610                 615                 620
   Gly Asp Phe
   625
<210> 49
   <211> 1420
   <212> DNA
   <213> Arabidopsis thaliana
<220>
   <221> CDS
   <222> (138)..(1046)
   <223> G793
<400> 49
   cattctctca cctaacaact ccacacaaga ttcttcgcat ggaaagttgt tcttcgactt      60
   ctcttctcta actcgctatc ttttaactca cccagctcca ctgagtcgaa aatttcaaac     120
   ctttactcgt ttccttc atg gct aat aac aac aac atc cca cat gat agc        170
                      Met Ala Asn Asn Asn Asn Ile Pro His Asp Ser
                      1               5                   10
   atc tcc gat cca tct cct acc gac gat ttc ttc gag cag atc ctc ggg      218
   Ile Ser Asp Pro Ser Pro Thr Asp Asp Phe Phe Glu Gln Ile Leu Gly
               15                  20                  25
   ctt tcc aac ttc tcc ggt tct tca ggt tct ggt ctc tct gga atc ggc      266
   Leu Ser Asn Phe Ser Gly Ser Ser Gly Ser Gly Leu Ser Gly Ile Gly
           30                  35                  40
   ggc gtg ggt cca cct ccg atg atg ctt cag ctt ggt tca ggc aac gaa      314
   Gly Val Gly Pro Pro Pro Met Met Leu Gln Leu Gly Ser Gly Asn Glu
       45                  50                  55
   ggg aat cat aat cat atg ggt gcc att gga gga ggt gga cct gta ggg      362
   Gly Asn His Asn His Met Gly Ala Ile Gly Gly Gly Gly Pro Val Gly
   60                  65                  70                  75
   ttt cat aat cag atg ttt ccg ttg gga tta agt ctc gat caa ggg aaa      410
   Phe His Asn Gln Met Phe Pro Leu Gly Leu Ser Leu Asp Gln Gly Lys
                   80                  85                  90
   gga cat ggc ttt ctt aaa cct gat gaa act ggt aaa cgt ttc caa gac      458
   Gly His Gly Phe Leu Lys Pro Asp Glu Thr Gly Lys Arg Phe Gln Asp
               95                  100                 105
   gat gtt ctt gat aat cga tgt tcc tct atg aaa cct att ttc cat ggg      506
   Asp Val Leu Asp Asn Arg Cys Ser Ser Met Lys Pro Ile Phe His Gly
           110                 115                 120
   cag cca atg tca cag cca gct cca cca atg ccg cat caa cag tct act      554
   Gln Pro Met Ser Gln Pro Ala Pro Pro Met Pro His Gln Gln Ser Thr
       125                 130                 135
   att cgg cct aga gtt agg gct agg cga ggt caa gct acc gat cca cat      602
   Ile Arg Pro Arg Val Arg Ala Arg Arg Gly Gln Ala Thr Asp Pro His
   140                 145                 150                 155
   agc atc gct gag agg ctc cga agg gaa aga ata gca gaa cgg atc agg      650
   Ser Ile Ala Glu Arg Leu Arg Arg Glu Arg Ile Ala Glu Arg Ile Arg
                   160                 165                 170
   tcg ttg cag gaa ctt gta cct acc gtt aac aag aca gat agg gct gct      698
   Ser Leu Gln Glu Leu Val Pro Thr Val Asn Lys Thr Asp Arg Ala Ala
               175                 180                 185
   atg atc gac gag att gtc gat tat gta aag ttt ctc agg ctc caa gtt      746
   Met Ile Asp Glu Ile Val Asp Tyr Val Lys Phe Leu Arg Leu Gln Val
           190                 195                 200
   aag gtc ctg agc atg agc cgt ctt ggt gga gcc ggt gct gtc gca cca      794
   Lys Val Leu Ser Met Ser Arg Leu Gly Gly Ala Gly Ala Val Ala Pro
       205                 210                 215
   cta gtc act gaa atg cca tta tct tca tca gtt gag gat gag acg cag      842
   Leu Val Thr Glu Met Pro Leu Ser Ser Ser Val Glu Asp Glu Thr Gln
   220                 225                 230                 235
   gcc gtg tgg gag aaa tgg tca aac gat ggg aca gag agg caa gtg gct      890
   Ala Val Trp Glu Lys Trp Ser Asn Asp Gly Thr Glu Arg Gln Val Ala
                   240                 245                 250
   aag ctg atg gaa gaa aac gtt gga gca gcg atg caa ctt ttg caa tca      938
   Lys Leu Met Glu Glu Asn Val Gly Ala Ala Met Gln Leu Leu Gln Ser
               255                 260                 265
   aag gct ctt tgc ata atg ccg atc tca ttg gca atg gcg att tac cat      986
   Lys Ala Leu Cys Ile Met Pro Ile Ser Leu Ala Met Ala Ile Tyr His
           270                 275                 280
   tct cag cca cca gac aca tct tct tca atc gtc aaa cca gag atg aat     1034
   Ser Gln Pro Pro Asp Thr Ser Ser Ser Ile Val Lys Pro Glu Met Asn
       285                 290                 295
   cct cca ccg tag atttttgttc atccaacggt ccccagctga tgattgacat         1086
   Pro Pro Pro
   300
   tttgctctgt ttcccactac tagacttttg tgactcatga aaggtaagta aaaaggcatt   1146
   ggagatggaa tctaagtagg atttgtgcag taaagaagta aaacgggatc tgtcaaaaga   1206
   aggaaaaagc tctcgcttgc ttggctagta tttatcattt tgatgaaagt aactcttttt   1266
   tgttcaaaga ctttagtgtg attttcagga ccaagggctt tgagggtagt gctagctgta   1326
   gtaatagtaa tgaaggtgtg ggatcgtgtt tttgaattat gtaaaaaagg aagaaaaaac   1386
   aaatgttggt attatattat ggttttgcct gaaa                               1420
<210> 50
   <211> 302
   <212> PRT
   <213> Arabidopsis thaliana
<400> 50
   Met Ala Asn Asn Asn Asn Ile Pro His Asp Ser Ile Ser Asp Pro Ser
   1               5                   10                  15
   Pro Thr Asp Asp Phe Phe Glu Gln Ile Leu Gly Leu Ser Asn Phe Ser
               20                  25                  30
   Gly Ser Ser Gly Ser Gly Leu Ser Gly Ile Gly Gly Val Gly Pro Pro
           35                  40                  45
   Pro Met Met Leu Gln Leu Gly Ser Gly Asn Glu Gly Asn His Asn His
       50                  55                  60
   Met Gly Ala Ile Gly Gly Gly Gly Pro Val Gly Phe His Asn Gln Met
   65                  70                  75                  80
   Phe Pro Leu Gly Leu Ser Leu Asp Gln Gly Lys Gly His Gly Phe Leu
                   85                  90                  95
   Lys Pro Asp Glu Thr Gly Lys Arg Phe Gln Asp Asp Val Leu Asp Asn
               100                 105                 110
   Arg Cys Ser Ser Met Lys Pro Ile Phe His Gly Gln Pro Met Ser Gln
           115                 120                 125
   Pro Ala Pro Pro Met Pro His Gln Gln Ser Thr Ile Arg Pro Arg Val
           130                 135                 140
   Arg Ala Arg Arg Gly Gln Ala Thr Asp Pro His Ser Ile Ala Glu Arg
   145                 150                 155                 160
   Leu Arg Arg Glu Arg Ile Ala Glu Arg Ile Arg Ser Leu Gln Glu Leu
                   165                 170                 175
   Val Pro Thr Val Asn Lys Thr Asp Arg Ala Ala Met Ile Asp Glu Ile
               180                 185                 190
   Val Asp Tyr Val Lys Phe Leu Arg Leu Gln Val Lys Val Leu Ser Met
           195                 200                 205
   Ser Arg Leu Gly Gly Ala Gly Ala Val Ala Pro Leu Val Thr Glu Met
       210                 215                 220
   Pro Leu Ser Ser Ser Val Glu Asp Glu Thr Gln Ala Val Trp Glu Lys
   225                 230                 235                 240
   Trp Ser Asn Asp Gly Thr Glu Arg Gln Val Ala Lys Leu Met Glu Glu
                   245                 250                 255
   Asn Val Gly Ala Ala Met Gln Leu Leu Gln Ser Lys Ala Leu Cys Ile
               260                 265                 270
   Met Pro Ile Ser Leu Ala Met Ala Ile Tyr His Ser Gln Pro Pro Asp
           275                 280                 285
   Thr Ser Ser Ser Ile Val Lys Pro Glu Met Asn Pro Pro Pro
       290                 295                 300
<210> 51
   <211> 1198
   <212> DNA
   <213> Arabidopsis thaliana
<220>
   <221> CDS
   <222> (96)..(1052)
   <223> G764
<400> 51
   atcgaattcg cggccgctcg atatctttac aaccattaaa caaaaaattt ggccactaca      60
   agttgaaaaa gttttgatta tatctaatcg ctgaa atg gat tac aag gta tca        113
                                          Met Asp Tyr Lys Val Ser
                                          1               5
   aga agt ggg gag ata gta gaa gga gaa gta gaa gat tca gaa aag att       161
   Arg Ser Gly Glu Ile Val Glu Gly Glu Val Glu Asp Ser Glu Lys Ile
               10                  15                  20
   gat tta cca cct ggt ttc aga ttt cac cca act gat gaa gaa ctt ata       209
   Asp Leu Pro Pro Gly Phe Arg Phe His Pro Thr Asp Glu Glu Leu Ile
           25                  30                  35
   aca cac tat cta aga cca aag gtt gta aac tct ttt ttc tct gct ata       257
   Thr His Tyr Leu Arg Pro Lys Val Val Asn Ser Phe Phe Ser Ala Ile
       40                  45                  50
   gct att ggt gaa gtt gat ctc aac aaa gtc gag cct tgg gac ttg cct       305
   Ala Ile Gly Glu Val Asp Leu Asn Lys Val Glu Pro Trp Asp Leu Pro
   55                  60                  65                  70
   tgg aag gct aag ctt ggg gaa aaa gag tgg tac ttc ttt tgc gta aga      353
   Trp Lys Ala Lys Leu Gly Glu Lys Glu Trp Tyr Phe Phe Cys Val Arg
                   75                  80                  85
   gac cga aaa tac ccg act ggt tta aga acg aat cgt gct act aaa gcc      401
   Asp Arg Lys Tyr Pro Thr Gly Leu Arg Thr Asn Arg Ala Thr Lys Ala
               90                  95                  100
   ggt tat tgg aaa gct aca ggg aaa gat aaa gag atc ttc aaa ggg aaa      449
   Gly Tyr Trp Lys Ala Thr Gly Lys Asp Lys Glu Ile Phe Lys Gly Lys
           105                 110                 115
   tct ctt gtt ggt atg aag aaa aca ttg gtt ttc tac aaa gga aga gct      497
   Ser Leu Val Gly Met Lys Lys Thr Leu Val Phe Tyr Lys Gly Arg Ala
       120                 125                 130
   cct aaa gga gta aaa aca aat tgg gtc atg cat gag tat cga tta gaa      545
   Pro Lys Gly Val Lys Thr Asn Trp Val Met His Glu Tyr Arg Leu Glu
   135                 140                 145                 150
   ggc aaa ttc gct atc gat aat ctc tct aaa acc gct aag aac gaa tgt      593
   Gly Lys Phe Ala Ile Asp Asn Leu Ser Lys Thr Ala Lys Asn Glu Cys
                   155                 160                 165
   gtt att agt cgt gtt ttt cat aca cgg act gat ggt acg aag gag cat      641
   Val Ile Ser Arg Val Phe His Thr Arg Thr Asp Gly Thr Lys Glu His
               170                 175                 180
   atg tcc gtt ggt tta cct ccg ctg atg gat tct tct cca tat cta aag      689
   Met Ser Val Gly Leu Pro Pro Leu Met Asp Ser Ser Pro Tyr Leu Lys
           185                 190                 195
   agt aga gga caa gac tct tta gcc ggg acc acc ctt ggt ggg ttg ttg      737
   Ser Arg Gly Gln Asp Ser Leu Ala Gly Thr Thr Leu Gly Gly Leu Leu
       200                 205                 210
   tct cac gtt acc tac ttc tcc gac caa aca acc gat gac aag agt ctt      785
   Ser His Val Thr Tyr Phe Ser Asp Gln Thr Thr Asp Asp Lys Ser Leu
   215                 220                 225                 230
   gtg gcc gat ttt aaa act acc atg ttt ggt tcc gga tcg act aac ttt      833
   Val Ala Asp Phe Lys Thr Thr Met Phe Gly Ser Gly Ser Thr Asn Phe
                   235                 240                 245
   tta cca aac ata ggt tct cta cta gac ttc gat cct ctg ttt cta caa      881
   Leu Pro Asn Ile Gly Ser Leu Leu Asp Phe Asp Pro Leu Phe Leu Gln
               250                 255                 260
   aac aat tct tca gta ctg aag atg ttg ctt gac aat gaa gaa acc caa      929
   Asn Asn Ser Ser Val Leu Lys Met Leu Leu Asp Asn Glu Glu Thr Gln
           265                 270                 275
   ttt aag aag aat ctt cac aat tca ggt tca tca gag agt gaa cta aca      977
   Phe Lys Lys Asn Leu His Asn Ser Gly Ser Ser Glu Ser Glu Leu Thr
       280                 285                 290
   gcg agt tct tgg caa ggt cac aat tct tat ggt tcc act ggt cca gtg      1025
   Ala Ser Ser Trp Gln Gly His Asn Ser Tyr Gly Ser Thr Gly Pro Val
   295                 300                 305                 310
   aat ctt gat tgc gtt tgg aaa ttc tga atttggaaaa tcgaaaattt            1072
   Asn Leu Asp Cys Val Trp Lys Phe
                   315
   ggatgttaac tagggggtat atagggtttt taaaaacagt gtatatatgc gttatgtgtt    1132
   agctttagat tctaggatat acaaagatga cactaataga ttcttataac attttgtaaa    1192
   aaaaaa                                                               1198
<210> 52
   <211> 318
   <212> PRT
   <213> Arabidopsis thaliana
<400> 52
   Met Asp Tyr Lys Val Ser Arg Ser Gly Glu Ile Val Glu Gly Glu Val
   1               5                   10                  15
   Glu Asp Ser Glu Lys Ile Asp Leu Pro Pro Gly Phe Arg Phe His Pro
               20                  25                  30
   Thr Asp Glu Glu Leu Ile Thr His Tyr Leu Arg Pro Lys Val Val Asn
           35                  40                  45
   Ser Phe Phe Ser Ala Ile Ala Ile Gly Glu Val Asp Leu Asn Lys Val
       50                  55                  60
   Glu Pro Trp Asp Leu Pro Trp Lys Ala Lys Leu Gly Glu Lys Glu Trp
   65                  70                  75                  80
   Tyr Phe Phe Cys Val Arg Asp Arg Lys Tyr Pro Thr Gly Leu Arg Thr
                   85                  90                  95
   Asn Arg Ala Thr Lys Ala Gly Tyr Trp Lys Ala Thr Gly Lys Asp Lys
               100                 105                 110
   Glu Ile Phe Lys Gly Lys Ser Leu Val Gly Met Lys Lys Thr Leu Val
           115                 120                 125
   Phe Tyr Lys Gly Arg Ala Pro Lys Gly Val Lys Thr Asn Trp Val Met
       130                 135                 140
   His Glu Tyr Arg Leu Glu Gly Lys Phe Ala Ile Asp Asn Leu Ser Lys
   145                 150                 155                 160
   Thr Ala Lys Asn Glu Cys Val Ile Ser Arg Val Phe His Thr Arg Thr
                   165                 170                 175
   Asp Gly Thr Lys Glu His Met Ser Val Gly Leu Pro Pro Leu Met Asp
               180                 185                 190
   Ser Ser Pro Tyr Leu Lys Ser Arg Gly Gln Asp Ser Leu Ala Gly Thr
           195                 200                 205
   Thr Leu Gly Gly Leu Leu Ser His Val Thr Tyr Phe Ser Asp Gln Thr
       210                 215                 220
   Thr Asp Asp Lys Ser Leu Val Ala Asp Phe Lys Thr Thr Met Phe Gly
   225                 230                 235                 240
   Ser Gly Ser Thr Asn Phe Leu Pro Asn Ile Gly Ser Leu Leu Asp Phe
                   245                 250                 255
   Asp Pro Leu Phe Leu Gln Asn Asn Ser Ser Val Leu Lys Met Leu Leu
               260                 265                 270
   Asp Asn Glu Glu Thr Gln Phe Lys Lys Asn Leu His Asn Ser Gly Ser
           275                 280                 285
   Ser Glu Ser Glu Leu Thr Ala Ser Ser Trp Gln Gly His Asn Ser Tyr
   290 295 300
   Gly Ser Thr Gly Pro Val Asn Leu Asp Cys Val Trp Lys Phe
   305 310 315
<210> 53
   <211> 932
   <212> DNA
   <213> Arabidopsis thaliana
<220>
   <221> CDS
   <222> (43)..(759)
   <223> G350
<400> 53
   ctttcttctc aatttagaac ttagtagcta gtcttcaaga ta atg gca ctt gaa 54
   Met Ala Leu Glu
   1
   act ctt act tct cca aga tta tct tct ccg atg ccg act ctg ttt caa 102
   Thr Leu Thr Ser Pro Arg Leu Ser Ser Pro Met Pro Thr Leu Phe Gln
   5 10 15 20
   gat tca gca cta ggg ttt cat gga agc aaa ggc aaa cga tct aag cga 150
   Asp Ser Ala Leu Gly Phe His Gly Ser Lys Gly Lys Arg Ser Lys Arg
   25 30 35
   tca aga tct gaa ttc gac cgt cag agt ctc acg gag gat gaa tat atc 198
   Ser Arg Ser Glu Phe Asp Arg Gln Ser Leu Thr Glu Asp Glu Tyr Ile
   40 45 50
   gct tta tgt ctc atg ctt ctt gct cgc gac gga gat aga aac cgt gac 246
   Ala Leu Cys Leu Met Leu Leu Ala Arg Asp Gly Asp Arg Asn Arg Asp
   55 60 65
   ctt gac ctg cct tct tct tcg tct tca cct cct ctg ctt cct cct ctt 294
   Leu Asp Leu Pro Ser Ser Ser Ser Ser Pro Pro Leu Leu Pro Pro Leu
   70 75 80
   cct act ccg atc tac aag tgt agc gtc tgt gac aag gcg ttt tcg tct 342
   Pro Thr Pro Ile Tyr Lys Cys Ser Val Cys Asp Lys Ala Phe Ser Ser
   85 90 95 100
   tac cag gct ctt ggt gga cac aag gca agt cac cgg aaa agc ttt tcg 390
   Tyr Gln Ala Leu Gly Gly His Lys Ala Ser His Arg Lys Ser Phe Ser
   105 110 115
   ctt act caa tct gcc gga gga gat gag ctg tcg aca tcg tcg gcg ata 438
   Leu Thr Gln Ser Ala Gly Gly Asp Glu Leu Ser Thr Ser Ser Ala Ile
   120 125 130
   acc acg tct ggt ata tcc ggt ggc ggg gga gga agt gtg aag tcg cac 486
   Thr Thr Ser Gly Ile Ser Gly Gly Gly Gly Gly Ser Val Lys Ser His
   135 140 145
   gtt tgc tct atc tgt cat aaa tcg ttc gcc acc ggt caa gct ctc ggc 534
   Val Cys Ser Ile Cys His Lys Ser Phe Ala Thr Gly Gln Ala Leu Gly
   150 155 160
   ggc cac aaa cgg tgc cac tac gaa gga aag aac gga ggc ggt gtg agt 582
   Gly His Lys Arg Cys His Tyr Glu Gly Lys Asn Gly Gly Gly Val Ser
   165 170 175 180
   agt agc gtg tcg aat tct gaa gat gtg ggg tct aca agc cac gtc agc 630
   Ser Ser Val Ser Asn Ser Glu Asp Val Gly Ser Thr Ser His Val Ser
   185 190 195
   agt ggc cac cgt ggg ttt gac ctc aac ata ccg ccg ata ccg gaa ttc 678
   Ser Gly His Arg Gly Phe Asp Leu Asn Ile Pro Pro Ile Pro Glu Phe
   200 205 210
   tcg atg gtc aac gga gac gaa gag gtg atg agt cct atg ccg gcg aag 726
   Ser Met Val Asn Gly Asp Glu Glu Val Met Ser Pro Met Pro Ala Lys
   215 220 225
   aaa ctc cgg ttt gac ttc ccg gag aaa ccc taa acataaacct aggaaaaact 779
   Lys Leu Arg Phe Asp Phe Pro Glu Lys Pro
   230 235
   ttacagaatt cattttatag gaaattgttt tactgtatat acaaatatcg attttgattg 839
   atgttcttct tcactgaaaa attatgattc tttgttgtat aattgatgtt tctgaaaaag 899
   atataacttt ttattaaaaa aaaaaaaaaa aaa 932
<210> 54
   <211> 238
   <212> PRT
   <213> Arabidopsis thaliana
<400> 54
   Met Ala Leu Glu Thr Leu Thr Ser Pro Arg Leu Ser Ser Pro Met Pro
   1               5                   10                  15
   Thr Leu Phe Gln Asp Ser Ala Leu Gly Phe His Gly Ser Lys Gly Lys
               20                  25                  30
   Arg Ser Lys Arg Ser Arg Ser Glu Phe Asp Arg Gln Ser Leu Thr Glu
           35                  40                  45
   Asp Glu Tyr Ile Ala Leu Cys Leu Met Leu Leu Ala Arg Asp Gly Asp
       50                  55                  60
   Arg Asn Arg Asp Leu Asp Leu Pro Ser Ser Ser Ser Ser Pro Pro Leu
   65                  70                  75                  80
   Leu Pro Pro Leu Pro Thr Pro Ile Tyr Lys Cys Ser Val Cys Asp Lys
                   85                  90                  95
   Ala Phe Ser Ser Tyr Gln Ala Leu Gly Gly His Lys Ala Ser His Arg
               100                 105                 110
   Lys Ser Phe Ser Leu Thr Gln Ser Ala Gly Gly Asp Glu Leu Ser Thr
           115                 120                 125
   Ser Ser Ala Ile Thr Thr Ser Gly Ile Ser Gly Gly Gly Gly Gly Ser
       130                 135                 140
   Val Lys Ser His Val Cys Ser Ile Cys His Lys Ser Phe Ala Thr Gly
   145                 150                 155                 160
   Gln Ala Leu Gly Gly His Lys Arg Cys His Tyr Glu Gly Lys Asn Gly
                   165                 170                 175
   Gly Gly Val Ser Ser Ser Val Ser Asn Ser Glu Asp Val Gly Ser Thr
               180                 185                 190
   Ser His Val Ser Ser Gly His Arg Gly Phe Asp Leu Asn Ile Pro Pro
           195                 200                 205
   Ile Pro Glu Phe Ser Met Val Asn Gly Asp Glu Glu Val Met Ser Pro
       210                 215                 220
   Met Pro Ala Lys Lys Leu Arg Phe Asp Phe Pro Glu Lys Pro
   225                 230                 235
<210> 55
   <211> 1022
   <212> DNA
   <213> Arabidopsis thaliana
<220>
   <221> CDS
   <222> (31)..(846)
   <223> G986
<400> 55
   cattaaattg gctcctgtga acctaaattt atg gac tat gat ccc aac acc aat       54
                                    Met Asp Tyr Asp Pro Asn Thr Asn
                                    1               5
   ccg ttc gac ctt cat ttc tcc ggt aaa ctt ccg aaa aga gaa gtc tcg       102
   Pro Phe Asp Leu His Phe Ser Gly Lys Leu Pro Lys Arg Glu Val Ser
       10                  15                  20
   gct tca gct tct aaa gtt gta gag aag aaa tgg tta gtg aaa gat gag       150
   Ala Ser Ala Ser Lys Val Val Glu Lys Lys Trp Leu Val Lys Asp Glu
   25                  30                  35                  40
   aag aga aat atg cta caa gat gaa ata aac cgg gtt aat tcg gag aac       198
   Lys Arg Asn Met Leu Gln Asp Glu Ile Asn Arg Val Asn Ser Glu Asn
                   45                  50                  55
   aag aag cta acc gaa atg tta gca aga gtc tgt gag aag tac tat gct       246
   Lys Lys Leu Thr Glu Met Leu Ala Arg Val Cys Glu Lys Tyr Tyr Ala
               60                  65                  70
   ctt aat aat ctt atg gag gag ttg cag agt cga aag agt cct gaa agt       294
   Leu Asn Asn Leu Met Glu Glu Leu Gln Ser Arg Lys Ser Pro Glu Ser
           75                  80                  85
   gtt aac ttt cag aac aaa cag cta acg ggg aaa cga aaa caa gaa ctt       342
   Val Asn Phe Gln Asn Lys Gln Leu Thr Gly Lys Arg Lys Gln Glu Leu
       90                  95                  100
   gat gag ttt gtt agc tcc cca att gga ctc agt ctc gga cca atc gag       390
   Asp Glu Phe Val Ser Ser Pro Ile Gly Leu Ser Leu Gly Pro Ile Glu
   105                 110                 115                 120
   aac atc acc aac gat aaa gcg acg gtt tca acc gct tac ttt gct gct       438
   Asn Ile Thr Asn Asp Lys Ala Thr Val Ser Thr Ala Tyr Phe Ala Ala
                   125                 130                 135
   gag aag tct gac aca agc ttg act gtg aaa gat gga tat caa tgg agg       486
   Glu Lys Ser Asp Thr Ser Leu Thr Val Lys Asp Gly Tyr Gln Trp Arg
               140                 145                 150
   aaa tac ggg caa aag att acg aga gat aat cca tct cct aga gct tac       534
   Lys Tyr Gly Gln Lys Ile Thr Arg Asp Asn Pro Ser Pro Arg Ala Tyr
           155                 160                 165
   ttc aga tgc tcg ttt tca ccg tct tgt cta gtc aag aag aag gtg caa       582
   Phe Arg Cys Ser Phe Ser Pro Ser Cys Leu Val Lys Lys Lys Val Gln
       170                 175                 180
   cga agt gca gaa gat cca tct ttc ttg gta gcc act tac gaa ggg aca       630
   Arg Ser Ala Glu Asp Pro Ser Phe Leu Val Ala Thr Tyr Glu Gly Thr
   185                 190                 195                 200
   cat aac cac acc gga cca cat gca agt gtg tcc agg aca gtg aaa ctt       678
   His Asn His Thr Gly Pro His Ala Ser Val Ser Arg Thr Val Lys Leu
                   205                 210                 215
   gat cta gtt caa ggt ggg ctt gaa cca gtt gag gaa aag aaa gag aga      726
   Asp Leu Val Gln Gly Gly Leu Glu Pro Val Glu Glu Lys Lys Glu Arg
               220                 225                 230
   ggg acg att caa gag gtt ttg gtg caa caa atg gct tct tcg ttg acc      774
   Gly Thr Ile Gln Glu Val Leu Val Gln Gln Met Ala Ser Ser Leu Thr
           235                 240                 245
   aaa gat cct aag ttc act gca gct ctt gcg act gct att tcc ggg aga      822
   Lys Asp Pro Lys Phe Thr Ala Ala Leu Ala Thr Ala Ile Ser Gly Arg
       250                 255                 260
   ttg ata gag cat tca aga aca tga aagttctcta gaacatgtat atttctgttt     876
   Leu Ile Glu His Ser Arg Thr
   265                 270
   tgttctattt tgttgctcat tcctagtaaa aaggtaaaga tttgtttgat cttgattagg    936
   aggcatagat gtcaatttta atgtgtgtgt atataattac atcaaatcta agtatccaaa    996
   aagggtcacc cccattttat cttatg                                        1022
<210> 56
   <211> 271
   <212> PRT
   <213> Arabidopsis thaliana
<400> 56
   Met Asp Tyr Asp Pro Asn Thr Asn Pro Phe Asp Leu His Phe Ser Gly
   1               5                   10                  15
   Lys Leu Pro Lys Arg Glu Val Ser Ala Ser Ala Ser Lys Val Val Glu
               20                  25                  30
   Lys Lys Trp Leu Val Lys Asp Glu Lys Arg Asn Met Leu Gln Asp Glu
           35                  40                  45
   Ile Asn Arg Val Asn Ser Glu Asn Lys Lys Leu Thr Glu Met Leu Ala
       50                  55                  60
   Arg Val Cys Glu Lys Tyr Tyr Ala Leu Asn Asn Leu Met Glu Glu Leu
   65                  70                  75                  80
   Gln Ser Arg Lys Ser Pro Glu Ser Val Asn Phe Gln Asn Lys Gln Leu
                   85                  90                  95
   Thr Gly Lys Arg Lys Gln Glu Leu Asp Glu Phe Val Ser Ser Pro Ile
               100                 105                 110
   Gly Leu Ser Leu Gly Pro Ile Glu Asn Ile Thr Asn Asp Lys Ala Thr
           115                 120                 125
   Val Ser Thr Ala Tyr Phe Ala Ala Glu Lys Ser Asp Thr Ser Leu Thr
       130                 135                 140
   Val Lys Asp Gly Tyr Gln Trp Arg Lys Tyr Gly Gln Lys Ile Thr Arg
   145                 150                 155                 160
   Asp Asn Pro Ser Pro Arg Ala Tyr Phe Arg Cys Ser Phe Ser Pro Ser
                   165                 170                 175
   Cys Leu Val Lys Lys Lys Val Gln Arg Ser Ala Glu Asp Pro Ser Phe
               180                 185                 190
   Leu Val Ala Thr Tyr Glu Gly Thr His Asn His Thr Gly Pro His Ala
           195                 200                 205
   Ser Val Ser Arg Thr Val Lys Leu Asp Leu Val Gln Gly Gly Leu Glu
       210                 215                 220
   Pro Val Glu Glu Lys Lys Glu Arg Gly Thr Ile Gln Glu Val Leu Val
   225                 230                 235                 240
   Gln Gln Met Ala Ser Ser Leu Thr Lys Asp Pro Lys Phe Thr Ala Ala
                   245                 250                 255
   Leu Ala Thr Ala Ile Ser Gly Arg Leu Ile Glu His Ser Arg Thr
               260                 265                 270
<210> 57
   <211> 1230
   <212> DNA
   <213> Arabidopsis thaliana
<220>
   <221> CDS
   <222> (1)..(1089)
   <223> G1349
<400> 57
   atg gct agt cgg aga gaa gta cgg tgc cgg tgc ggc aga cgg atg tgg      48
   Met Ala Ser Arg Arg Glu Val Arg Cys Arg Cys Gly Arg Arg Met Trp
   1               5                   10                  15
   gtt caa cca gac gcc cgt acc gtc caa tgc tca acc tgc cac acc gtc      96
   Val Gln Pro Asp Ala Arg Thr Val Gln Cys Ser Thr Cys His Thr Val
               20                  25                  30
   acg cag ctc tac tcg cta gtg gac ata gct cgc ggt gca aac cgc ata     144
   Thr Gln Leu Tyr Ser Leu Val Asp Ile Ala Arg Gly Ala Asn Arg Ile
           35                  40                  45
   att cat ggg ttt caa cag cta ctt aga caa cac caa ccg caa cat cat     192
   Ile His Gly Phe Gln Gln Leu Leu Arg Gln His Gln Pro Gln His His
       50                  55                  60
   gaa caa caa caa caa caa atg atg gct caa ccg cca cca cgg ctg ctt     240
   Glu Gln Gln Gln Gln Gln Met Met Ala Gln Pro Pro Pro Arg Leu Leu
   65                  70                  75                  80
   gag cct ctt ccc tcg ccg ttt ggg aag aag aga gca gtt tta tgc ggc     288
   Glu Pro Leu Pro Ser Pro Phe Gly Lys Lys Arg Ala Val Leu Cys Gly
                   85                  90                  95
   gtg aac tat aag gga aaa agt tat agc ttg aaa ggt tgc atc agt gat     336
   Val Asn Tyr Lys Gly Lys Ser Tyr Ser Leu Lys Gly Cys Ile Ser Asp
               100                 105                 110
   gct aag tcc atg aga tct tta ttg gtt caa caa atg ggt ttc cct att     384
   Ala Lys Ser Met Arg Ser Leu Leu Val Gln Gln Met Gly Phe Pro Ile
           115                 120                 125
   gac tct att ctc atg ctc aca gaa gat gaa gcc agc ccg cag aga ata     432
   Asp Ser Ile Leu Met Leu Thr Glu Asp Glu Ala Ser Pro Gln Arg Ile
       130                 135                 140
   ccg acg aag aga aac att agg aag gcg atg aga tgg tta gtt gaa ggg     480
   Pro Thr Lys Arg Asn Ile Arg Lys Ala Met Arg Trp Leu Val Glu Gly
   145                 150                 155                 160
   aac aga gca agg gac tca cta gtg ttc cat ttc tct ggt cat gga tct      528
   Asn Arg Ala Arg Asp Ser Leu Val Phe His Phe Ser Gly His Gly Ser
                   165                 170                 175
   cag cag aat gac tac aac gga gac gag atc gat ggt caa gat gaa gcc      576
   Gln Gln Asn Asp Tyr Asn Gly Asp Glu Ile Asp Gly Gln Asp Glu Ala
               180                 185                 190
   ttg tgc cct tta gac cat gaa aca gaa gga aaa atc att gat gac gag      624
   Leu Cys Pro Leu Asp His Glu Thr Glu Gly Lys Ile Ile Asp Asp Glu
           195                 200                 205
   att aac cgg ata ctc gtg agg cct ctc gtc cat gga gct aag ctt cac      672
   Ile Asn Arg Ile Leu Val Arg Pro Leu Val His Gly Ala Lys Leu His
       210                 215                 220
   gct gtc atc gac gcc tgt aac agc ggg act gtc ctt gat tta ccc ttc      720
   Ala Val Ile Asp Ala Cys Asn Ser Gly Thr Val Leu Asp Leu Pro Phe
   225                 230                 235                 240
   att tgc agg atg gag agg aat ggt tct tat gaa tgg gaa gac cat aga      768
   Ile Cys Arg Met Glu Arg Asn Gly Ser Tyr Glu Trp Glu Asp His Arg
                   245                 250                 255
   tca gtc aga gct tac aaa gga aca gat ggt gga gca gct ttc tgt ttc      816
   Ser Val Arg Ala Tyr Lys Gly Thr Asp Gly Gly Ala Ala Phe Cys Phe
               260                 265                 270
   agt gct tgt gac gat gat gaa tcc agt ggt tac act cct gtg ttc acg      864
   Ser Ala Cys Asp Asp Asp Glu Ser Ser Gly Tyr Thr Pro Val Phe Thr
           275                 280                 285
   ggg aag aac aca gga gcc atg act tat agc ttc ata aag gcg gtg aag      912
   Gly Lys Asn Thr Gly Ala Met Thr Tyr Ser Phe Ile Lys Ala Val Lys
       290                 295                 300
   aca gct gga cca gca ccc acg tat ggc cac ctg ctt aac ctt atg tgt      960
   Thr Ala Gly Pro Ala Pro Thr Tyr Gly His Leu Leu Asn Leu Met Cys
   305                 310                 315                 320
   tct gca ata cga gag gcc cag tct cgc ctc gcc ttt aac ggg gac tac     1008
   Ser Ala Ile Arg Glu Ala Gln Ser Arg Leu Ala Phe Asn Gly Asp Tyr
                   325                 330                 335
   aca agc tct gat gca tcc gcg gag cca ctg cta aca tca tct gag gaa     1056
   Thr Ser Ser Asp Ala Ser Ala Glu Pro Leu Leu Thr Ser Ser Glu Glu
               340                 345                 350
   ttt gac gtg tac gcg aca aag ttt gta ctc tga atgctgtaca tatgatgctg   1109
   Phe Asp Val Tyr Ala Thr Lys Phe Val Leu
           355                 360
   caaatagctc ggaaacgttt ctatgtgtat gtatcatgta atgattatgt tgcatagcct   1169
   ctctcttctt acgagcaata agctatgaaa taattgattc gctaagaaat ttaaaatgaa   1229
   a                                                                   1230
<210> 58
   <211> 362
   <212> PRT
   <213> Arabidopsis thaliana
<400> 58
   Met Ala Ser Arg Arg Glu Val Arg Cys Arg Cys Gly Arg Arg Met Trp
   1               5                   10                  15
   Val Gln Pro Asp Ala Arg Thr Val Gln Cys Ser Thr Cys His Thr Val
               20                  25                  30
   Thr Gln Leu Tyr Ser Leu Val Asp Ile Ala Arg Gly Ala Asn Arg Ile
   35 40 45
   Ile His Gly Phe Gln Gln Leu Leu Arg Gln His Gln Pro Gln His His
   50 55 60
   Glu Gln Gln Gln Gln Gln Met Met Ala Gln Pro Pro Pro Arg Leu Leu
   65 70 75 80
   Glu Pro Leu Pro Ser Pro Phe Gly Lys Lys Arg Ala Val Leu Cys Gly
   85 90 95
   Val Asn Tyr Lys Gly Lys Ser Tyr Ser Leu Lys Gly Cys Ile Ser Asp
   100 105 110
   Ala Lys Ser Met Arg Ser Leu Leu Val Gln Gln Met Gly Phe Pro Ile
   115 120 125
   Asp Ser Ile Leu Met Leu Thr Glu Asp Glu Ala Ser Pro Gln Arg Ile
   130 135 140
   Pro Thr Lys Arg Asn Ile Arg Lys Ala Met Arg Trp Leu Val Glu Gly
   145 150 155 160
   Asn Arg Ala Arg Asp Ser Leu Val Phe His Phe Ser Gly His Gly Ser
   165 170 175
   Gln Gln Asn Asp Tyr Asn Gly Asp Glu Ile Asp Gly Gln Asp Glu Ala
   180 185 190
   Leu Cys Pro Leu Asp His Glu Thr Glu Gly Lys Ile Ile Asp Asp Glu
   195 200 205
   Ile Asn Arg Ile Leu Val Arg Pro Leu Val His Gly Ala Lys Leu His
   210 215 220
   Ala Val Ile Asp Ala Cys Asn Ser Gly Thr Val Leu Asp Leu Pro Phe
   225 230 235 240
   Ile Cys Arg Met Glu Arg Asn Gly Ser Tyr Glu Trp Glu Asp His Arg
   245 250 255
   Ser Val Arg Ala Tyr Lys Gly Thr Asp Gly Gly Ala Ala Phe Cys Phe
   260 265 270
   Ser Ala Cys Asp Asp Asp Glu Ser Ser Gly Tyr Thr Pro Val Phe Thr
   275 280 285
   Gly Lys Asn Thr Gly Ala Met Thr Tyr Ser Phe Ile Lys Ala Val Lys
   290 295 300
   Thr Ala Gly Pro Ala Pro Thr Tyr Gly His Leu Leu Asn Leu Met Cys
   305 310 315 320
   Ser Ala Ile Arg Glu Ala Gln Ser Arg Leu Ala Phe Asn Gly Asp Tyr
                       325                 330                 335
   Thr Ser Ser Asp Ala Ser Ala Glu Pro Leu Leu Thr Ser Ser Glu Glu
               340                 345                 350
   Phe Asp Val Tyr Ala Thr Lys Phe Val Leu
           355                 360